# EUROPEAN PATENT SPECIFICATION

(11) **EP 1 996 188 B1**
(45) Date of publication and mention of the grant of the patent: **15.05.2013**
(21) Application number: 07727116.1
(22) Date of filing: 20.03.2007
(51) Int. Cl.: A61K 31/4184, C07D 235/26, A61P 25/18

(54) **BENZIMIDAZOLES WHICH HAVE ACTIVITY AT M1 RECEPTOR AND THEIR USES IN MEDICINE**
BENZIMIDAZOLE MIT WIRKUNG AM M1-REZEPTOR UND IHRE VERWENDUNGEN IN DER MEDIZIN
BENZIMIDAZOLES PRÉSENTANT UNE ACTIVITÉ AU NIVEAU DU RÉCEPTEUR M1 ET LEURS UTILISATIONS EN MÉDECINE

(30) Priority: 22.03.2006 GB 0605784
(43) Date of publication of application: 03.12.2008
(73) Proprietor: Glaxo Group Limited, Brentford, Middlesex TW8 9GS (GB)
(72) Inventor: COOPER, David, Gwyn, Harlow Essex CM19 5AW (GB); FORBES, Ian, Thomson, Harlow Essex CM19 5AW (GB); GARZYA, Vincenzo, Harlow Essex CM19 5AW (GB); JIN, Jian, Collegeville, PA 19426 (US); LOUCHART, Yann, Harlow Essex CM19 5AW (GB); WALKER, Graham, Harlow Essex CM19 5AW (GB); WYMAN, Paul, Adrian, Harlow Essex CM19 5AW (GB)
(74) Representative: Kondo, Rie
(86) International application number: PCT/EP2007/052639
(87) International publication number: WO 2007/107566

(56) References cited:
- EP-A- 1 491 212
- EP-A1- 0 068 261
- WO-A-96/13262
- WO-A-97/16186
- WO-A-03/105781
- WO-A-2007/036711
- WO-A-2007/036718
- US-A- 3 989 707
- US-A- 4 292 321
- HENNING R ET AL: "SYNTHESIS AND NEUROLEPTIC OF A SIERIES OF 1-1-(BENZO-1,4-DIOXAN-2-YLM ETHYL)-4-PIPERIDINYLBENZIMIDAZOLONE DERIVATIVES" JOURNAL OF MEDICINAL CHEMISTRY, AMERICAN CHEMICAL SOCIETY. WASHINGTON, US, vol. 30, no. 5, 1987, pages 814-819, XP001084049 ISSN: 0022-2623
- BURGEY ET AL: "Benzodiazepine calcitonin gene-related peptide (CGRP) receptor antagonists: Optimization of the 4-substituted piperidine" BIOORGANIC & MEDICINAL CHEMISTRY LETTERS, OXFORD, GB, vol. 16, no. 19, 1 October 2006 (2006-10-01), pages 5052-5056, XP005611693 ISSN: 0960-894X

## Description

This invention relates to novel compounds, pharmaceutical compositions containing them and their use in therapy, in particular as antipsychotic agents.

Muscarinic acetylcholine receptors are members of the G protein coupled receptor superfamily which mediate the actions of the neurotransmitter acetylcholine in both the central and peripheral nervous system. Five muscarinic receptor subtypes have been cloned, M₁ to M₅. The muscarinic M₁ receptor is predominantly expressed in the cerebral cortex and hippocampus, although it is also expressed in the periphery e.g. exocrine glands.

Muscarinic receptors in the central nervous system, especially M₁, play a critical role in mediating higher cognitive processing. Diseases associated with cognitive impairments, such as Alzheimer's disease, are accompanied by loss of cholinergic neurons in the basal forebrain. Furthermore, in animal models, blockade or lesion of central cholinergic pathways results in profound cognitive deficits.

Cholinergic replacement therapy has largely been based on the use of acetylcholinesterase inhibitors to prevent the breakdown of endogenous acetylcholine. These compounds have shown efficacy versus symptomatic cognitive decline in the clinic, but give rise to side effects resulting from stimulation of peripheral muscarinic receptors including disturbed gastrointestinal motility and nausea.

The dopamine hypothesis of schizophrenia suggests that excess dopaminergic stimulation is responsible for the positive symptoms of the disease, hence the utility of dopamine receptor antagonists to reduce psychotic symptoms. However, conventional dopamine receptor antagonists can cause extrapyramidal side effects (EPS) in patients, including tremor and tardive dyskinesias.

M₁ receptor agonists have been sought for the symptomatic treatment of cognitive decline. More recently, a number of groups have shown that muscarinic receptor agonists display an atypical antipsychotic-like profile in a range of pre-clinical paradigms. The muscarinic agonist, xanomeline, reverses a number of dopamine driven behaviours, including amphetamine induced locomotion in rats, apomorphine induced climbing in mice, dopamine agonist driven turning in unilateral 6-OH-DA lesioned rats and amphetamine-induced motor unrest in monkeys (without EPS liability). It also has been shown to inhibit A10, but not A9, dopamine cell firing and conditioned avoidance and induces c-*fos* expression in prefrontal cortex and nucleus accumbens, but not in striatum in rats. These data are all suggestive of an atypical antipsychotic-like profile.

Xanomeline has also been shown to reduce psychotic symptoms such as suspiciousness, hallucinations and delusions in Alzheimer's patients. However, the relatively non-selective nature of the compound gives rise to dose-limiting peripheral cholinergic side effects.

Certain M₁ receptor agonists are known, for example in WO2007036711. We have now found a novel group of compounds which are M₁ receptor agonists.

In a first aspect therefore, the invention provides a compound of formula (I) or a salt or solvate thereof: 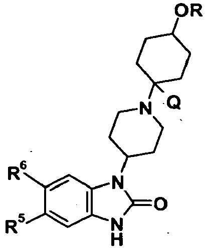 wherein:
- R⁵ is selected from halogen, cyano, C₁₋₆alkyl, C₁₋₆alkyl substituted with one or more fluorine atoms, C₁₋₆alkoxy, and C₁₋₆alkoxy substituted with one or more fluorine atoms;
- R⁶ is selected from hydrogen, halogen, cyano, C₁₋₆alkyl, C₁₋₆alkyl substituted with one or more fluorine atoms, C₁₋₆alkylsulfonyl, C₃₋₆cycloalkyl, C₃₋₆cycloalkyl substituted with one or more fluorine atoms, C₁₋₆alkoxy and C₁₋₆alkoxy substituted with one or more fluorine atoms;
- R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted with one or more fluorine atoms; and Q is hydrogen or C₁₋₆alkyl.

As used herein, the term "alkyl" refers to straight or branched hydrocarbon chains containing the specified number of carbon atoms. For example, C₁₋₆alkyl means a straight or branched alkyl containing at least 1, and at most 6, carbon atoms. C₁₋₃alkyl means a straight or branched alkyl containing at least 1, and at most 3, carbon atoms. Examples of "alkyl" as used herein include, but are not limited to, methyl, ethyl, n-propyl, n-butyl, n-pentyl, n-hexyl, isobutyl, isopropyl, t-butyl and 1,1-dimethylpropyl.

As used herein, the term "alkoxy" refers to a straight or branched alkoxy group containing the specified number of carbon atoms. For example, C₁₋₆alkoxy means a straight or branched alkoxy group containing at least 1, and at most 6, carbon atoms. Examples of "alkoxy" as used herein include, but are not limited to, methoxy, ethoxy, propoxy, prop-2-oxy, butoxy, but-2-oxy, 1-methylethyl-oxy, 2-methylprop-1-oxy, 2-methylprop-2-oxy, pentoxy or hexyloxy.

As used herein, the term "alkynyl" refers to a linear or branched hydrocarbon group containing one or more carbon-carbon triple bonds and the specified number of carbon atoms. For example, C₂₋₆alkynyl means a linear or branched hydrocarbon group containing one or more carbon-carbon triple bonds and at least two, and at most six, carbon atoms. Examples of "alkynyl" as used herein include, but are not limited to, include ethynyl, propynyl, butynyl, pentynyl and hexynyl.

As used herein, the term "cycloalkyl" refers to a non-aromatic hydrocarbon ring containing the specified number of carbon atoms. For example, C₃₋₆cycloalkyl means a non-aromatic ring containing at least three, and at most six, ring carbon atoms. Examples of "cycloalkyl" as used herein include, but are not limited to, cyclopropyl, cyclobutyl, cyclopentyl and cyclohexyl.

As used herein, the term "halogen" (or the abbreviated form "halo") refers to the elements fluorine (which may be abbreviated to "fluoro"), chlorine (which may be abbreviated to "chloro"), bromine (which may be abbreviated to "bromo")and iodine (which may be abbreviated to "iodo"). Examples of halogens are fluorine, chlorine and bromine.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include water, methanol, ethanol and acetic acid. The solvent used may be water and the solvate may also be referred to as a hydrate.

As used herein, the term "substituted" refers to substitution with the named substituent or substituents, multiple degrees of substitution being allowed unless otherwise stated. For example, there may be 1, 2, 3 or 4 substituents on a given group. For example there may be 3 fluoro groups on an R⁶ group. For example, there may be 1, 2, 3 or 4 substituents on a given substituted group. For example, if R⁶ is a C₁₋₆alkyl group, it may be substituted by 1, 2, 3 or 4 fluoro groups; and if R⁶ is a C₁₋₆alkoxy group, it may be substituted by 1, 2, 3 or 4 fluoro groups. For example, R⁶ may be a C₁₋₆alkyl group substituted by 3 fluoro groups; and R⁶ may be a C₁₋₆alkoxy group substituted by 3 fluoro groups. For example, R⁶ may be CF₃. Similarly, if R⁵ is a C₁₋₆alkyl group substituted by one or more fluoro groups, it may be substituted by 1, 2, 3 or 4 fluoro groups; and if R⁵ is a C₁₋₆alkoxy group, it may be substituted by 1, 2, 3 or 4 fluoro groups. For example, R⁵ may be a C₁₋₆alkyl group substituted by 3 fluoro groups; and R⁵ may be a C₁₋₆alkoxy group substituted by 3 fluoro groups. For example, R⁵ may be CF₃ or CH₂F.

In one embodiment, R⁵ is selected from halogen, cyano, C₁₋₆alkyl, C₁₋₆alkyl substituted with one, two or three fluorine atoms, C₁₋₆alkoxy, and C₁₋₆alkoxy substituted with one, two or three fluorine atoms.

In one embodiment, R⁵ is selected from halogen, cyano, C₁₋₄alkyl, C₁₋₄alkyl substituted with one, two or three fluorine atoms, C₁₋₄alkoxy, and C₁₋₄alkoxy substituted with one, two or three fluorine atoms.

In one embodiment, R⁵ is selected from halogen, cyano, C₁₋₂alkyl, C₁₋₂alkyl substituted with one, two or three fluorine atoms, C₁₋₂alkoxy, and C₁₋₂alkoxy substituted with one, two or three fluorine atoms.

In one embodiment of the invention, R⁵ is selected from chloro, bromo, fluoro, C₁₋₄alkoxy, C₁₋₄alkyl and C₁₋₄alkyl substituted with one or more fluorine atoms.

In a further embodiment of the invention, R⁵ is selected from chloro, bromo, fluoro, methyl, ethyl, methoxy and trifluoromethyl. In one embodiment, R⁵ is selected from chloro, fluoro and trifluoromethyl.

In one embodiment, R⁶ is selected from hydrogen, halogen, cyano, C₁₋₆alkyl, C₁₋₆alkyl substituted with one, two or three fluorine atoms, C₁₋₆alkylsulfonyl, C₃₋₆cycloalkyl, C₃-₅cycloalkyl substituted with one or more fluorine atoms, C₁₋₆alkoxy and C₁₋₆alkoxy and C₁₋₆alkoxy substituted with one, two or three fluorine atoms.

In one embodiment, R⁶ is selected from hydrogen, halogen, cyano, C₁₋₄alkyl, C₁₋₄alkyl substituted with one, two or three fluorine atoms, C₁₋₄alkylsulfonyl, C₃₋₆cycloalkyl, C₃-₆cycloalkyl substituted with one or more fluorine atoms, C₁₋₄alkoxy and C₁₋₄alkoxy substituted with one, two or three fluorine atoms.

In one embodiment, R⁶ is selected from hydrogen, halogen, cyano, C₁₋₂alkyl, C₁₋₂alkyl substituted with one, two or three fluorine atoms, C₁₋₂alkylsulfonyl, C₃₋₆cycloalkyl, C₃₋₆cycloalkyl substituted with one or more fluorine atoms, C₁₋₂alkoxy and C₁₋₂alkoxy substituted with one, two or three fluorine atoms.

In one aspect of the invention, R⁶ is selected from chloro, bromo, fluoro, methyl, ethyl, isopropyl, methoxy, trifluoromethoxy and trifluoromethyl.

In a further aspect of the invention, R⁶ is selected from chloro, bromo, methyl, ethyl, isopropyl, methoxy, trifluoromethoxy and trifluoromethyl. In one embodiment, R⁶ is selected from methyl and bromo.

In one embodiment, R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted with one, two or three fluorine atoms.

In one embodiment, R is selected from C₁₋₄alkyl, C₃₋₆cycloalkyl, C₃₋₆cycloalkylC₁₋₄alkyl and C₂₋₄alkynyl, any alkyl or cycloalkyl group being optionally substituted with one, two or three fluorine atoms.

In one embodiment of the invention, R is selected from C₁₋₆alkyl, C₃₋₆cycloalkyl, C₃₋₆cycloalkylC₁₋₆alkyl, any alkyl group being optionally substituted with one or more fluorine atoms.

In a further embodiment of the invention, R is selected from C₁₋₃alkyl and C₃₋₆cycloalkylC₁₋₃alkyl, any alkyl or cycloalkyl group (for example any alkyl group) being optionally substituted with one or more fluorine atoms.

In a further embodiment, R is selected from methyl, ethyl, propyl, isopropyl and cyclopropylmethyl. For example, R may be selected from methyl, ethyl and propyl.

In one embodiment of the invention Q is selected from hydrogen and C₁₋₃alkyl. In a further embodiment, Q is selected from hydrogen, methyl, ethyl and propyl. For example, Q represents hydrogen or methyl.

In one embodiment, in formula (I) above, R⁵ is selected from chloro, bromo, fluoro, C₁- ₄alkyl, C₁₋₄alkyl substituted with one or more fluorine atoms, and C₁₋₄alkoxy, R⁶ is selected from chloro, bromo, methyl, ethyl, isopropyl, methoxy, trifluoromethoxy and trifluoromethyl, R is selected from methyl, ethyl, propyl, isopropyl and, cyclopropylmethyl, and Q is selected from hydrogen and methyl.

The present invention also provides a compound of formula (Ia): 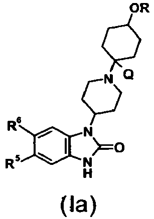 wherein:
- R⁵ is selected from halogen, C₁₋₆alkyl, C₁₋₆alkyl substituted with one or more fluorine atoms, C₁₋₆ alkoxy, and C₁₋₆ alkoxy substituted with one or more fluorine atoms;
- R⁶ is selected from halogen, C₁₋₆alkyl, C₁₋₆alkyl substituted with one or more fluorine atoms, C₃₋₆cycloalkyl, C₃₋₆cycloalkyl substituted with one or more fluorine atoms, C₁₋₆ alkoxy and C₁₋₆ alkoxy substituted with one or more fluorine atoms;
R is selected from C₁₋₆alkyl, C₃₋₆cycloalkyl, C₃₋₆cycloalkylC₁₋₆alkyl, any alkyl or cycloalkyl group being optionally substituted with one or more fluorine atoms; and
or a pharmaceutically acceptable salt or solvate thereof.

In one embodiment the salt or solvate of the compound of formula (I) is a pharmaceutically acceptable salt or solvate. In one embodiment, the invention provides a compound of formula (I), or a pharmaceutically acceptable salt or solvate thereof.

It will be appreciated that for use in medicine the salts of formula (I) should be pharmaceutically acceptable. Suitable salts will be apparent to those skilled in the art and include for example mono- or di- basic salts formed with inorganic acids e.g. hydrochloric, hydrobromic, sulfuric, nitric, sulfamic phosphoric, hydroiodic, phosphoric or metaphosphoric acid; and with organic acids, such as tartaric, acetic, trifluoroacetic, citric, malic, lactic, fumaric, benzoic, formic, propionic, glycolic, gluconic, maleic, succinic, (1S)-(-)-10-camphorsulphonic, (1S)-(+)-10-camphorsulphonic, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example naphthalene-1,5-disulphonic, naphthalene-1,3-disulphonic, benzenesulfonic, and p-toluenesulfonic, acids. Other non-pharmaceutically acceptable salts e.g. oxalates, may be used, for example in the isolation of compounds of formula (I) and are included within the scope of this invention. The compounds of the present invention may be in the form of their free base or pharmaceutically acceptable salts thereof, particularly the monohydrochloride, monoformate or monotrifluoroacetate salts.

Certain of the compounds of formula (I) may form acid addition salts with less than one (for example, 0.5 equivalent of a dibasic acid) or one or more equivalents of an acid. The present invention includes within its scope all possible stoichiometric and non-stoichiometric forms thereof.

It will be appreciated that compounds of formula (I) can exist in *cis* or *trans* isomeric forms (the OR group on the cyclohexane ring in relation to the piperidine substituent).

*Cis* form:

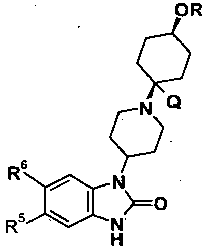

It will be appreciated that the *trans* form may be drawn in the following different ways, although both represent the same isomeric form:

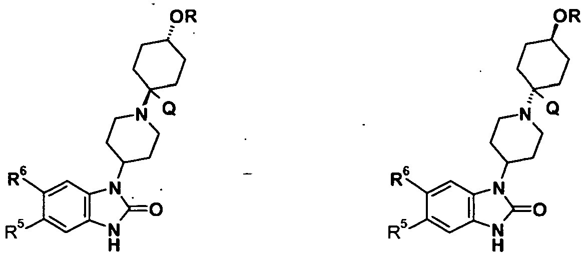

The individual isomers (*cis* and *trans*) and mixtures of these are included within the scope of the present invention. The isomers may be separated one from the other by the usual methods or by methods detailed for the example compounds below. Any given isomer may also be obtained by stereospecific or asymmetric synthesis. The invention also extends to any tautomeric forms and mixtures thereof.

In one embodiment, the compounds of formula (I) are *trans* isomers

In another embodiment, the compounds of formula (I) are *cis* isomers.

Mixtures of *cis-* and *trans-* compounds, or compounds in which the cis/trans conformation have not been determined, are drawn herein as shown below:

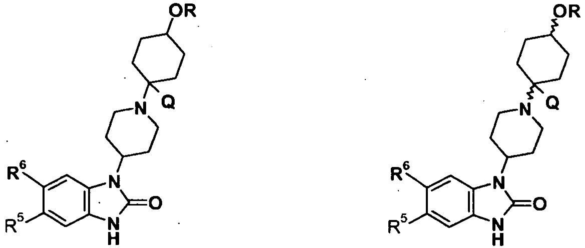

Compounds according to the invention include those specifically exemplified in the Examples section and named hereinafter including, without limitation:-
1. 5-Fluoro-6-methyl-1-{1-[*cis*-4-(methyloxy)cyclohexyl]-4-piperidinyl}-1,3-dihydro-2*H-*benzimidazol-2-one
2. 5-Fluoro-6-methyl-1-{1-[*trans*-4-(methyloxy)cyclohexyl]-4-piperidinyl}-1,3-dihydro-2*H-*benzimidazol-2-one
3. 5-Chloro-6-methyl-1-{1-[*cis*-4-(methyloxy)cyclohexyl]-4-piperidinyl}-1,3-dihydro-*2H-*benzimidazol-2-one
4. 5-Chloro-6-methyl-1-{1-[*trans*-4-(methyloxy)cyclohexyl]-4-piperidinyl}-1,3-dihydro-*2H-*benzimidazol-2-one
5. 6-Bromo-1-{1-[*cis*-4-(methyloxy)cyclohexyl]-4-piperidinyl}-5-(trifluoromethyl)-1,3-dihydro-2*H*-benzimidazol-2-one
6. 6-Bromo-1-{1-[*trans*-4-(methyloxy)cyclohexyl]-4-piperidinyl}-5-(trifluoromethyl)-1,3-dihydro-2*H*-benzimidazol-2-one
7. 1-{1-[*trans*-4-(Ethyloxy)cyclohexyl]-4-piperidinyl}-5-fluoro-6-methyl-1 ,3-dihydro-2*H-*benzimidazol-2-one
8. 5-Chloro-6-methyl-1-{1-[*cis*-4-(propyloxy)cyclohexyl]-4-piperidinyl}-1,3-dihydro-2*H-*benzimidazol-2-one
9. 5-Chloro-6-methyl-1-{1-[*trans*-4-(propyloxy)cyclohexyl]-4-piperidinyl}-1,3-dihydro-2*H-*benzimidazol-2-one
10. 5-Fluoro-6-methyl-1-{1-[*cis*-4-(propyloxy)cyclohexyl]-4-piperidinyl}-1,3-dihydro-2*H-*benzimidazol-2-one
11. 5-Fluoro-6-methyl-1-{1-[*trans*-4-(propyloxy)cyclohexyl]-4-piperidinyl}-1,3-dihydro-2*H-*benzimidazol-2-one
12. 6-Bromo-1-{1-[*cis*-4-(propyloxy)cyclohexyl]-4-piperidinyl}-5-(trifluoromethyl)-1,3-dihydro-2*H*-benzimidazol-2-one
13.6-Bromo-1-{1-[*trans*-4-(propyloxy)cyclohexyl]-4-piperidinyl}-5-(trifluoromethyl)-1,3-dihydro-2*H-*benzimidazol-2-one
and salts and solvated thereof, for example the hydrochloride salt, the trifluoroacetate salt or the formate salt.

Further example compounds include:
1. *cis* 1-{1-[4-(Ethyloxy)cyclohexyl]-4-piperidinyl}-5,6-dimethyl-1,3-dihydro-2*H-*benzimidazol-2-one
2. *trans* 1-{1-[4-(Ethyloxy)cyclohexyl]-4-piperidinyl}-5,6-dimethyl-1,3-dihydro-2*H-*benzimidazol-2-one
3. 5-Bromo-6-methyl-1-[1-(*trans-*4-ethoxycyclohexyl)-4-piperidinyl]-1,3-dihydro-2*H-*benzimidazol-2-one
4. 5-Chloro-1-{1-[*trans*-4-(ethyloxy)cyclohexyl]-4-piperidinyl}-6-methyl-1,3-dihydro-2*H-*benzimidazol-2-one
5. 6-Chloro-5-methyl-1-[1-(*trans*-4-ethoxycyclohexyl)-4-piperidinyl]-1,3-dihydro-2*H-*benzimidazol-2-one
6. 6-Chloro-1-{1-[4-(ethyloxy)cyclohexyl]-4-piperidinyl}-5-methyl-1,3-dihydro-2*H-*benzimidazol-2-one
7. 5-Bromo-6-methyl-1-[1-(*trans*-4-propoxycyclohexyl)-4-piperidinyl]-1,3-dihydro-2*H-*benzimidazol-2-one
8. 6-Chloro-1-{1-[*trans*-4-(propyloxy)cyclohexyl]-4-piperidinyl}-5-(trifluoromethyl)-1,3-dihydro-2*H*-benzimidazol-2-one
9. 5-Fluoro-6-methyl-1-(1-{*trans*-4-[(1-methylethyl)oxy]cyclohexyl}-4-piperidinyl)-1,3-dihydro-2*H*-benzimidazol-2-one
10. 5-Fluoro-6-methyl-1-{1-[*trans*-1-methyl-4-(methyloxy)cyclohexyl]-4-piperidinyl}-1,3-dihydro-2*H*-benzimidazol-2-one
11. 1-{1-[*trans*-4-(Ethyloxy)-1-methylcyclohexyl]-4-piperidinyl}-5-fluoro-6-methyl-1,3-dihydro-2*H*-benzimidazol-2-one
12. 5-Chloro-1-{1-[*trans*-4-(ethyloxy)-1-methylcyclohexyl]-4-piperidinyl}-6-methyl-1,3-dihydro-2*H*-benzimidazol-2-one
13. 5-Fluoro-6-methyl-1-{1-[*cis*-1-methyl-4-(propyloxy)cyclohexyl]-4-piperidinyl}-1,3-dihydro-2*H*-benzimidazol-2-one
14. 5-Fluoro-6-methyl-1-{1-[*trans*-1-methyl-4-(propyloxy)cyclohexyl]-4-piperidinyl}-1,3-dihydro-2*H*-benzimidazol-2-one hydrochloride
15. 5-Chloro-6-methyl-1-{1-[*cis*-1-methyl-4-(propyloxy)cyclohexyl]-4-piperidinyl}-1,3-dihydro-2*H*-benzimidazol-2-one
16. 5-Chloro-6-methyl-1-{1-[*trans*-1-methyl-4-(propyloxy)cyclohexyl]-4-piperidinyl}-1,3-dihydro-2*H*-benzimidazol-2-one
17. 6-Bromo-1-{1-[*trans*-1-methyl-4-(propyloxy)cyclohexyl]-4-piperidinyl}-5-(trifluoromethyl)-1,3-dihydro-2*H*-benzimidazol-2-one
18. 1-{1-[*trans*-4-(Ethyloxy)-1-methylcyclohexyl]-4-piperidinyl}-5,6-dimethyl-1,3-dihydro-2*H*-benzimidazol-2-one
19. 1-{1-[*tran*s-4-(Ethyloxy)-1-methylcyclohexyl]-4-piperidinyl}-5,6-difluoro-1,3-dihydro-2*H-*benzimidazol-2-one
20. 5-Chloro-1-{1-[*trans*-1-methyl-4-(methyloxy)cyclohexyl]-4-piperidinyl}-1,3-dihydro-2*H-*benzimidazol-2-one
21. 5-Chloro-1-{1-[*trans*-4-(ethyloxy)-1-methylcyclohexyl]-4-piperidinyl}-1,3-dihydro-2*H-*benzimidazol-2-one
22. 6-Chloro-3-{1-[*trans*-4-(ethyloxy)cyclohexyl]-4-piperidinyl}-2-oxo-2,3-dihydro-1*H-*benzimidazole-5-carbonitrile
23. 5,6-Dimethyl-1-{1-[*trans*-1-methyl-4-(methyloxy)cyclohexyl]-4-piperidinyl}-1,3-dihydro-2*H*-benzimidazol-2-one
24. 5,6-Difluoro-1-{1-[*trans*-1-methyl-4-(methyloxy)cyclohexyl]-4-piperidinyl}-1,3-dihydro-2*H*-benzimidazol-2-one
25. 6-chloro-3-{1-[*trans*-1-methyl-4-(methyloxy)cyclohexyl]-4-piperidinyl}-2-oxo-2,3-dihydro-1*H*-benzimidazole-5-carbonitrile
26. 6-Chloro-3-{1-[*trans*-4-(ethyloxy)-1-methylcyclohexyl]-4-piperidinyl}-2-oxo-2,3-dihydro-1*H*-benzimidazole-5-carbonitrile
and salts and solvated thereof, for example the hydrochloride salt, the trifluoroacetate salt or the formate salt.

It will be appreciated by those skilled in the art that certain protected derivatives of compounds of formula (I), which may be made prior to a final deprotection stage, may not possess pharmacological activity as such, but may, in certain instances, be administered orally or parenterally and thereafter metabolised in the body to form compounds of the invention which are pharmacologically active. Such derivatives may therefore be described as "prodrugs". Further, certain compounds of the invention may act as prodrugs of other compounds of the invention. Examples of suitable protecting groups for the compounds of the present invention are described in Drugs of Today, Volume 19, Number 9, 1983, pp 499 - 538 and in Topics in Chemistry, Chapter 31, pp 306 - 316 and in "Design of Prodrugs" by H. Bundgaard, Elsevier, 1985, Chapter 1 . It will further be appreciated by those skilled in the art, that certain moieties, known to those skilled in the art as "pro-moieties", for example as described by H. Bundgaard in "Design of Prodrugs" may be placed on appropriate functionalities when such functionalities are present within compounds of the invention. Suitable prodrugs for compounds of the invention include : esters, carbonate esters, hemi-esters, phosphate esters, nitro esters, sulfate esters, sulfoxides, amides, carbamates, azo-compounds, phosphamides, glycosides, ethers, acetals and ketals.

In a further aspect, the invention provides a general process (A1) for preparing compounds of formula (I) in which Q =H, which process comprises:
coupling a compound of formula (II) 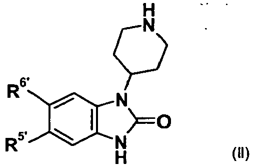 with a compound of formula (III) 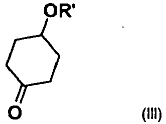 wherein
   R^{5'} is a group R⁵ as previously defined, or a group convertible to R⁵, R^{6'} is a group R⁶ as previously defined, or a group convertible to R⁶, and R' is a group R as previously defined, or a group convertible to R.

The reaction is carried out under conditions suitable for reductive alkylation. The reductive alkylation reaction is typically carried out using sodium triacetoxyborohydride in dichloroethane, optionally in the presence of triethylamine, and optionally in the presence of titanium tetraisopropoxide. Alternatively sodium cyanoborohydride can be used as the reducing reagent in solvents such as methanol or ethanol, or the reductive alkylation can be effected under catalytic hydrogenation conditions using a palladium catalyst. In a further variation, the compounds (II) and (III) can be condensed under dehydrating conditions e.g. molecular sieves or magnesium sulfate, and the resultant imine or enamine reduced using for example sodium borohydride or by catalytic hydrogenation. This reaction can generate a mixture of *cis* and *trans* isomers which can be separated by chromatography or crystallisation.

A modification of general process (A1) is required where Q is C₁₋₆ alkyl. Thus, in general process (A2), a compound of formula (II) can be reacted with a compound of formula (III) in the presence of a source of cyanide, e.g. acetone cyanohydrin, to form the cyano intermediate (XXXX) which can be reacted with an alkyl Grignard reagent QMgX to form compounds of formula (I) in which Q is C₁₋₆ alkyl. 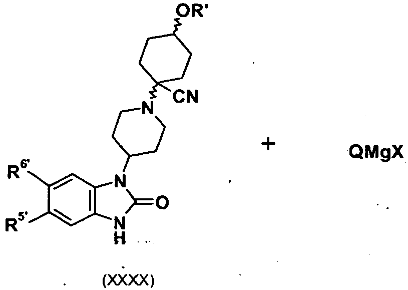 wherein
R^{5'} is a group R⁵ as previously defined, or a group convertible to R⁵, R^{6'} is a group R⁶ as previously defined, or a group convertible to R⁶, and R' is a group R as previously defined, or a group convertible to R, Q is C₁₋₆ alkyl, and X is bromo or iodo or chloro.

This reaction can generate a mixture of *cis* and *trans* isomers which can be separated by chromatography or crystallisation.

In a further aspect, the invention provides a general process (B) for preparing compounds of formula (I) which process comprises:
coupling a compound of formula (IV) 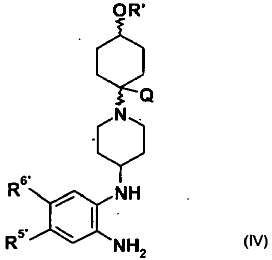 with a compound of formula (V) 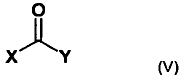 wherein R^{5'} is a group R⁵ as previously defined, or a group convertible to R⁵, R^{6'} is a group R⁶ as previously defined, or a group convertible to R⁶, R' is a group R as previously defined, or a group convertible to R, Q is as previously defined, and X and Y both represent leaving groups. X and Y can be the same or different and examples are Cl, PhO, EtO, imidazole. When X and Y are both Cl, i.e. phosgene, this reagent can be generated *in situ* e.g. from diphosgene or triphosgene.

The above reaction is carried out using standard methodology e.g. reacting the diamine (IV) with the reagent (V) in an inert solvent for example dichloromethane or toluene, optionally in the presence of a base such as triethylamine or potassium carbonate, and optionally with heating.

It will be appreciated that compounds of formula (IV) can be pure *cis* or *trans* isomers, or a mixture of isomers. If necessary, separation of pure *cis* and *trans* isomers after the reaction with (V) can be achieved by chromatography or crystallisation.

In a further aspect, the invention provides a general process (C) for preparing compounds of formula (I) which process comprises:
treatment of a compound of formula (VI) 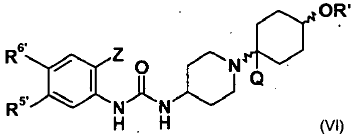 with a palladium or copper catalyst (VII) to effect an intramolecular cyclisation
   wherein R^{5'} is a group R⁵ as previously defined, or a group convertible to R⁵, R^{6'} is a group R⁶ as previously defined, or a group convertible to R⁶, R' is a group R as previously defined, or a group convertible to R, Q is as previously defined, and Z is a leaving group such as bromo, iodo, chloro or triflate.

The cyclisation reaction can be carried out using a variety of palladium or copper reagents as described in the literature (JACS, 2003, 125, 6653, Tet. Lett., 2004, 45, 8535, or JACS, 2002, 124, 7421.)

It will be appreciated that compounds of formula (VI) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (D) for preparing compounds of formula (I) which process comprises:
coupling a compound of formula (VIII) 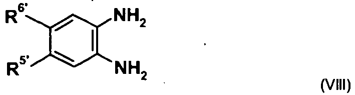 with a compound of formula (IX) 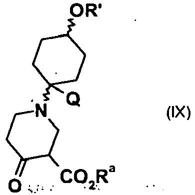 wherein R^{5'} is a group R⁵ as previously defined, or a group convertible to R⁵, R^{6'} is a group R⁶ as previously defined, or a group convertible to R⁶, R' is a group R as previously defined, or a group convertible to R, Q is as previously defined, and R^{a} is a C₁₋₅ alkyl group.

The condensation and cyclisation reactions can be carried out under reaction conditions similar to those described in the literature for an analogous process (US 3161645) (for example heating in an inert solvent such as xylene) followed by reduction of the piperidine double bond using for example catalytic hydrogenation over palladium or Raney nickel. It will be appreciated that compounds of formula (IX) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (E) for preparing compounds of formula (I) which process comprises:
reaction of a compound of formula (X) 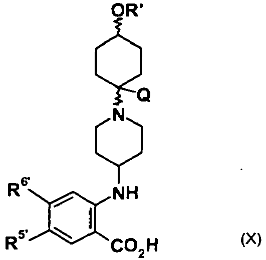 with diphenylphosphoryl azide or other reagent/combination of reagents to effect the Curtius rearrangement of compound (X), followed by intramolecular cyclisation.
   wherein R^{5'} is a group R⁵ as previously defined, or a group convertible to R⁵, R^{6'} is a group R⁶ as previously defined, or a group convertible to R⁶, R' is a group R as previously defined, or a group convertible to R, and Q is as previously defined.

The Curtius rearrangement is typically carried out by mixing the two reactants in an inert solvent such as toluene, optionally with heating.

It will be appreciated that compounds of formula (X) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (F) for preparing compounds of formula (I) which process comprises:
coupling a compound of formula (XI) 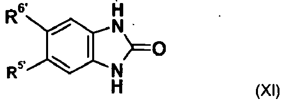 with a compound of formula (XII) 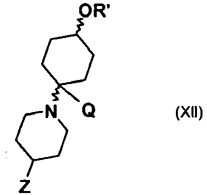 wherein R^{5'} is a group R⁵ as previously defined, or a group convertible to R⁵, R^{6'} is a group R⁶ as previously defined, or a group convertible to R⁶, R' is a group R as previously defined, or a group convertible to R, Q is as previously defined, and Z is hydroxy or a leaving group such as chloro, bromo or iodo, or alkyl/aryl sulfonate.

The alkylation reaction can be carried out under classical alkylation (Z = a leaving group) or Mitsunobu reaction (Z = OH) conditions. Using classical alkylation conditions, the benzimidazolone intermediate (XI) can be deprotonated using a base such as sodium hydride in an inert solvent such as dimethylformamide, and then treated with the alkylating reagent (XII), optionally with heating. The Mitsunobu reaction with (XII) Z = OH can be carried out using standard conditions e.g. triphenylphosphine and diethylazodicarboxylate in an inert solvent such as dichloromethane or tetrahydrofuran at room temperature.

It will be appreciated that compounds of formula (X) can be pure *cis* or *trans* isomers, or a mixture of isomers. If necessary, separation of pure *cis* and *trans* isomers after the intramolecular cyclisation can be achieved by chromatography or crystallisation.

Conversion of R^{6'} to R⁶ or interconversions of R⁶ may be accomplished as indicated below.

For example, when R^{6'} is a halogen, it can be converted to an alkoxy or trifluoromethyl group by copper catalysed reaction, using an alcohol, or methyl fluorosulfonyl(difluoro)acetate, respectively. It may also be converted to an alkyl group with an organometallic reagent, for example an alkylstannane.

As another example, when R^{6'} is hydroxy, it may be converted to alkoxy by reaction with an alkyl halide or sulfonate, or to trifluoromethoxy by conversion to the xanthate followed by oxidation in the presence of fluoride ion.

As a further example, when R^{6'} is methyl, it may be converted to trifluoromethyl by chlorination or bromination followed by displacement of the introduced halogens with fluoride.

Similarly, conversion of R^{5'} to R⁵ or interconversions of R⁵ may be accomplished as described for R⁶.

Conversion of R to R or interconversions of R may be accomplished as indicated below. For example when R' is benzyl, the benzyl group can be removed using standard methodology, e.g. catalytic hydrogenation over palladium on carbon, to provide the alcohol. Alkylation of the resultant alcohol using a strong base e.g. sodium hydride and a C₁₋₆ alkylating agent e.g. methyl iodide or ethyl iodide or propyl iodide, will afford the desired product. It will be appreciated that protection of any NH functionality present in the molecule may be necessary

As another example, when R is methyl, the methyl group can be removed by treatment with a dealkylating agent such as boron tribromide to afford the alcohol intermediate, which can be alkylated in a similar manner to that described above.

Compounds of formula (II) are generally known in the literature or can be prepared by a range of different processes for example:
(a) displacement of an ortho-fluoro or ortho-chloro nitrobenzene intermediate (XIII) with the amine (XIV), wherein R^{5'} is a group R⁵ as previously defined, or a group convertible to R⁵, R^{6'} is a group R⁶ as previously defined, or a group convertible to R⁶, and P represents a nitrogen protecting group e.g. Boc, acetyl, trifluoroacetyl, ethoxycarbonyl, benzyloxycarbonyl, to give (XXIII), followed by reduction of the nitro group, cyclisation using phosgene or a phosgene equivalent, and deprotection of the piperidine nitrogen using standard literature conditions (Scheme 1). 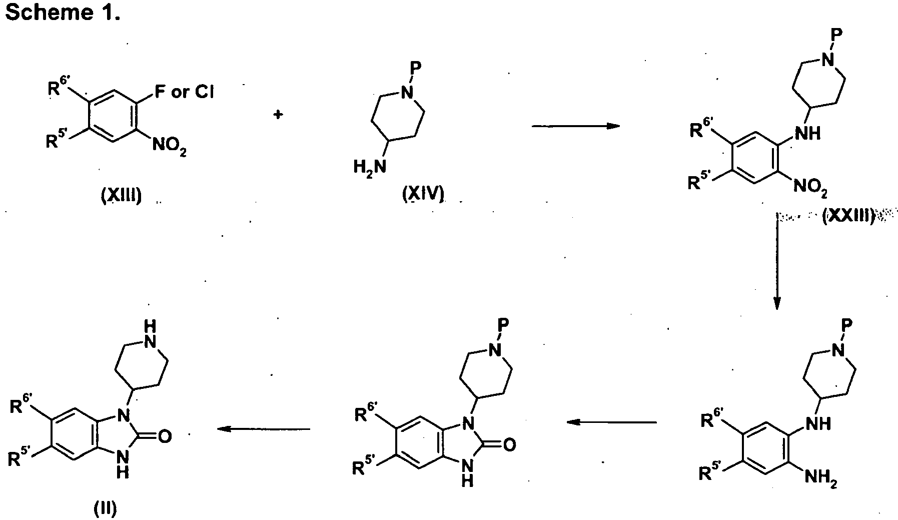 Compounds of formula (XIII) are commercially available or can be prepared by standard methodology. The compound (XIV) in which P = Boc is commercially available
(b) metal catalysed cyclisation of an intermediate (XV) followed by deprotection of the piperidine nitrogen, wherein R^{5'} is a group R⁵ as previously defined, or a group convertible to R⁵, R^{6'} is a group R⁶ as previously defined, or a group convertible to R⁶, P represents a nitrogen protecting group e.g. Boc, acetyl, trifluoroacetyl, benzyloxycarbonyl, and Z represents a leaving group such as bromo, iodo, chloro or triflate. Reaction conditions for the metal catalysed cyclisation are summarised in Process C. The urea (XV) can be prepared using any of the classical methods for urea formation as illustrated in Scheme 2. The starting materials for this process are commercially available or can be prepared using standard methodology. 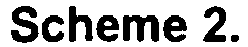 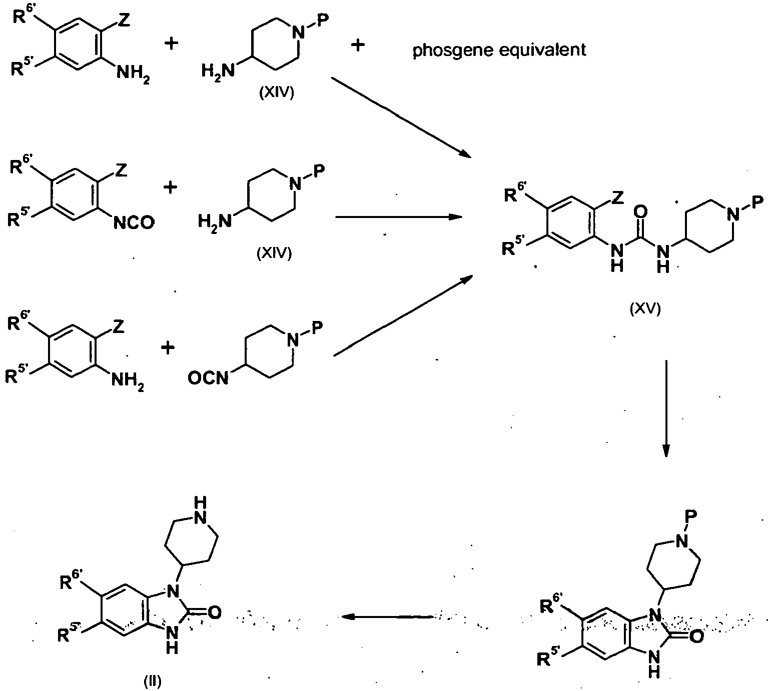
(c) Curtius rearrangement of an intermediate (XVI), wherein R^{5'} is a group R⁵ as previously defined, or a group convertible to R⁵, R^{6'} is a group R⁶ as previously defined, or a group convertible to R⁶, P represents a nitrogen protecting group e.g. Boc, acetyl, trifluoroacetyl, benzyloxycarbonyl, and R^{b} represents H or a C₁₋₅ alkyl group e.g. methyl or ethyl, followed by intramolecular cyclisation and deprotection of the piperidine nitrogen (Scheme 3). The anthranilic acid or ester starting materials (XVII) are commercially available or can be made by standard methodology. The piperidone starting material (P = Boc or benzyl) is commercially available. The Curtius rearrangement can be effected using the conditions described under process E. 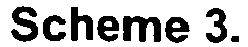 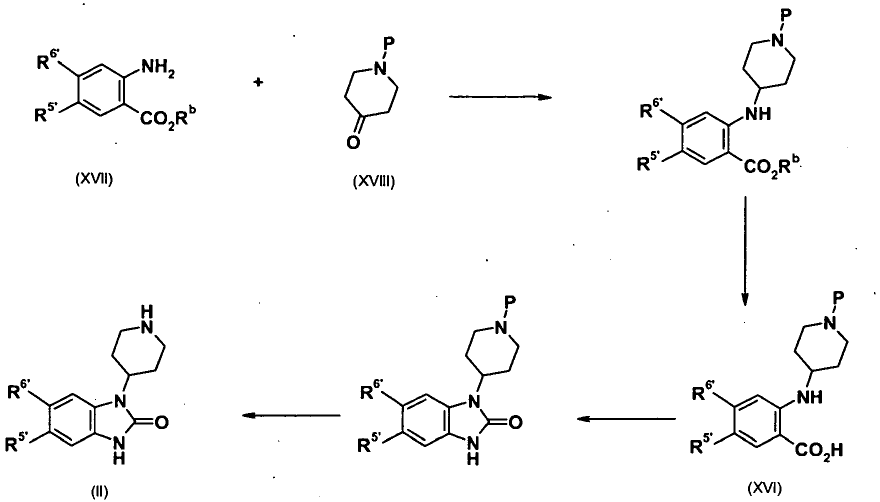
(d) Condensation of an orthophenylenediamine (VIII) with a 3-alkoxycarbonyl-4-piperidone (XX), wherein R^{5'} is a group R⁵ as previously defined, or a group convertible to R⁵, R^{6'} is a group R⁶ as previously defined, or a group convertible to R⁶, P represents a nitrogen protecting group e.g. Boc, acetyl, trifluoroacetyl, benzyloxycarbonyl and R^{b} is a C₁₋₅ alkyl group (Scheme 4), by heating in an inert solvent at elevated temperature, to afford the tetrahydropyridine intermediate (XXI). Hydrogenation of the double bond and deprotection of the piperidine nitrogen can be accomplished separately or concomitantly dependent on the precise nature of the protecting group P, to afford the desired product (II). Compounds of formula (VIII) are commercially available or can be prepared by standard methodology. Compounds of formula (XX) are commercially available or can be prepared by standard methodology. 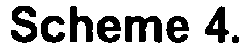 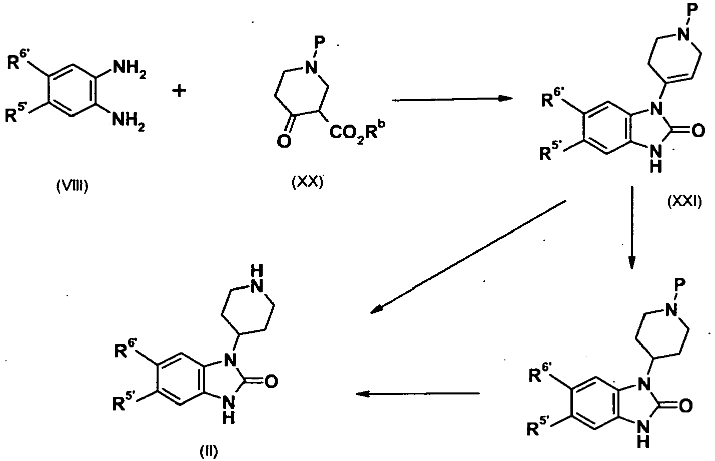
(e) Reductive alkylation of an ortho nitroaniline (XXII) with an N-protected 4-piperidone (XVIII), wherein R^{5'} is a group R⁵ as previously defined, or a group convertible to R⁵, R^{6'} is a group R⁶ as previously defined, or a group convertible to R⁶, and P represents a nitrogen protecting group e.g. Boc, acetyl, trifluoroacetyl benzyloxycarbonyl using for example sodium triacetoxyborohydride to give the intermediate (XXIII). Reduction of the nitro group, followed by cyclisation and deprotection as described hereinbefore provides the desired product (II) (Scheme 5). Compounds of formula (XXII) and (XVIII) are commercially available or can be prepared by standard methodology 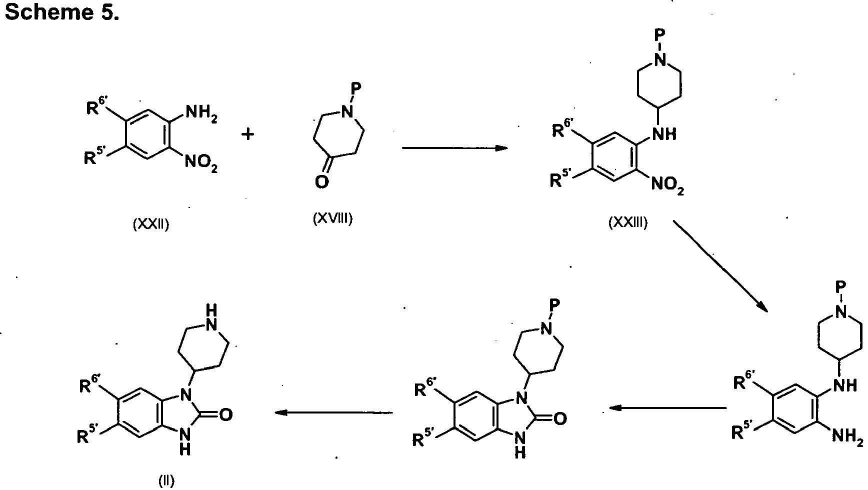
(f) metal catalysed reaction between the amine (XIV) and a suitably substituted nitrobenzene compound (XXIV) wherein R^{5'} is a group R⁵ as previously defined, or a group convertible to R⁵, R^{6'} is a group R⁶ as previously defined, or a group convertible to R⁶, P represents a nitrogen protecting group e.g. Boc, acetyl, trifluoroacetyl, benzyloxycarbonyl, and Z represents a leaving group such as bromo, iodo, chloro or triflate (Scheme 6). This process generates intermediates of formula (XXIII) and subsequent reactions are similar to that for Scheme 5. Compounds of formula (XXIV) are commercially available or can be prepared by known methodology. The compound (XIV) in which P = Boc is commercially available 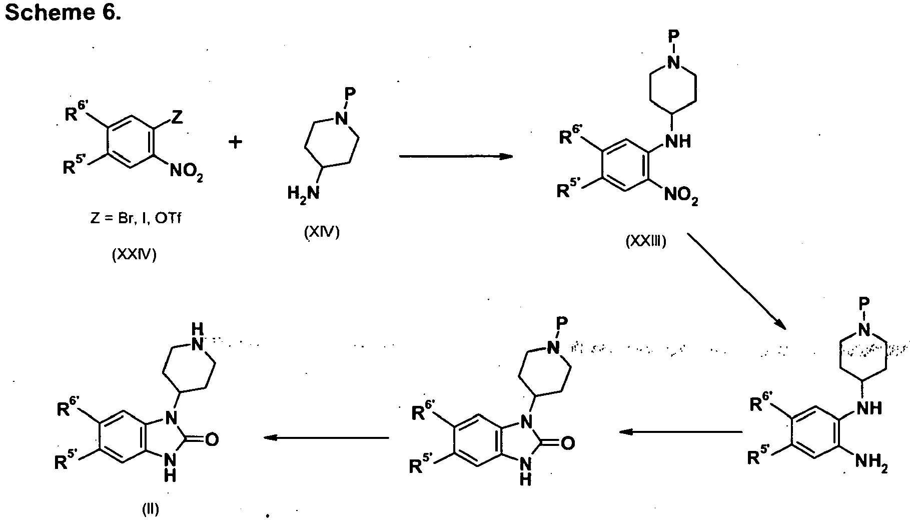
(g) metal catalysed reaction between the amine (XIV) and the protected aniline (XXV), wherein R^{5'} is a group R⁵ as previously defined, or a group convertible to R⁵, R^{6'} is a group R⁶ as previously defined, or a group convertible to R⁶, P and P' independently represent a nitrogen protecting group e.g. Boc, acetyl, trifluoroacetyl, benzyloxycarbonyl, and Z represents a leaving group such as bromo, iodo, chloro or triflate, to give the intermediate (XXVI) (Scheme 7). Deprotection of the aniline followed by the same reaction sequence as in Scheme 6 affords the desired intermediate (II). Compounds of formula (XXV) are commercially available or can be prepared by known methodology e.g. halogenation ortho to the optionally protected aniline group. The compound (XIV) in which P = Boc is commercially available.

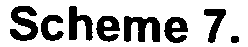

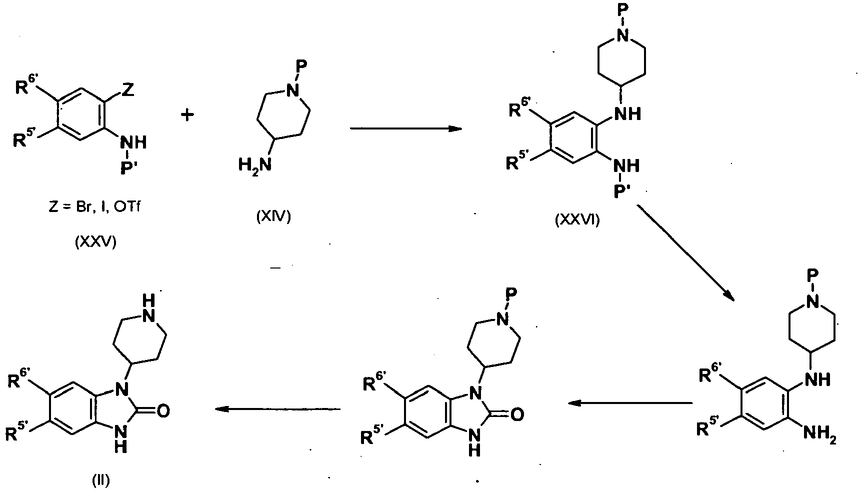

The compounds of formula (III) can be prepared by standard literature methodology.

Compounds of formula (IV) can be prepared by a number of different processes e.g.
(h) displacement of an ortho-fluoro or ortho-chloro nitrobenzene intermediate (XIII) with the amine (XXVII) wherein R^{5'} is a group R⁵ as previously defined, or a group convertible to R⁵, R^{6'} is a group R⁶ as previously defined, or a group convertible to R⁶, R' is a group R as previously defined, or a group convertible to R, and Q is as previously defined, to afford compound (XXVIII) followed by reduction of the nitro group using standard conditions e.g. hydrogenation over palladium or Raney nickel (Scheme 8). Compounds of formula (XIII) are commercially available or can be prepared by standard methodology. It will be appreciated that separation of the *cis* and *trans* isomers can be achieved at any suitable stage in the synthesis. 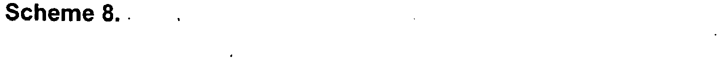 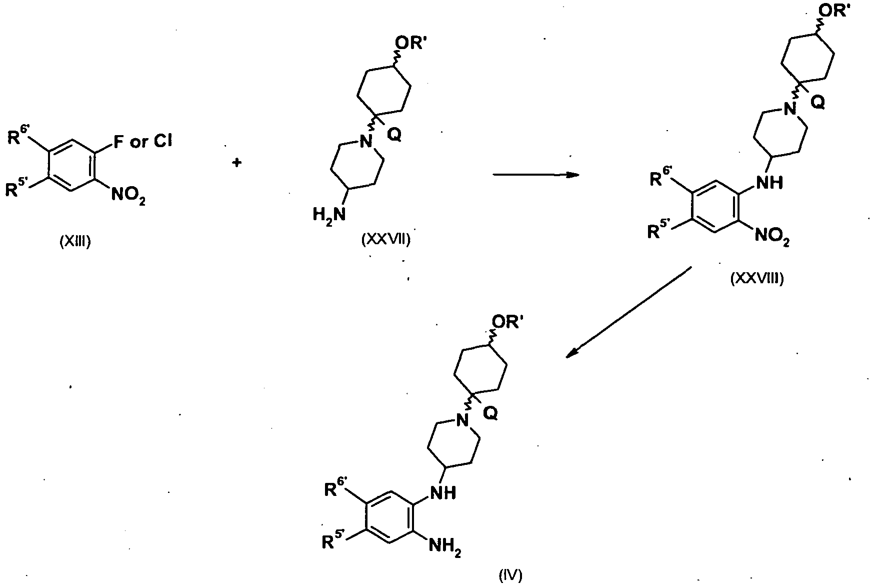
(i) metal catalysed reaction of the amine (XXVII) with the ortho substituted nitrobenzene (XXIX), wherein R^{5'} is a group R⁵ as previously defined, or a group convertible to R⁵, R^{6'} is a group R⁶ as previously defined, or a group convertible to R⁶, R' is a group R as previously defined, or a group convertible to R, and Q is as previously defined, to afford compound (XXVIII) (Scheme 9) followed by the same reactions as illustrated in Scheme 8. Compounds of formula (XXIX) are commercially available or can be prepared by standard methodology. It will be appreciated that separation of the *cis* and *trans* isomers can be achieved at any suitable stage in the synthesis. 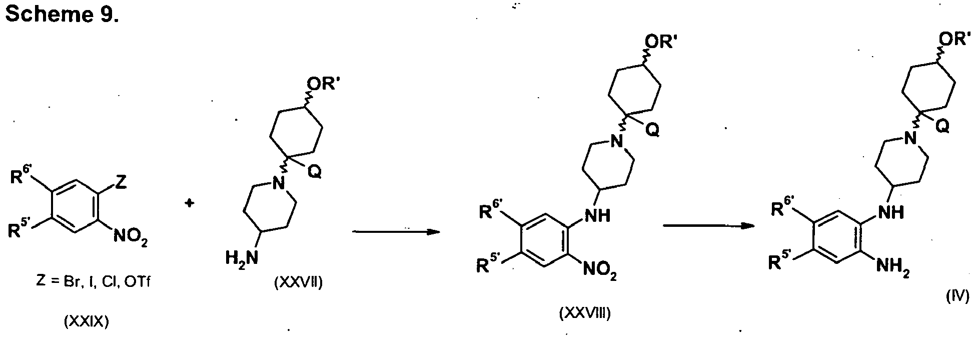
(j) metal catalysed reaction of the amine (XXVII) with the protected aniline derivative (XXV), wherein R^{5'} is a group R⁵ as previously defined, or a group convertible to R⁵, R^{6'} is a group R⁶ as previously defined, or a group convertible to R⁶, R' is a group R as previously defined, or a group convertible to R, and Q is as previously defined, and P' represents a nitrogen protecting group such as acetyl, trifluoroacetyl, Boc, phthalimide, to afford compound (XXXI) (Scheme 10) followed by deprotection of the aniline group. Compounds of formula (XXV) are commercially available or can be prepared by standard methodology. It will be appreciated that separation of the *cis* and *trans* isomers can be achieved at any suitable stage in the synthesis. 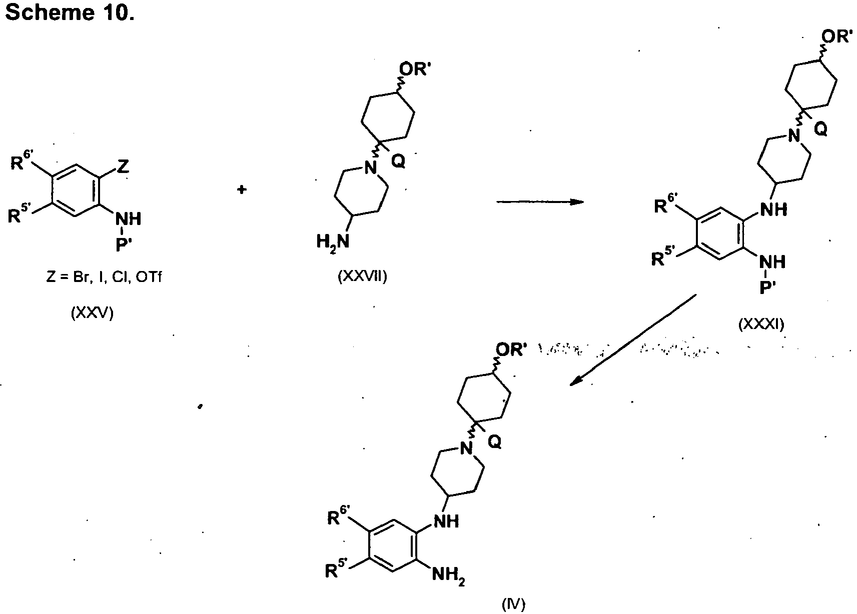
(k) Reductive alkylation of an ortho nitroaniline (XXII) with the piperidone (XXXII) wherein R^{5'} is a group R⁵ as previously defined, or a group convertible to R⁵, R^{6'} is a group R⁶ as previously defined, or a group convertible to R⁶, R' is a group R as previously defined, or a group convertible to R, and Q is as previously defined, using for example sodium triacetoxyborohydride in dichloroethane to give the intermediate (XXVIII) (Scheme 11). Reduction of the nitro group using, for example, palladium on carbon or Raney nickel affords the desired intermediate (IV). It will be appreciated that separation of the *cis* and *trans* isomers can be achieved at any suitable stage in the synthesis.

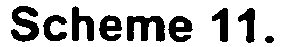

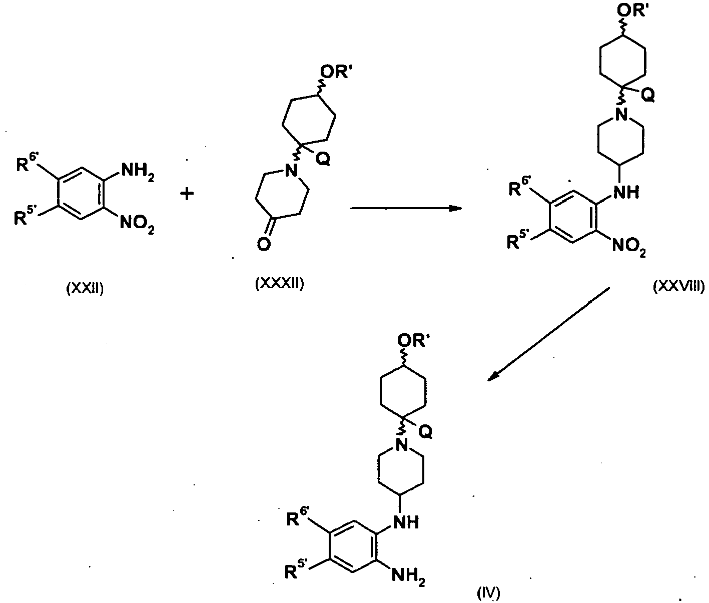

Compounds of formula (V) are commercially available e.g. carbonyl diimidazole, phosgene, phosgene solution in toluene, diphosgene, triphosgene, phenyl chloroformate, diethyl carbonate.

Compounds of formula (VI) can be prepared by a variety of processes e.g. urea formation can be achieved as shown in Scheme 12 by
- combining the two amines (XXXIV) and (XXVII) with phosgene or a phosgene equivalent using standard conditions Phosgene equivalents include carbonyl diimidazole, diphosgene, triphosgene, phenyl chloroformate
- reacting the amine (XXVII) with the isocyanate (XXXV)
- reacting the amine (XXXIV) with the isocyanate (XXXVI)

Both isocyanates can be prepared from the corresponding amines using standard methodology for isocyanate formation. It will be appreciated that separation of the *cis* and *trans* isomers can be achieved at any suitable stage in the synthesis.

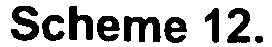

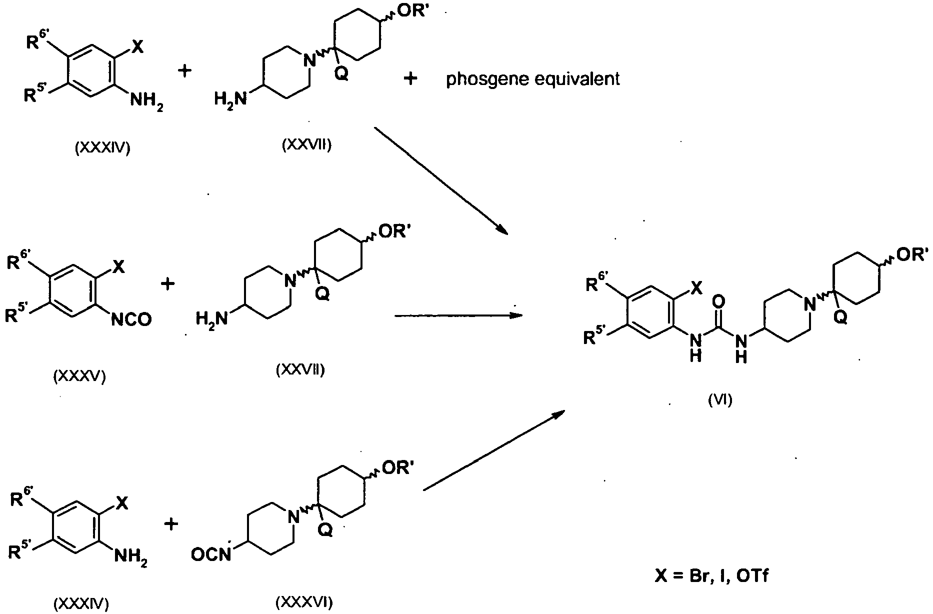

Palladium and copper catalysts (VII) are commercially available or can be prepared as described in the literature (see references in Process C).

Compounds of formula (VIII) are commercially available or can be prepared by known literature routes e.g. reduction of a mono or dinitrobenzene precursor.

Compounds of formula (IX) can be prepared by reductive alkylation of the 3-alkoxycarbonyl-4-piperidone with cyclohexanone.

Compounds of formula (X) can be prepared as shown in Scheme 13. Reductive alkylation of an anthranilic acid or ester (XVII) with the ketone (XXXII), followed if appropriate by hydrolysis of the ester group. It will be appreciated that separation of the *cis* and *trans* isomers can be achieved at any suitable stage in the synthesis.

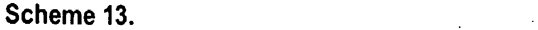

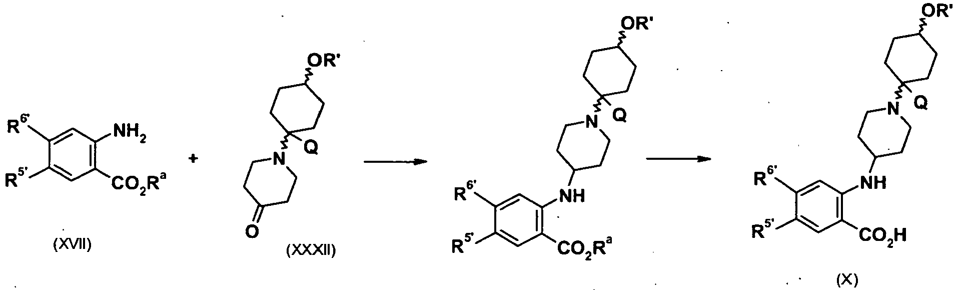

Compounds of formula (XI) are commercially available or can be prepared by literature processes.

Compounds of formula (XII) where Q = H can be prepared as shown in Scheme 14, by reductive alkylation of (XXXVII) where Z' represents Z or a group convertible to Z with the ketone (III). Conversion of a Z' hydroxy group to Z = chloro or bromo can be accomplished using standard methodology e.g. treatment with thionyl chloride or triphenylphosphine/carbon tetrabromide. It will be appreciated that separation of the *cis* and *trans* isomers can be achieved at any suitable stage in the synthesis.

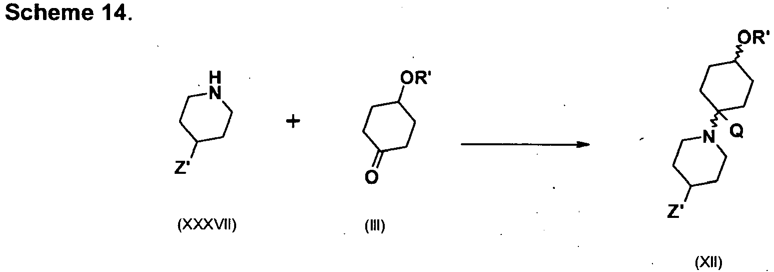

The compound (XXVII) where Q = H can be prepared as shown in Scheme 15. Reductive alkylation of the commercially available amine (XXXVIII) with cyclohexanone (III) using for example sodium triacetoxyborohydride in dichloroethane provides the intermediate (XXXIX) which is deprotected using HCl in ethanol or trifluoroacetic acid to afford the primary amine (XXVII). It will be appreciated that separation of the *cis* and *trans* isomers can be achieved at any suitable stage in the synthesis.

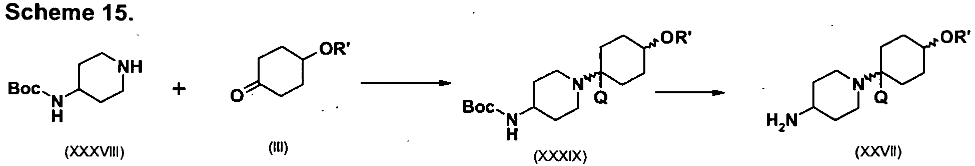

The compound (XXVII) where Q = alkyl can be prepared as in process A2, followed by deprotection.

Compounds of the present invention are M₁ receptor agonists. Selective M₁ receptor agonists are said to be useful to ameliorate positive and cognitive symptoms of psychotic disorders such as schizophrenia, schizo-affective disorders, schizophreniform diseases, psychotic depression, mania, acute mania, paranoid and delusional disorders, and cognitive impairment including memory disorders such as Alzheimer's disease without peripheral cholinergic side effects mediated predominantly through M₂ and M₃ receptors. M₁ receptor agonists may also be suitable for combination with other typical and atypical antipsychotics and other actives such as mood stabilisers, antidepressants, anxiolytics, drugs for extrapyrimidal side effects and cognitive enhancers, to provide improved treatment of psychotic disorders.

Thus in a further aspect, the invention provides a compound of formula (I) as hereinbefore described or a salt or solvate thereof for use in therapy.

In another aspect, the invention provides a compound of formula (I) or a salt or solvate thereof for use in the treatment of a condition which requires agonism of a muscarinic M₁ receptor.

The terms describing the indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

Within the context of the present invention, the term psychotic disorder includes Schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9);

Other conditions the treatment of which require agonism of a muscarinic M₁ receptor include:
Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90);
Anxiety disorders including Social Anxiety Disorder, Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00);
Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide;
Sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type;
Eating disorders such as Anorexia Nervosa (307.1) including the subtypes Restricting Type and Binge-Eating/Purging Type; Bulimia Nervosa (307.51) including the subtypes Purging Type and Nonpurging Type; Obesity Compulsive Eating Disorder, and Eating Disorder Not Otherwise Specified (307.50);
Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23);
Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not. Otherwise Specified (301.9); and
Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Mate Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The compounds of formula (I) may also be useful for the enhancement of cognition, including both the treatment of cognitive impairment on its own and the treatment of cognition impairment in other diseases such as schizophrenia, bipolar disorder, depression, other psychiatric disorders and psychotic conditions associated with cognitive impairment.

Within the context of the present invention, the term cognitive impairment includes, for example, impairment of cognitive functions including attention, orientation, learning disorders, memory (i.e. memory disorders, amnesia, amnesic disorders; transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

The therapy of the present invention may also be used as a memory and/or cognition enhancer in healthy humans with no cognitive and/or memory deficit.

In another aspect, the invention provides a compound of formula (I) as hereinbefore described or a salt or solvate thereof for use in the treatment of a psychotic disorder. In one embodiment, the invention provides a compound of formula (I) as hereinbefore described or a salt or solvate thereof for use in the treatment of schizophrenia.

The invention also provides a compound of formula (I) as hereinbefore described or a salt or solvate thereof for use in the treatment of cognitive impairment.

In another aspect, the invention provides the use of a compound of formula (I) as hereinbefore described or a salt or solvate thereof in the manufacture of a medicament for the treatment of a condition which requires agonism of a muscarinic M₁ receptor.

In another aspect, the invention provides the use of a compound of formula (I) as hereinbefore described or a salt or solvate thereof in the manufacture of a medicament for the treatment of a psychotic disorder. In one embodiment, the invention provides the use of a compound of formula (I) as hereinbefore described or a salt or solvate thereof in the manufacture of a medicament for the treatment of schizophrenia.

The invention also provides the use of a compound of formula (I) as hereinbefore described or a salt or solvate thereof in the manufacture of a medicament for the treatment of cognitive impairment.

The compounds of formula (I) and their salts and solvates thereof may alsbo be suitable for combination with other actives, such as typical and atypical antipsychotics, mood stabilisers, antidepressants, anxiolytics, drugs for extrapyrimidal side effects and cognitive enhancers to provide improved treatment of psychotic disorders.

The combination therapies of the invention are, for example, administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of formula (I) or a salt or solvate thereof and at least one antipsychotic agent, a mood stabiliser, an antidepressant, an anxiolytic, a drug for extrapyrimidal side effects or a cognitive enhancer are within the scope of the current invention. In one embodiment of adjunctive therapeutic administration as described herein, a patient is typically stabilised on a therapeutic administration of one or more of the components for a period of time and then receives administration of another component. The compounds of formula (I) or a salt or solvate thereof may be administered as adjunctive therapeutic treatment to patients who are receiving administration of at least one antipsychotic agent, a mood stabiliser, an antidepressant, an anxiolytic, a drug for extrapyrimidal side effects or a cognitive enhancer, but the scope of the invention also includes the adjunctive therapeutic administration of at least one antipsychotic agent, a mood stabiliser, an antidepressant, an anxiolytic, a drug for extrapyrimidal side effects or a cognitive enhancer to patients who are receiving administration of compounds of formula (I) or a salt or solvate thereof.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect, the invention provides the use of compounds of formula (I) or a salt or solvate thereof in the manufacture of a medicament for adjunctive therapeutic administration for the treatment of a psychotic disorder in a patient receiving therapeutic administration of at least one antipsychotic agent. The invention further provides compounds of formula (I) or a salt or solvate thereof for use for adjunctive therapeutic administration for the treatment of a psychotic disorder in a patient receiving therapeutic administration of at least one antipsychotic agent.

In a further aspect, the invention provides the use of at least one antipsychotic agent in the manufacture of a medicament for adjunctive therapeutic administration for the treatment of a psychotic disorder in a patient receiving therapeutic administration of compounds of formula (I) or a salt or solvate thereof. The invention further provides at least one antipsychotic agent for adjunctive therapeutic administration for the treatment of a psychotic disorder in a patient receiving therapeutic administration of compounds of formula (I) or a salt or solvate thereof.

The invention further provides the use of a combination of compounds of formula (I) or a salt or solvate thereof and at least one antipsychotic agent in the manufacture of a medicament for simultaneous therapeutic administration in the treatment of a psychotic disorder. The invention further provides the use of compounds of formula (I) or a salt thereof in the manufacture of a medicament for simultaneous therapeutic administration with at least one antipsychotic agent in the treatment of a psychotic disorder. The invention further provides compounds of formula (I) or a salt thereof for use for simultaneous therapeutic administration with at least one antipsychotic agent in the treatment of a psychotic disorder. The invention further provides the use of at least one antipsychotic agent in the manufacture of a medicament for simultaneous therapeutic administration with compounds of formula (I) or a salt thereof in the treatment of a psychotic disorder.

In a further aspect, the invention provides a kit-of-parts for use in the treatment of a psychotic disorder comprising a first dosage form comprising compounds of formula (I) or a salt or solvate thereof and one or more further dosage forms each comprising a antipsychotic agent for simultaneous therapeutic administration.

In a further aspect, the invention provides the use of a compound of the present invention in the manufacture of a medicament for adjunctive therapeutic administration for the treatment of a psychotic disorder in a patient receiving therapeutic administration of an active ingredient selected from the group consisting of: a mood stabiliser, an antidepressant, an anxiolytic, a drug for extrapyrimidal side effects and a cognitive enhancer.

In a further aspect, the invention provides the use of an active ingredient selected from the group consisting of: a mood stabiliser, an antidepressant, an anxiolytic, a drug for extrapyrimidal side effects and a cognitive enhancer in the manufacture of a medicament for adjunctive therapeutic administration for the treatment of a psychotic disorder in a patient receiving therapeutic administration of a compound of the present invention.

The invention further provides the use of a combination of a compound of the present invention and an active ingredient selected from the group consisting of: a mood stabiliser, an antidepressant, an anxiolytic, a drug for extrapyrimidal side effects and a cognitive enhancer in the manufacture of a medicament for simultaneous therapeutic administration in the treatment of a psychotic disorder.

The invention further provides the use of a compound of the present invention in the manufacture of a medicament for simultaneous therapeutic administration with an active ingredient selected from the group consisting of: a mood stabiliser, an antidepressant, an anxiolytic, a drug for extrapyrimidal side effects and a cognitive enhancer in the treatment of a psychotic disorder.

The invention further provides a compound of the present invention for use for simultaneous therapeutic administration with an active ingredient selected from the group consisting of: a mood stabiliser, an antidepressant, an anxiolytic, a drug for extrapyrimidal side effects and a cognitive enhancer in the treatment of a psychotic disorder.

The invention further provides the use of an active ingredient selected from the group consisting of: a mood stabiliser, an antidepressant, an anxiolytic, a drug for extrapyrimidal side effects and a cognitive enhancer in the manufacture of a medicament for simultaneous therapeutic administration with a compound of the present invention in the treatment of a psychotic disorder.

In a further aspect, the invention provides a kit-of-parts for use in the treatment of a psychotic disorder comprising a first dosage form comprising a compound of the present invention and one or more further dosage forms each comprising an active ingredient selected from the group consisting of: a mood stabiliser, an antidepressant, an anxiolytic, a drug for extrapyrimidal side effects and a cognitive enhancer for simultaneous therapeutic administration.

In one embodiment, the patient is a human.

Examples of antipsychotic drugs that may be useful in the present invention include, but are not limited to: sodium channel blockers; mixed 5HT/dopamine receptor antagonists; mGluR5 positive modulators; D3 antagonists; 5HT6 angatonists; nicotinic alpha-7 modulators; glycine transporter GlyT1 inhibitors; D2 partial agonist/D3 antanogist/H3 antagonists; AMPA modulators; NK3 antagonists such as osanetant and talnetant; an atypical antipsychotic, for example clozapine, olanzapine, risperidone, quetiapine, aripirazole, ziprasidone and amisulpride; butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene; thienobenzodiazepines; dibenzodiazepines; benzisoxazoles; dibenzothiazepines; imidazolidinones; benzisothiazolyl-piperazines; triazine such as lamotrigine; dibenzoxazepines, such as loxapine; dihydroindolones, such as molindone; aripiprazole; and derivatives thereof that have antipsychotic activity.

Examples of tradenames and suppliers of selected antipsychotic drugs that may be suitable for use in the present invention are as follows : clozapine (available under the tradename CLOZARIL®, from Mylan, Zenith Goldline, UDL, Novartis); olanzapine (available under the tradename ZYPREXA®, from Lilly ; ziprasidone (available under the tradename GEODON®, from Pfizer); risperidone (available under the tradename RISPERDAL®, from Janssen); quetiapine fumarate (available under the tradename SEROQUEL®, from AstraZeneca); sertindole (available under the tradename SERLECT®); amisulpride (available under the tradename SOLION®, from Sanofi-Synthelabo); haloperidol (available under the tradename HALDOL®, from Ortho-McNeil); haloperidol decanoate (available under the tradename HALDOL decanoate®); haloperidol lactate (available under the tradenames HALDOL® and INTENSOL®); chlorpromazine (available under the tradename THORAZINE®, from SmithKline Beecham (GSK); fluphenazine (available under the tradename PROLIXIN®, from Apothecon, Copley, Schering, Teva, and American Pharmaceutical Partners, Pasadena); fluphenazine decanoate (available under the tradename PROLIXIN decanoate®); fluphenazine enanthate (available under the tradename PROLIXIN®); fluphenazine hydrochloride (available under the tradename PROLIXIN®); thiothixene (available under the tradename NAVANE®;, from Pfizer); thiothixene hydrochloride (available under the tradename NAVANE®); trifluoperazine (10-[3-(4-methyl-1-piperazinyl)propyl]-2-(trifluoromethyl)phenothiazine dihydrochloride, available under the tradename STELAZINE®, from SmithKline Beckman; perphenazine (available under the tradename TRILAFON®; from Schering); perphenazine and amitriptyline hydrochloride (available under the tradename ETRAFON TRILAFON®); thioridazine (available under the tradename MELLARIL®; from Novartis, Roxane, HiTech, Teva, and Alpharma) ; molindone (available under the tradename MOBAN®, from Endo); molindone hydrochloride (available under the tradename MOBAN®); loxapine, (available under the tradename LOXITANE®; from Watson); loxapine hydrochloride (available under the tradename LOXITANE®); and loxapine succinate (available under the tradename LOXITANE®). Furthermore, benperidol (Glianimon®), perazine (Taxilan®) or melperone (Eunerpan®)) may be used.

Other suitable antipsychotic drugs include promazine (available under the tradename SPARINE®), triflurpromazine (available under the tradename VESPRIN®), chlorprothixene (available under the tradename TARACTAN®), droperidol (available under the tradename INAPSINE®), acetophenazine (available under the tradename TINDAL®;), prochlorperazine (available under the tradename COMPAZINE®), methotrimeprazine (available under the tradename NOZINAN®), pipotiazine (available under the tradename PIPOTRIL®), iloperidone, pimozide and flupenthixol.

The antipsychotic drugs listed above by Tradename may also be available from other suppliers under a different Tradename.

In one further aspect of the invention, suitable antipsychotic agents include olanzapine, risperidone, quetiapine, aripiprazole, haloperidol, clozapine, ziprasidone, talnetant and osanetant.

Mood stabilisers which may be used in the therapy of the present invention include lithium, sodium valproate/valproic acid/divalproex, carbamazepine, lamotrigine, gabapentin; topiramate, oxcarbazepine and tiagabine.

Antidepressant drugs which may be used in the therapy of the present invention include serotonin antagonists, CRF-1 antagonists, Cox-2 inhibitor/SSRI dual antagonists; dopamine/noradrenaline/serotonin triple reuptake inhibitors; NK1 antagonists; NK1 and NK2 dual antagonists; NK1/SSRI dual antagonists; NK2 antagonists; serotonin agonists (such as rauwolscine, yohimbine and metoclopramide); serotonin reuptake inhibitors (such as citalopram, escitalopram, fluoxetine, fluvoxamine, femoxetine, indalpine, zimeldine, paroxetine and sertraline); dual serotonin/noradrenaline reuptake inhibitors (such as venlafaxine, reboxetine, duloxetine and milnacipran); Noradrenaline reuptake inhibitors (such as reboxetine); tricyclic antidepressants (such as amitriptyline, clomipramine, imipramine, maprotiline, nortriptyline and trimipramine); monoamine oxidase inhibitors (such as isocarboxazide, moclobemide, phenelzine and tranylcypromine); 5HT3 antagonists (such as example ondansetron and granisetron); and others (such as bupropion, amineptine, radafaxine, mianserin, mirtazapine, nefazodone and trazodone).

Anxiolytics which may be used in the therapy of the present invention include V1b antagonists, 5HT7 antagonists and benzodiazepines such as alprazolam and lorazepam.

Drugs for extrapyramidal side effects which may be used in the therapy of the present invention include anticholinergics (such as benztropine, biperiden, procyclidine and trihexyphenidyl), antihistamines (such as diphenhydramine) and dopaminergics (such as amantadine).

Cognitive enhancers which may be used in the therapy of the present invention include example cholinesterase inhibitors (such as tacrine, donepezil, rivastigmine and galantamine), H3 antagonists and muscarinic M1 agonists (such as cevimeline).

In one embodiment, the active ingredient for use in combination with a compound of the present invention, is an atypical antipsychotic, for example clozapine, olanzapine, risperidone, quetiapine, aripirazole, ziprasidone or amisulpride.

In one embodiment, the active ingredient for use in combination with a compound of the present invention is a typical antipsychotic, for example chlorpromazine, thioridazine, mesoridazine, fluphenazine, perphenazine, prochlorperazine, trifluoperazine, thiothixine, haloperidol, thiflurpromazine, pimozide, droperidol, chlorprothixene, molindone or loxapine.

In another embodiment, the active ingredient for use in combination with a compound of the present invention is a mood stabiliser, for example lithium, sodium valproate/valproic acid/divalproex, carbamazepine, lamotrigine, gabapentin, topiramate, oxcarbazepine or tiagabine.

In another embodiment, the active ingredient for use in combination with a compound of the present invention is an antidepressant, for example a serotonin agonist (such as rauwolscine, yohimbine or metoclopramide); a serotonin reuptake inhibitor (such as citalopram, escitalopram, fluoxetine, fluvoxamine, femoxetine, indalpine, zimeldine, paroxetine or sertraline); a dual serotonin/noradrenaline reuptake inhibitor (such as venlafaxine, reboxetine, duloxetine or milnacipran); a noradrenaline reuptake inhibitors (such as reboxetine); a tricyclic antidepressants (such as amitriptyline, clomipramine, imipramine, maprotiline, nortriptyline or trimipramine); a monoamine oxidase inhibitor (such as isocarboxazide, moclobemide, phenelzine or tranylcypromine); or other (such as bupropion, amineptine, radafaxine, mianserin, mirtazapine, nefazodone or trazodone).

In another embodiment, the active ingredient for use in combination with a compound of the present invention is an anxiolytic, for example a benzodiazepine such as alprazolam or lorazepam.

For use in medicine, the compounds of the present invention are usually administered as a standard pharmaceutical composition. The present invention therefore provides in a further aspect a pharmaceutical composition comprising a compound of formula (I) as hereinbefore described or a salt or solvate thereof and a pharmaceutically acceptable carrier. The pharmaceutical composition can be for use in the treatment of any of the conditions described herein.

The compounds of formula (I) may be administered by any convenient method, for example by oral, parenteral (e.g. intravenous), buccal, sublingual, nasal, rectal or transdermal administration and the pharmaceutical compositions adapted accordingly.

The compounds of formula (I) as hereinbefore described and their salts or solvates which are active when given orally can be formulated as liquids or solids, for example syrups, suspensions or emulsions, tablets, capsules and lozenges.

A liquid formulation will generally consist of a suspension or solution of the compound or salt or solvate in a suitable liquid carrier(s) for example an aqueous solvent such as water, ethanol or glycerine, or a non-aqueous solvent, such as polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or salt or solvate in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active substance in a pharmaceutically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas such as compressed air or an organic propellant such as a fluorochloro-hydrocarbon. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches. The composition may be in unit dose form such as a tablet, capsule or ampoule.

Each dosage unit for oral administration contains, for example, from 1 to 250 mg (and for parenteral administration contains, for example, from 0.1 to 25 mg) of a compound of the formula (I) or a salt thereof calculated as the free base.

The antipsychotic agent component or components used in the adjunctive therapy of the present invention may also be administered in their basic or acidic forms as appropriate or, where appropriate, in the form of a salt or other derivative. All solvates and all alternative physical forms of the antipsychotic agent or agents or their salts or derivatives as described herein, including but not limited to alternative crystalline forms, amorphous forms and polymorphs, are also within the scope of this invention. In the case of the antipsychotic agent or agents, the forms and derivatives are, for example, those which are approved for therapeutic administration as monotherapies, including those mentioned above, but all references to antipsychotic agents herein include all salts or other derivatives thereof, and all solvates and alternative physical forms thereof.

For adjunctive therapeutic administration according to the invention, compounds of formula (I) or salts or solvates and the antipsychotic agent or agents or their salts, derivatives or solvates may each be administered in pure form, but each of the components will, for example, be formulated into any suitable pharmaceutically acceptable and effective composition which provides effective levels of the respective component in the body. The choice of the most appropriate pharmaceutical compositions for each component is within the skill of the art, and may be the same form or different forms for each of the components. Suitable formulations include, but are not limited to tablets, capsules, powders, granules, lozenges, suppositories, reconstitutable powders, or liquid preparations such as oral or sterile parenteral solutions or suspensions.

For simultaneous administration as a combined composition of compounds of formula (I) and the antipsychotic agent or agents according to the invention, compounds of formula (I) or their salts or solvates and the antipsychotic agent or agents and their salts, derivatives or solvates may be administered together in pure form, but the combined components will, for example, be formulated into any suitable pharmaceutically acceptable and effective composition which provides effective levels of each of the components in the body. The choice of the most appropriate pharmaceutical compositions for the combined components is within the skill of the art. Suitable formulations include, but are not limited to tablets, sub-lingual tablets, buccal compositions, capsules, powders, granules, lozenges, suppositories, reconstitutable powders, or liquid preparations such as oral or sterile parenteral solutions or suspensions.

In order to obtain consistency of adjunctive administration, the compositions of each of the components, or of the combination of the components is, for example, in the form of a unit dose.

The term "treatment" includes prophylaxis, where this is appropriate for the relevant condition(s).

### Biological Test Methods

### FLIPR experiments on M₁ receptor to determine agonist/antagonist potency

### Assay A

Compounds of the invention were characterized in a functional assay to determine their ability to activate the intracellular calcium pathway in CHO cells with stable expression of human muscarinic receptors using FLIPR (Fluorometric Imaging Plate Reader) technology. Briefly, CHO-M1 cells were plated (20,000/well) and allowed to grow overnight at 37 degrees. Media was removed and 30uL loading buffer (HBSS with 20mM HEPES, pH 7.4) containing FLIPR Calcium 3 dye (Molecular Devices Co., Sunnyvale, CA) was added according to manufacturer's instructions. After incubation at 37 degrees for 45-60 minutes, 10uL of the assay buffer (HBSS with 20mM HEPES and 2.5 mM probenecid, pH 7.4) containing test compounds was added to each well on FLIPR instrument. Calcium response was monitored to determine agonism. Plates were then incubated for another 30 minutes before 10uL of assay buffer containing acetylcholine was added at an EC₈₀, as the agonist challenge. Calcium response was then monitored again to determine compound's antagonism to acetylcholine. Concentration-response curves of both agonism and antagonism on M1 receptors were performed for each compound. Results were imported into ActivityBase data analysis suite (ID Business Solution Inc., Parsippany, NJ) where the curves were analysed by non-linear curve fitting and the resulting pEC₅₀/pIC₅₀ were calculated. The intrinsic activities of agonist compounds were calculated as percentage of maximum FLIPR response induced by acetylcholine (ie using acetylcholine at EC₁₀₀ as the control).

### Assay B

Compounds of the invention were characterized in a functional assay to determine their ability to activate the intracellular calcium pathway in CHO cells with stable expression of human muscarinic receptors using FLIPR (Fluorometric Imaging Plate Reader) technology. Briefly, CHO-M1 cells were plated (15,000/well) and allowed to grow overnight at 37 degrees. Media was removed and 30uL loading buffer (HBSS with 2.5mM probenicid, 2uM Fluo-4, 500uM Brilliant Black, pH 7.4) was added. After incubation at 37 degrees for 90 minutes, 10uL of the assay buffer (HBSS with 2.5 mM probenecid, pH 7.4) containing test compounds was added to each well on the FLIPR instrument. Calcium response was monitored to determine agonism. Plates were then incubated for another 30 minutes before 10uL of assay buffer containing acetylcholine was added at an EC80, as the agonist challenge. Calcium response was then monitored again to determine compound's antagonism to acetylcholine. Concentration-response curves of both agonism and antagonism on M1 receptors were performed for each compound. Results were imported into ActivityBase data analysis suite (ID Business Solution Inc., Parsippany, NJ) where the curves were analysed by non-linear curve fitting and the resulting pEC50/fpKi were calculated. The intrinsic activities of agonist compounds were calculated as percentage of maximum FLIPR response induced by acetylcholine added as control on the same compound plates, and converted to a fraction between 0 and 1 (ie calculated using a 100% max response from a fitted acetylcholine standard curve, containing multiple concentrations, as control).

Examples 1-32 below were tested in one or both of the above assays and were found to have a pEC₅₀ value of > 6.0 at the muscarinic M₁ receptor, and intrinsic activity > 50%.

### FLIPR experiments on M₁ receptor to determine agonist intrinsic activity

### Assay A

To determine the intrinsic activities of M1 agonist compounds, compounds of the invention were characterized in FLIPR experiments on U2OS cells with transient expression of human muscarinic M1 receptors. Briefly, U2OS cells were transduced with M1 BacMam virus (Ames, R S; Fornwald, J A; Nuthulaganti, P; Trill, J J; Foley, J J; Buckley, P T; Kost, T A; Wu, Z and Romanos, M A. (2004) Use of BacMam recombinant baculoviruses to support G protein-coupled receptor drug discovery. Receptors and Channels 10 (3-4): 99-109) in 2x10e⁵/mL cell suspension with 0.1 % virus/cell ratio (v/v). The virus to cell ratio was determined in separate experiments by functional titration to be most appropriate to measure intrinsic activities of partial agonists. After mixing with virus in suspension, cells were then plated (10,000/well) and allowed to grow overnight at 37 degrees. FLIPR experiment was then carried out next day using the same protocol as described above for CHO-M1 cells. Results were imported into ActivityBase data analysis suite where the curves were analysed by non-linear curve fitting and the resulting pEC50 values were calculated. The intrinsic activities of agonist compounds were calculated as percentage of maximum FLIPR response induced by acetylcholine added as control on the same compound plates, and converted to a fraction between 0 and 1 (ie calculated using a 100%max response from a fitted acetylcholine standard curve, containing multiple concentrations, as control).

### Assay B

To determine the intrinsic activities of M1 agonist compounds, compounds of the invention were characterized in FLIPR experiments on CHO cells with transient expression of human muscarinic M1 receptors. Briefly, CHO cells were transduced with M1 BacMam virus (Ames, R S; Fornwald, J A; Nuthulaganti, P; Trill, J J; Foley, J J; Buckley, P T; Kost, T A; Wu, Z and Romanos, M A. (2004) Use of BacMam recombinant baculoviruses to support G protein-coupled receptor drug discovery. Receptors and Channels 10 (3-4): 99-109) at a multiplicity of infection of 6. The virus to cell ratio was determined in separate experiments by functional titration to be most appropriate to measure intrinsic activities of partial agonists. After mixing with virus in suspension, cells were then plated (15,000/well) and allowed to grow overnight at 37 degrees.

Alternatively, cells were then frozen in 1ml vials at a concentration of 4.8x10e7 cells/ml in 90% dialysed Foetal Bovine Serum, 10% DimethylSulphoxide at -140 degrees. Cells could then be thawed on the day prior to assay, plated (15,000/well) and allowed to grow overnight at 37 degrees.

The FLIPR experiment was carried out on the day following plating using the same protocol as described above for CHO-M1 cells. Results were imported into ActivityBase data analysis suite where the curves were analysed by non-linear curve fitting and the resulting pEC50 values were calculated. The intrinsic activities of agonist compounds were calculated as percentage of maximum FLIPR response induced by acetylcholine added as control on the same compound plates, and converted to a fraction between 0 and 1 (ie calculated using a 100% max response from a fitted acetylcholine standard curve, containing multiple concentrations, as control).

Examples 1-32 below were tested in one or both of the above assays, and were found to have a pEC₅₀ value of > 6.0 at the muscarinic M₁ receptor, and intrinsic activity of greater than or equal to 0.3.

### FLIPR experiments on M₂₋₅ receptor to determine receptor subtype selectivity

### Assay A

To determine selectivity of compounds of the invention against other muscarinic receptor subtypes, compounds were characterized in FLIPR experiments in CHO cells with stable expression of human muscarinic receptors, M2, M3, M4 or M5. In the case of M2 and M4 receptors, chimeric G-protein Gqi5 was also co-expressed to couple receptors to the calcium signaling pathway. Briefly, cells were plated (20,000/well) and allowed to grow overnight at 37 degrees. FLIPR experiment was then carried out next day using the same protocol as described above for CHO-M1 cells. Results were imported into ActivityBase data analysis suite where the curves were analysed by non-linear curve fitting and the resulting pEC50/pIC50 values were calculated.

### Assay B

To determine selectivity of compounds of the invention against other muscarinic receptor subtypes, compounds were characterized in FLIPR experiments in CHO cells with stable expression of human muscarinic receptors, M2, M3, M4 or M5. In the case of M2 and M4 receptors, chimeric G-protein Gqi5 was also co-expressed to couple receptors to the calcium signaling pathway. Briefly, cells were plated (15,000/well) and allowed to grow overnight at 37 degrees. The FLIPR experiment was then carried out on the next day using the same protocol as described above for CHO-M1 cells. Results were imported into ActivityBase data analysis suite where the curves were analysed by non-linear curve fitting and the resulting pEC50/fpKi values were calculated.

Examples 1-32 below were tested in one or both of the above assays and were found to be selective for the M1 receptor over M2, M3, M4 and M5 receptors, with typical selectivity (ratio of pEC50's) of ≥10-fold, and in certain cases ≥ 100-fold.

The invention is further illustrated by the following non-limiting examples. In the procedures that follow, after each starting material, reference to a Description by number is typically provided. This is provided merely for assistance to the skilled chemist. The starting material may not necessarily have been prepared from the batch referred to. SCX refers to a sulfonic acid ion exchange resin supplied by Varian. MDAP refers to purification by HPLC, wherein fraction collection is by reverse phase chromatography on C₁₈ stationary phase eluted with acetonitrile/water/0.1% formic acid, and is triggered by detection of the programmed mass ion for the compound of interest. All reactions were either done under argon or can be done under argon, unless stated otherwise (for example hydrogenation reactions). All ¹H NMR are consistent with the structures shown.

### Description 1. 1-Chloro-4-fluoro-2-methyl-5-nitrobenzene (D1).

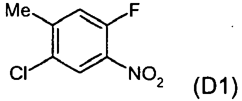

1-Chloro-4-fluoro-2-methylbenzene (10 mmol, 1.44 g) was dissolved in concentrated sulphuric acid under argon and cooled to 0°C. KNO₃ (10 mmol, 1.01 g, 1 eq) was then added cautiously while keeping the temperature around 0°C. The mixture was then allowed to stir for 2h while warming up to room temperature, then it was poured cautiously onto ice and extracted twice with ether. The organics were combined, dried on MgSO₄ and concentrated under reduced pressure to give an oil (9.1 mmol, 1.73 g, 91% yield), which was chromatographed (0-25% ethyl acetate/petrol ether) to give the title compound as a transparent oil (7 mmol, 1.3 g, 70% total yield).
¹H NMR □ (CDCl₃): 8.09 (1H, d), 7.19 (1H, d), 2.46 (3H, s).

### Description 2. 1-Bromo-5-fluoro-4-nitro-2-(trifluoromethyl)benzene (D2).

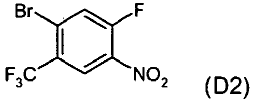

A stirred solution of 2-bromo-4-fluorobenzotrifluoride (1.0 g, 4.1 mmole) in concentrated sulphuric acid (10 ml) at 0°C was treated portionwise with KNO₃ (0.46 g, 4.6 mmole) and maintained at 0°C for 0.5 hr before warming to room temp. over 2 hr. The mixture was added to well stirred ice/water (100 ml) and then extracted with ethyl acetate. The extract was dried and concentrated under vacuum to afford the title compound as a yellow solid (1.2 g, 100%).
¹H NMR (400MHz, CDCl₃) □: 7.75 (1H, d), 8.45 (1H, d).

### Description 3. 1,4-Dioxaspiro[4.5]decan-8-ol (D3)

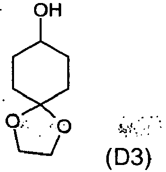

1,4-Dioxaspiro[4.5]decan-8-one (64 mmol, 10 g) was dissolved in ethanol (125 ml) and treated with NaBH₄ (1.2eq., 76.8 mmol, 2.9 g), at 0°C and the mixture was stirred at room temperature for 1 hour. Reaction was quenched with NaOH (25 ml, 2N aqueous solution). The aqueous solution was extracted with dichloromethane (2x). The organics were combined, dried over Na₂SO₄, filtered and the solvent was evaporated to afford the title compound, 8.3 g, 82%, as a colourless oil.
¹HNMR □ (d⁶DMSO, 400 MHz) 1.443 (4H, m), 1.645 (4H, m), 3.549 (1H, d broad), 3.827 (4H, m), 4.480 (1H, d).

### Description 4. 8-(Methyloxy)-1,4-dioxaspiro[4.5]decane (D4)

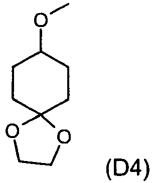

1,4-dioxaspiro[4.5]decan-8-ol (D3, 52.5 mmol, 8.3g) was dissolved in dimethylformamide (200 ml) and NaH (2eq., 105 mmol, 4.2 g) was added at 0°C portionwise. The mixture was stirred at 0°C for 10 minutes and iodomethane (2 eq., 6.5 ml) was added at room temperature. The mixture was stirred at room temperature for 2 hours, then it was quenched with methanol, partitioned between ethyl acetate and water and the two phases were separated. The aqueous phase was extracted with ethyl acetate (2x), the combined organics were washed with brine, dried over Na₂SO₄, filtered and the solvent was evaporated to afford the crude compound. Chromatography (ethyl acetate/n-hexane) afforded title compound, 7.3 g, 80%, as a colourless oil.
¹HNMR □ (d⁶DMSO, 400 MHz) 1.462 (2H, m),1.557 (2H, m), 1.642 (2H, m), 1.732 (2H, m), 3.210 (3H, s), 3.242 (1H, m), 3.834 (4H, m)

### Description 5. 4-(Methyloxy)cyclohexanone (D5)

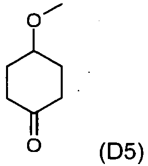

8-(Methyloxy)-1,4-dioxaspiro[4.5]decane (D4, 62.2 mmol, 11.9g) was dissolved in 10 ml of tetrahydrofuran and HCl (50 ml of 5M aqueous solution) was added at room temperature; the mixture was stirred at room temperature overnight. Tetrahydrofuran was then evaporated, mixture was basified to pH = 10 and it was extracted with ethyl acetate (3x); the organic phases were combined and washed with brine, dried over Na₂SO₄, filtered and the solvent was evaporated to afford the title compound, 8.2g, complete conversion.
¹HNMR □ (d⁶DMSO, 400 MHz) 1.920 (4H, m), 2.199 (2H, m), 2.348 (2H, m), 3.287 (3H, s), 3.531 (1H, m)

### Description 6. 1,1-Dimethylethyl (1-(4-methoxycyclohexyl)-4-piperidinyl]carbamate (D6)

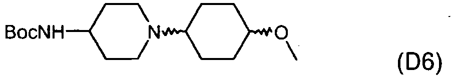

A solution of 4-methoxycyclohexanone (D5, 4.5g) in dichloromethane (200ml) at room temperature under argon was treated with 1,1-dimethylethyl 4-piperidinylcarbamate (9.0g) and sodium triacetoxyborohydride (16.0g) then stirred at room temperature for 18h, then partitioned between dichloromethane and water at pH9. Drying, evaporation and chromatography (50g silica, 0-10% methanol in dichloromethane) gave the title compound as a mixture of *cis* and *trans* isomers, 7.0g.

### Description 7. cis/trans-1-(4-Methoxycyclohexyl)-4-piperidinamine dihydrochloride (D7)

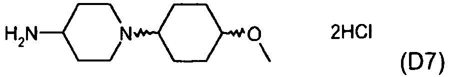

A solution of 1,1-dimethylethyl [1-(4-methoxycyclohexyl)-4-piperidinyl]carbamate (D6, 7.0g) in dichloromethane (50ml) at room temperature was treated with 4M HCl in dioxane (25ml). After 4h evaporation gave the title compound, 6.3g.

### Description 8. cis and trans-N-(4-Fluoro-5-methyl-2-nitrophenyl)-1-(4-methoxycyclohexyl)-4-piperidinamine (D8a, D8b)

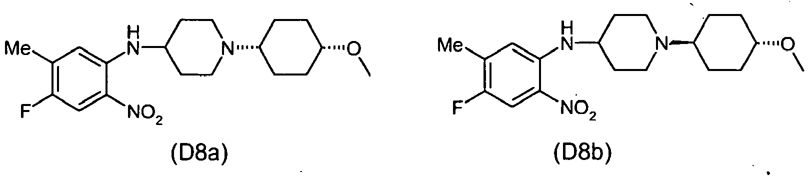

A stirred solution of 2,5-difluoro-4-nitrotoluene (0.7g) in dimethylformamide (15ml) at room temperature under argon was treated with diisopropylethylamine (2.0ml) and 1-[4-(methoxy)cyclohexyl]-4-piperidinamine dihydrochloride (D7, cis:trans mixture, 0.85g) and heated at 80°C for 18h. The cooled reaction was diluted with ethyl acetate and washed three times with water then dried, evaporated and chromatographed on Biotage KP-NH^{™} (NH-silica column eluting with 0-25% ethyl acetate/hexane) to give the separate cis (D8a, 370mg) and trans (D8b, 180mg) isomers of the title compound.

### Description 9. (2-Amino-4-fluoro-5-methylphenyl){1-[cis-4-(methyloxy)cyclohexyl]-4-piperidinyl}amine (D9)

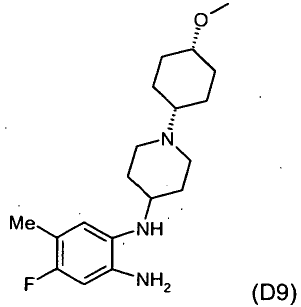

A stirred solution of *N*-(4-fluoro-5-methyl-2-nitrophenyl)-1-[*cis*-4-(methyloxy)cyclohexyl]-4-piperidinamine (D8a, 0.38g, 1.09 mmole) in ethanol (30ml) at room temperature under argon was treated with Raney Nickel (∼50mg) followed by a dropwise addition of hydrazine hydrate (1.5ml, 50.4 mmole). The mixture was heated at 50°C for 7h then cooled and filtered through a Kieselguhr pad to remove the catalyst and the solution was concentrated under vacuum to afford the title compound as a pale yellow oil (0.33g, 95%yield).
MH⁺ = 336, 337 and 338.

### Description 10. (2-Amino-4-fluoro-5-methylphenyl){1-[trans-4-(methyloxy)-cyclohexyl]-4-piperidinyl}amine (D10)

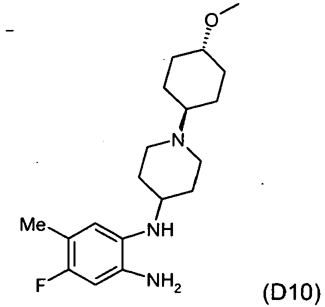

A stirred solution of *N*-(4-fluoro-5-methyl-2-nitrophenyl)-1-[*trans*-4-(methyloxy)cyclohexyl]-4-piperidinamine (D8b, 0.29g, 0.83 mmole) in ethanol (30ml) at room temperature under argon was treated with Raney Nickel (∼50mg) followed by a dropwise addition of hydrazine hydrate (1.1ml, 36.96 mmole). The mixture was maintained overnight then filtered through a Kieselguhr pad to remove the catalyst and the solution was concentrated under vacuum to afford the title compound as a pale yellow oil (0.25g, 95%yield).
MH⁺ = 336 and 337.

### Description 11. cis and trans-N-(4-Chloro-5-methyl-2-nitrophenyl)-1-(4-methoxycyclohexyl)-4-piperidinamine (D11a, D11b)

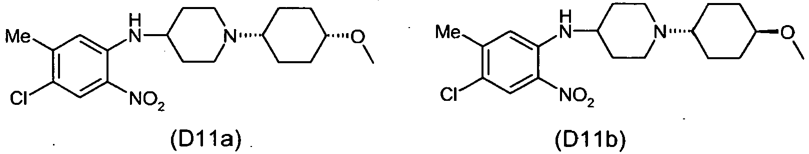

A stirred solution of 2-chloro-5-difluoro-4-nitrotoluene (D1, 0.8g) in dimethylformamide (15ml) at room temperature under argon was treated with diisopropylethylamine (2.0ml) and 1-[4-(methoxy)cyclohexyl]-4-piperidinamine dihydrochloride (D7, cis:trans mixture, 0.85g) and heated at 80°C for 18h. The cooled reaction was diluted with ethyl acetate and washed three times with water then dried, evaporated and was repeatedly chromatographed on a Biotage KP-NH^{™} column eluted with ethyl acetate and hexane using a gradient from 0 to 25% ethyl acetate to give the separate cis and trans isomers. *N*-(4-chloro-5-methyl-2-nitrophenyl)-1-[*cis*-4-(methyloxy)cyclohexyl]-4-piperidinamine (D11a, 0.236g) was isolated as the fast running isomer. ¹H NMR □ (CDCl₃, 400 MHz): 1.42 (2H, m), 1.65 (obs , m), 2.05 (4H, m), 2.36 (3H, s), 2.45 (3H, m), 3.90 (2H, m), 3.30 (3H, s), 3.39 (1H, m), 3.51 (1H, m), 6.70 (1H, s), 8.09 (1H, d), 8.16 (1H, s).

*N*-(4-chloro-5-methyl-2-nitrophenyl)-1-[*trans*-4-(methyloxy)cyclohexyl]-4-piperidinamine (D11b, 0.140g) was isolated as the slow running isomer. ¹H NMR □ (CDCl₃, 400 MHz): 1.24 (4H, m), 1.65 (2H, m), 1.90 (2H, m), 2.1 (4H, m), 2.4 (6H, m), 2.85 (2H, m), 3.10 (1H, m), 3.30 (3H, s), 3.53 (1H, m), 6.70 (1H, s), 8.08 (1H, d), 8.16 (1H, s).

### Description 12. (2-Amino-4-chloro-5-methylphenyl){1-[cis-4-(methyloxy)cyclohexyl]-4-piperidinyl}amine (D12)

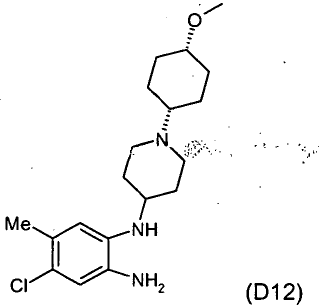

A stirred solution of *N*-(4-chloro-5-methyl-2-nitrophenyl)-1-[*cis*-4-(methyloxy)cyclohexyl]-4-piperidinamine (D11a, 0.25g, 0.65mmole) in ethanol (50ml) at room temperature under argon was treated with Raney Nickel (-50mg) followed by a dropwise addition of hydrazine hydrate (1ml, 33.6mmole). The mixture was maintained for 30 minutes then filtered through a Kieselguhr pad to remove the catalyst and the solution was concentrated under vacuum to afford the title compound as a yellow oil (0.22g, 95%yield).
MH⁺ = 352 and 354.

### Description 13. (2-Amino-4-chloro-5-methylphenyl){1-[trans-4-(methyloxy)-cyclohexyl]-4-piperidinyl}amine (D13)

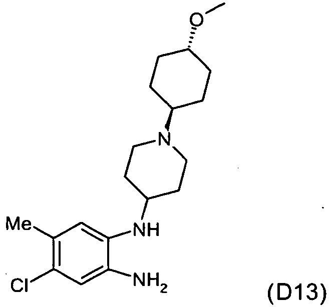

A stirred solution of *N*-(4-chloro-5-methyl-2-nitrophenyl)-1-[*trans*-4-(methyloxy)cyclohexyl] 4-piperidinamine (D11b, 0.14g, 0.37mmole) in ethanol (35ml) at room temperature under argon was treated with Raney Nickel (∼20mg) followed by a dropwise addition of hydrazine hydrate (0.8ml, 36.88mmole). The mixture was maintained for 30 minutes then filtered through a Kieselguhr pad to remove the catalyst and the solution was concentrated under vacuum to afford the title compound as a pale yellow oil (0.13g, 99%yield).
MH⁺ = 352 and 354.

### Description 14. cis and trans-N-(5-Bromo-2-nitro-4-(trifluoromethyl)phenyl)-1-(4-methoxycyclohexyl)-4-piperidinamine (cis = D14a, trans = D14b)

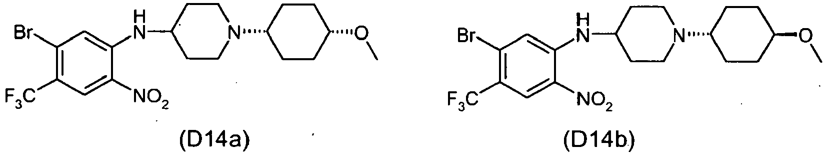

A stirred solution of 2-fluoro-5-trifluoromethyl-4-bromonitrobenzene (D2,1.2g) in dimethylformamide (15ml) at room temperature under argon was. treated with diisopropylethylamine (2.0ml) and 1-[4-(methoxy)cyclohexyl]-4-piperidinamine dihydrochloride (D7, cis:trans mixture, 0.85g) and stirred at room temperature for 18h. The cooled reaction was diluted with dichloromethane and washed twice with water then dried, evaporated and repeatedly chromatographed on a Biotage KP-NH^{™} column eluted with ethyl acetate and hexane using a gradient from 0 to 25% ethyl acetate to give the separate *cis* and *trans* isomers.

*N*-[5-bromo-2-nitro-4-(trifluoromethyl)phenyl]-1-[*cis*-4-(methyloxy)cyclohexyl]-4-piperidinamine (D14a, 0.508g) was isolated as the fast running isomer. ¹H NMR □ (CDCl₃, 400 MHz): 1.40 (2H, m), 1.60 (obs, m), 2.06 (4H, m), 2.38 (1H, m), 2.50 (2H, m), 2.90 (2H, m), 3.30 (3H, s), 3.40 (1H, m), 3.55 (1H, m), 7.18 (1H, s), 8.29 (1H, d), 8.49 (1H, s).

*N*-[5-bromo-2-nitro-4-(trifluoromethyl)phenyl]-1-[*trans*-4-(methyloxy)cyclohexyl]-4-piperidinamine (D14b, 0.236g) was isolated as the slow running isomer. ¹H NMR □ (CDCl₃, 400 MHz): 1.24 (4H, m), 1.65 (2H, m), 1.90 (2H, m), 2.12 (4H, m), 2.38 (1H, m), 2.45 (2H, m), 2.90 (2H, m), 3.10 (1H, m), 3.35 (3H, s), 3.55 (1H, m), 7.17 (1H, s), 8.28 (1H, d), 8.49 (1 H, s).

### Description 15. [2-Amino-5-bromo-4-(trifluoromethyl)phenyl]{1-[cis-4-(methyloxy) cyclohexyl]-4-piperidinyl}amine (D15)

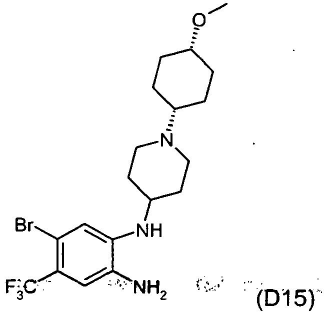

A stirred solution of *N*-[5-bromo-2-nitro-4-(trifluoromethyl)phenyl]-1-[*cis*-4-(methyloxy) cyclohexyl]-4-piperidinamine (D14a, 508mg, 1.06 mmole) in ethanol (40ml) at room temperature under argon was treated with Raney Nickel (∼20mg) followed by a dropwise addition of hydrazine hydrate (0.75ml, 25.2 mmole). The mixture was maintained for 1.8h then filtered through a Kieselguhr pad to remove the catalyst and the solution was concentrated under vacuum to afford the title compound as a pale yellow oil (0.480g, 99%yield).
MH⁺ = 451, 452, 453 and 454.

### Description 16. [2-Amino-5-bromo-4-(trifluoromethyl)phenyl]{1-[trans-4-(methyloxy)cyclohexyl]-4-piperidinyl}amine (D16)

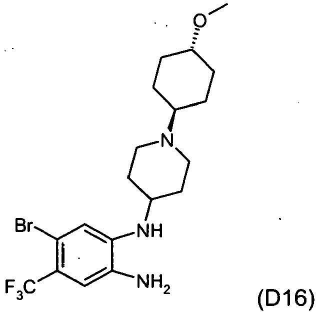

A stirred solution of *N*-[5-bromo-2-nitro-4-(trifluoromethyl)phenyl]-1-[trans-4-(methyloxy) cyclohexyl]-4-piperidinamine (D14b, 236mg, 0.5mmole) in ethanol (30ml) at room temperature under argon was treated with Raney Nickel (~20mg) followed by a dropwise addition of hydrazine hydrate (0.35ml, 11.8mmole). The mixture was maintained for 18h then filtered through a Kieselguhr pad to remove the catalyst and the solution was concentrated under vacuum to afford the title compound (212mg, 97%yield).
MH⁺ = 451, 452, 453 and 454.

### Description 17. 8-(Ethyloxy)-1,4-dioxaspiro[4.5]decane (D17)

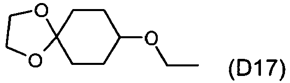

A stirred solution of 1,4-dioxaspiro[4.5]decan-8-ol (D3, 4.0g, 0.025mole) in N-methylpyrrolidinone (35ml) at 10°C under argon was treated portionwise with NaH (1.1g of 60% oil dispersion, 0.028mole) and maintained for 1h, then iodoethane (2.6ml, 0.032mole) was added. The mixture was allowed to warm to room temperature and stir for 18h. Further NaH (0.5g of 60% oil dispersion) was added and the mixture stirred for 0.5h, then more iodoethane (2.0ml) was added and the mixture maintained at room temperature for 3h. The mixture was cautiously treated with ethanol (1ml) to destroy excess NaH and then water (300ml) was added and the mixture extracted with hexane (2 x 200ml). The combined extract as washed with water (2 x 200ml), then dried (Na₂SO₄) and concentrated under vacuum to afford a colourless oil (4.7g) containing the title compound contaminated with mineral oil residues.
¹HNMR □ (CDCl₃, 400 MHz): 1.20 (3H, t), 1.50-1.60 (2H, m), 1.63-1.77 (2H, m), 1.77-1.90 (4H, m), 3.35-3.43 (1H, m), 3.49 (2H, q), 3.90-4.00 (4H, m).

### Description 18. 4-(Ethyloxy)cyclohexanone (D18)

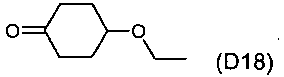

A solution of 8-(ethyloxy)-1,4-dioxaspiro[4.5]decane (D17, 4.7g, 0.025mole) in tetrahydrofuran (10ml) at room temperature under argon was treated with 5M HCl acid (40ml) and stirred for 18h, at which stage conc. HCl acid (5ml) was added and the mixture heated at 40°C for 3.5h to complete the reaction. The resulting mixture was diluted with water (75ml) and extracted with dichloromethane (2 x 80ml). The combined extract was dried (Na₂SO₄) and carefully concentrated under partial vacuum at <20°C to afford the title compound as a pale yellow oil (3.8g, ∼90% purity).
¹HNMR □(CDCl₃, 400 MHz): 1.25 (3H, t), 1.90-2.00 (2H, m), 2.01-2.13 (2H, m), 2.20-2.32 (2H, m), 2.53-2.65 (2H, m), 3.55 (2H, q), 3.58-3.70 (1H, m).

### Description 19. 1,1-Dimethylethyl {1-[cis-4-(ethyloxy)cyclohexyl]-4-piperidinyl}carbamate (D19a) and 1,1-dimethylethyl {1-[trans-4-(ethyloxy)cyclohexyl]-4-piperidinyl}carbamate (D19b)

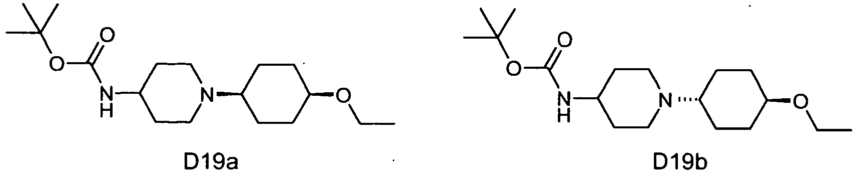

A stirred solution of 4-(ethyloxy)cyclohexanone (D18, 3.5g, 0.025 mole) in dichloromethane (100ml) at room temperature under argon was treated with 1,1-dimethylethyl 4-piperidinylcarbamate (5.0g, 0.025 mole), followed by portionwise addition over 5 minutes of sodium triacetoxyborohydride (7.4g, 0.035mole), then the resulting mixture stirred well at room temperature for 20h. The reaction mixture was treated with methanol (8ml) and the resulting solution allowed to stand for 3 days, then 5% Na₂CO₃ solution (100ml) was added and the mixture extracted with dichloromethane (200ml). The extract was washed with brine, then dried (Na₂SO₄) and concentrated under vacuum. The residual yellow oil was dissolved in 60-80 petrol ether (100ml)) and allowed to stand at room temperature overnight whereupon the crystals which had formed were filtered off, washed with petrol and dried to give 1.7g of ∼45:55 mixture of title compound trans isomer and starting 1,1-dimethylethyl 4-piperidinylcarbamate. Purification by column chromatography on silica gel eluting with 0-5% methanol/dichloromethane afforded the pure title compound *trans* isomer (D19b) as a white solid (0.93g).
¹H NMR □ (CDCl₃, 400 MHz): 1.15-1.50 (6H, m), 1.18 (3H, t), 1.44 (9H, s), 1.82-2.00 (4H, m), 2.04-2.14 (2H, m), 2.20-2.32 (3H, m), 2.85 (2H, br d), 3.10-3.20 (1H, m), 3.38-3.50 (1H, m), 3.50 (2H, q), 4.40 (1H, br d).

Concentration under vacuum of the mother liquors from the crystallisation afforded 6g of a yellow oil containing the title compound *cis* isomer (D19a) as ∼4:1 mixture with the *trans* isomer.

### Description 20. 1-(trans-4-Ethoxycyclohexyl)piperidin-4-amine (D20)

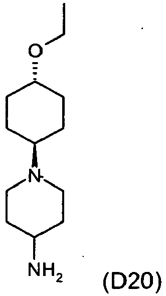

### Method A

1,1-Dimethylethyl {1-[*trans*-4-(ethyloxy)cyclohexyl]-4-piperidinyl}carbamate (D19b, 920mg, 5.0 mmol) was dissolved in dichloromethane (6ml) and treated with HCl (21 ml of a 4M solution in 1,4-dioxane) at room temperature. The mixture was stirred at room temperature for 1 hour and the solvent was evaporated. The crude was passed through an SCX cartridge to yield the free base title compound as a pale brown powder (865mg, 4.0 mmol, 89%). M⁺ + H = 227.

### Method B

A mixture of *trans*-1-(4-ethoxyclohexyl)-4-piperidone (D50, 13.2g), 2M ammonia in methanol (300ml), and 10% palladium on carbon paste (4g) was hydrogenated at 50psi at room temperature for 18h, then filtered and evaporated to give the title compound (9.5g) as a oil.

### Description 21. 1-[trans-4-(Ethyloxy)cyclohexyl]-N-(4-fluoro-5-methyl-2-nitrophenyl)-4-piperidinamine (D21)

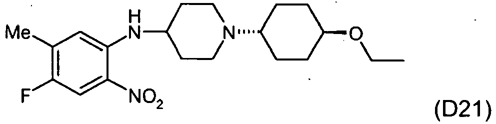

A stirred mixture of 2,5-difluoro-4-nitrotoluene (168mg, 0.972mmole), diisopropylethylamine (1.5eqs, 0.21 ml, 1.21 mmole) and 1-(*trans*-4-ethoxycyclohexyl)piperidin-4-amine (D20, 183mg, 0.810mmole) in dimethylformamide (3ml) was heated at 80°C over one weekend. The mixture was concentrated under vacuum and the residue treated with 10% Na₂CO₃ solution and extracted with dichloromethane. The extract was dried (Na₂SO₄) and concentrated under vacuum. The residue was chromatographed on silica gel (20g) eluting with 0-10% methanol/dichloromethane to afford the title compound as an orange solid (90mg, 29%).
¹H NMR □ (CDCl₃, 400 MHz): 1.17-1.40 (7H including 4H, m and 3H, t), 1.58-1.72 (2H, m), 1.85-2.00 (2H, m), 2.02-2.17 (4H, m), 2.30 (3H, s), 2.30-2.50 (3H, m), 2.82-2.92 (2H, m), 3.12-3.22 (1H, m), 3.47-3.57 (3H including 1H,m and 2H, q), 6.63 (1H, d), 7.82 (1H, d), 8.06 (1H, d).

### Description 22. N-{1-[trans-4-(Ethyloxy)cyclohexyl]-4-piperidinyl}-4-fluoro-5-methyl-1,2-benzenediamine (D22)

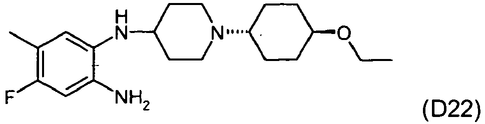

A stirred suspension of 1-[*trans*-4-(ethyloxy)cyclohexyl]-*N*-(4-fluoro-5-methyl-2-nitrophenyl)-4-piperidinamine (D21, 90mg, 0.24mmole) in ethanol (10ml) at room temperature under argon was treated with Raney nickel (-12mg) followed by dropwise addition of hydrazine hydrate (0.073ml, 2.4mmole), then maintained for 1 h. The catalyst was removed by filtration through Kieselguhr and the filtrate concentrated under vacuum to afford the title compound as a pale grey solid (74mg, 89%).
¹H NMR □ (CDCl₃, 400 MHz): 1.16-1.40 (7H including 4H, m and 3H, t), 1.48-1.62 (2H, m), 1.90-2.18 (5H,m), 2.13 (3H, s), 2.32-2.43 (3H, m), 2.88-3.00 (2H, m), 3.08-3.22 (3H, br m), 3.40-3.60 (4H including 2H, q), 6.38-6.48 (2H, m). 1H not discernible.

### Description 23. 8-(Propyloxy)-1,4-dioxaspiro[4.5]decane (D23)

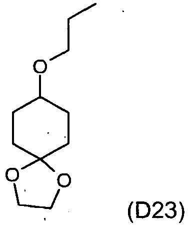

A stirred solution of 1,4-dioxaspiro[4.5]decan-8-ol (D3, 10g, 0.063mole) in N-methylpyrrolidin-2-one (100ml) at 10°C under argon was treated portionwise over 15 minutes with NaH (2.78g of 60% oil dispersion, 0.069mole), then maintained at 10°C for 20 minutes before warming to room temperature over 1 h. The mixture was re-cooled to 5°C and treated dropwise over 10 minutes with 1-bromopropane (8.6ml, 0.095mole), then maintained at 5-10°C for 1h, before warming to room temperature and stirring for 3 days. The mixture was cautiously treated with methanol (5ml) to destroy remaining NaH, then water (1200ml) was added and the mixture extracted with n-hexane (3 x 300ml). The combined extract was washed with water (2 x 250ml), dried (Na₂SO₄) and concentrated under vacuum to afford the title compound as a colourless oil (9.96g, 79%).
¹H NMR δ (CDCl₃ 400MHz): 0.91 (3H, t), 1.48-1.62 (4H, m), 1.62-1.90 (6H, m), 3.35-3.42 (3H, m), 3.90-4.00 (4H, m).

### Description 24. 4-(Propyloxy)cyclohexanone (D24)

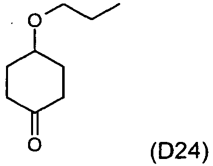

A stirred solution of 8-(propyloxy)-1,4-dioxaspiro[4.5]decane (D23, 9.96g, 0.050mole) in tetrahydrofuran (20ml) at room temperature under argon was treated with 5M HCl acid (100ml) and maintained for 18h. The mixture was diluted with water (300ml) and extracted with dichloromethane (2 x 250ml). The combined extract was washed with dil. brine, then dried (Na₂SO₄) and concentrated under vacuum at ∼15C to leave the title compound as a colourless oil (7.8g, 100%).
¹H NMR δ (CDCl₃ 400MHz): 0.95 (3H, t), 1.57-1.69 (2H, m), 1.87-2.00 (2H, m), 2.04-2.14 (2H, m), 2.20-2.32 (2H, m), 2.52-2.63 (2H, m), 3.46 (2H, t), 3.67-3.72 (1H, m).

### Description 25.1,1-Dimethylethyl {1-[cis-4-(propyloxy)cyclohexyl]-4-piperidinyl}carbamate (D25a) and 1,1-dimethylethyl {1-[trans-4-(propyloxy)cyclohexyl]-4-piperidinyl}carbamate (D25b)

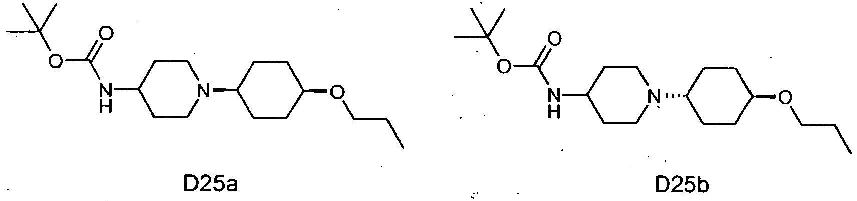

A stirred solution of 4-(propyloxy)cyclohexanone (D24, 7.8g, 0.050 mole) in dichloromethane (200ml) at room temperature under argon was treated with 1,1-dimethylethyl 4-piperidinylcarbamate (10.0g, 0.050 mole), followed by portionwise addition over 10 minutes of sodium triacetoxyborohydride (14.8g, 0.070 mole). The resulting mixture was diluted with further dichloromethane (50ml) and stirred well at room temperature for 18h. The reaction mixture was carefully treated with methanol (10ml) and the resulting solution stirred for 1h, then 2% Na₂CO₃ solution (200ml) was added and the mixture extracted with dichloromethane (2 x 200ml). The combined extract was washed with water (200ml), then dried (Na₂SO₄) and concentrated under vacuum. The residual yellow oil was dissolved in 60-80 petrol ether (120ml) and allowed to stand at room temperature overnight whereupon the crystals which had formed were filtered off, washed with petrol and dried to give 0.95g of title compound *trans* isomer (D25b) (>90% purity) as a white solid. Concentration of the mother liquors and a second crystallisation of the residue from 60-80 petrol (80ml) over 4h afforded a further 0.65g of *trans* isomer (∼90% purity) as a white solid.
¹H NMR □ (CDCl₃, 400 MHz): 0.91 (3H, t), 1.15-1.33 (4H, m), 1.33-1.50 (2H, m), 1.44 (9H, s), 1.56 (2H, m), 1.85-2.00 (4H, m), 2.05-2.14 (2H, m), 2.20-2.35 (3H, m), 2.80-2.90 (2H, m), 3.10-3.20 (1H, m), 3.40 (2H, t), 3.4-3.50 (1H, m), 4.40 (1H, br d).

Concentration under vacuum of the mother liquors from the second crystallisation afforded 13.9g of a yellow oil containing the title compound *cis* isomer (D25a) as ∼3:1 mixture with the *trans* isomer.

### Description 26. 1-[trans-4-(Propyloxy)cyclohexyl]-4-piperidinamine (D26)

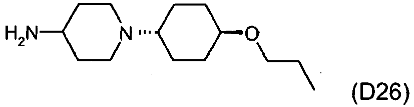

A solution of 1,1-dimethylethyl {1-[*trans*-4-(propyloxy)cyclohexyl]-4-piperidinyl}carbamate (D25b, 0.95g, 2.8mmole) in dichloromethane (20ml) at room temperature under argon was treated with 1 M HCl/diethyl ether (25ml) and allowed to stand for 18h when a white solid had formed, however reaction was only partially complete. The mixture was concentrated under vacuum and the residue dissolved in methanol (20ml) and treated with a concentrated solution of HCl in methanol. After 3h the solution was concentrated under vacuum and the residual gum treated with toluene and then concentrated under vacuum to remove water, however no solid was obtained. The gum was therefore treated with ethyl acetate and excess 10% Na₂CO₃ solution, shaken well and the ethyl acetate layer separated, dried (Na₂SO₄) and concentrated under vacuum to afford the title compound as a white semi-solid (0.50g, 75%).
¹H NMR □ (CDCl₃, 400 MHz): 0.90 (3H, t), 1.15-1.45 (8H, m), 1.57 (2H, m), 1.82 (2H, br d), 1.87-1.95 (2H, m), 2.03-2.12 (2H, m), 2.21 (2H, dt), 2.22-2.32 (1H, m), 2.56-2.66 (1H, m), 2.86 (2H, br d), 3.10-3.20 (1H, m), 3.40 (2H, t).

### Description 27. 1-[trans-4-(Propyloxy)cyclohexyl]-4-piperidinamine dihydrochloride (D27)

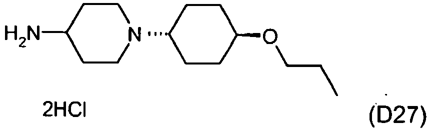

### Method A

A stirred solution of 1,1-dimethylethyl {1-[*trans*-4-(propyloxy)cyclohexyl]-4-piperidinyl}carbamate (D25b, 0.65g, 1.9mmole) in dichloromethane (15ml) at room temperature under argon was treated with 4M HCl/dioxane (5ml) and maintained for 3 days. The mixture containing a white precipitate was concentrated under vacuum and then dried at 50°C under vacuum to afford the title compound as a white solid (0.60g, 100%).

### Method B

A mixture of *trans*-1-(4-propoxycyclohexyl)-4-piperidone (D53, 7.5g), 2M ammonia in methanol (100ml), and 10% palladium on carbon paste (100mg) was hydrogenated at 50psi at room temperature for 18h then filtered and evaporated. The residue was converted to the dihydrochloride salt to give the title compound, crystallised from diethyl ether, 7.5g.

### Description 28. cis/trans-1-[4-(Propyloxy)cyclohexyl]-4-piperidinamine dihydrochloride (D28)

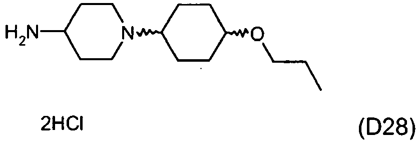

A stirred solution of *cis*/*trans*-1,1-dimethylethyl {1-[cis-4-(propyloxy)cyclohexyl]-4-piperidinyl}carbamate (D25a, ∼3:1 *cis:trans,* 7.0g, 0.021mole) in dichloromethane (50ml) at room temperature under argon was treated with 4M HCl/dioxane (25ml) and maintained for 3 days. The mixture containing a white precipitate was concentrated under vacuum and then dried at 50°C under vacuum for 1h to afford the title compound (-3:1 mixture of *cis:trans* isomers) as a white solid (6.7g, 100%).

### Description 29. N-(4-Chloro-5-methyl-2-nitrophenyl)-1-[cis-4-(propyloxy)cyclohexyl]-4-piperidinamine (D29)

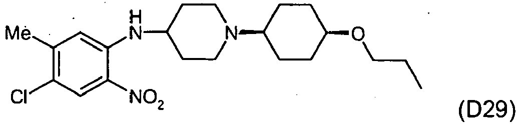

A stirred suspension of *cis*/*trans*-1-[4-(propyloxy)cyclohexyl]-4-piperidinamine dihydrochloride (D28, ∼3:1 cis:trans mixture, 1.5g, 4.8mmole) in dimethylformamide (12ml) at room temperature under argon was treated with diisopropylethylamine (3.0ml, 17mmole) and 2-chloro-5-fluoro-4-nitrotoluene (D1, 1.05g, 5.5mmole) and heated at 80°C. for 18h. The resulting solution was concentrated under vacuum and the residue treated with 10% Na₂CO₃solution (40ml) and extracted with dichloromethane. The extract was dried, concentrated under vacuum to ∼5ml and loaded onto a SCX cartridge (10g), which was eluted with dichloromethane followed by methanol to remove non-basic contaminants, then with 2M NH₃/methanol to remove the product. The product solution was concentrated under vacuum and the residue chromatographed on NH-silica column (100g) eluting with 0-20% ethyl acetate/hexane to obtain the title compound as the fastest eluting isomer. This was obtained as an orange solid (0.68g, 35%).
¹H NMR □(CDCl₃, 400 MHz): 0.94 (3H, t), 1.33-1.45 (2H, m), 1.50-1.75 (8H, m), 1.93-2.03 (2H, m), 2.03-2.13 (2H, m), 2.32-2.41 (1H, m and 3H, s), 2.41-2.51 (2H, m), 2.84-2.94 (2H, m), 3.34 (2H, t), 3.45-3.57 (2H, m), 6.71 (1H, s), 8.08 (1H, d), 8.16 (1H, s).
Latter fractions contained a small amount of the pure *trans* isomer (112mg).

### Description 30. 4-Chloro-5-methyl-N-{1-[cis-4-(propyloxy)cyclohexyl]-4-piperidinyl}-1,2-benzenediamine (D30)

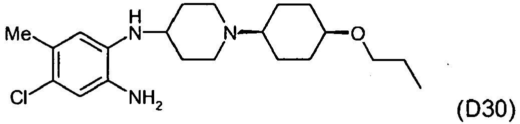

A stirred suspension of *N*-(4-chloro-5-methyl-2-nitrophenyl)-1-[*cis*-4-(propyloxy)cyclohexyl]-4-piperidinamine (D29, 680mg, 1.66mmole) in ethanol (40ml) at room temperature under argon was treated with Raney nickel (∼20mg) followed by dropwise addition of hydrazine hydrate (0.50ml, 16mmole). The mixture was maintained for 3 days then filtered through Kieselguhr to remove the catalyst and the filtrate concentrated under vacuum to afford the title compound as a pale purple oil (630mg, 100%).
¹H NMR □ (CDCl₃, 400 MHz): 0.94 (3H, t), 1.30-1.45 (2H, m), 1.45-1.75 (8H, m), 1.92-2.02 (2H, m), 2.02-2.12 (2H, m), 2.25 (3H, s), 2.32-2.45 (3H, m), 2.89-2.98 (2H, m), 3.00-3.40 (4H, br), 3.33 (2H, t), 3.45-3.50 (1H, m), 6.47 (1H, s), 6.70 (1H, s).

### Description 31. N-(4-Chloro-5-methyl-2-nitrophenyl)-1-[trans--4-(propyloxy)-cyclohexyl]-4-piperidinamine (D31)

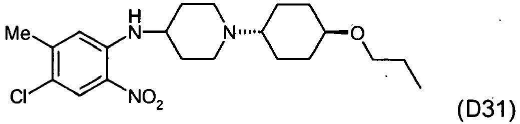

A stirred solution of 1-[*trans*-4-(propyloxy)cyclohexyl]-4-piperidinamine (D26, 250mg, 1.0mmole) and diisopropylethylamine (0.43ml, 2.4mmole) in dimethylformamide (4ml) at room temperature under argon was treated with 2-chloro-5-fluoro-4-nitrotoluene (D1, 227mg, 1.2mmole) and heated at 70°C for 18h. The resulting solution was concentrated under vacuum and the residue treated with 10% Na₂CO₃solution (20ml) and extracted with dichloromethane. The extract was dried, concentrated under vacuum to ∼4ml and loaded onto a SCX cartridge (5g), which was eluted with dichloromethane followed by methanol to remove non-basic contaminants, then with 2M NH3/methanol to remove the product. The product solution was concentrated under vacuum and the residue crystallised from methanol to afford the title compound as a crystalline orange solid (310mg, 76%).
¹H NMR □ (CDCl₃, 400 MHz): 0.93 (3H, t), 1.17-1.37 (4H, m), 1.52-1.70 (4H, m), 1.88-1.95 (2H, m), 2.01-2.15 (4H, m), 2.29-2.49 (3H, m), 2.37 (3H, s), 2.83-2.92 (2H, m), 3.10-3.20 (1H, m), 3.40 (2H, t), 3.46-3.58 (1H, m), 6.70 (1H, s), 8.08 (1H, d), 8.17 (1H, s).

### Description 32. 4-Chloro-5-methyl-N-{1-[trans-4-(propyloxy)cyclohexyl]-4-piperidinyl}-1,2-benzenediamine (D32)

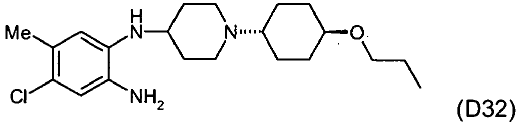

A stirred suspension of *N*-(4-chloro-5-methyl-2-nitrophenyl)-1-[*trans*-4-(propyloxy)cyclohexyl]-4-piperidinamine (D31, 420mg, 1.02mmole) in ethanol (15ml) at room temperature under argon was treated with Raney nickel (∼20mg) followed by dropwise addition of hydrazine hydrate (0.30ml, 9.6mmole). The mixture was stirred at room temperature for 2h followed by 45°C for 1h, then filtered through Kieselguhr to remove the catalyst and the filtrate concentrated under vacuum to afford the title compound as a pale purple solid (360mg, 93%).
¹H NMR □ (CDCl₃, 400 MHz): 0.91 (3H, t), 1.18-1.38 (4H, m), 1.40-1.52 (2H, m), 1.52-1.62 (2H, m), 1.88-1.96 (2H, m), 2.00-2.15 (4H, m), 2.25 (3H, s), 2.28-2.40 (3H, m), 2.85-2.95 (2H, m), 3.10-3.30 (5H, br m), 3.40 (2H, t), 6.47 (1H, s), 6.70 (1H, s).

### Description 33. N-(4-Fluoro-5-methyl-2-nitrophenyl)-1-[cis-4-(propyloxy)cyclohexyl]-4-piperidinamine (D33)

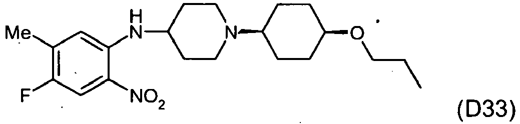

A stirred solution of 2,5-difluoro-4-nitrotoluene (0.95g, 5.5mmole) in dimethylformamide (12ml) at room temperature under argon was treated with diisopropylethylamine (3.0ml, 17mmole) and *cis*/*trans*-1-[4-(propyloxy)cyclohexyl]-4-piperidinamine dihydrochloride (D28, -3:1 *cis:trans* mixture, 1.5g, 4.8mmole) and heated at 80°C for 24h. The resulting solution was concentrated under vacuum and the residue treated with 10% Na₂CO₃ solution (40ml) and extracted with dichloromethane (40ml). The extract was dried, concentrated under vacuum to ∼5ml and loaded onto a SCX cartridge (10g), which was eluted with dichloromethane followed by methanol to remove non-basic contaminants, then with 2M NH₃/methanol to remove the product. The product solution was concentrated under vacuum and the residue chromatographed on NH-silica column (100g) eluting with 0-20% ethyl acetate/hexane to obtain the title compound as the fastest eluting isomer. This was obtained as an orange solid (0.675g, 36%).
¹H NMR □(CDCl₃, 400 MHz): 0.94 (3H, t), 1.34-1.44 (2H, m), 1.53-1.80 (8H, m), 1.92-2.02 (2H, m), 2.02-2.12 (2H, m), 2.29 (3H, s), 2.30-2.40 (1H, m), 2.40-2.50 (2H, m), 2.82-2.92 (2H, m), 3.35 (2H, t), 3.45-3.55 (2H, m), 6.64 (1H, d), 7.82 (1H, d), 8.06 (1H, d).
Latter fractions afforded some of the trans isomer (190mg, >90% purity).

### Description 34. 4-Fluoro-5-methyl-N-{1-[cis-4-(propyloxy)cyclohexyl]-4-piperidinyl)-1,2-benzenediamine (D34)

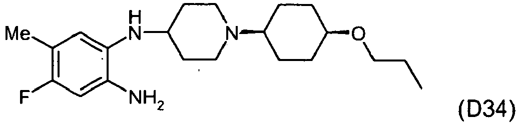

A stirred suspension of *N*-(4-fluoro-5-methyl-2-nitrophenyl)-1-[*cis*-4-(propyloxy)cyclohexyl]-4-piperidinamine (D33, 330mg, 0.84mmole) in ethanol (25ml) at room temperature under argon was treated with Raney nickel (∼20mg) followed by dropwise addition of hydrazine hydrate (0.25ml, 8.4mmole). The mixture was maintained for 4h, then filtered through Kieselguhr to remove the catalyst and the filtrate concentrated under vacuum to afford the title compound as a pale purple oil (290mg, 95%).
¹H NMR □(CDCl₃, 400 MHz): 0.92 (3H, t), 1.30-1.42 (2H, m), 1.50-1.75 (8H, m), 1.95-2.10 (4H, m), 2.15 (3H, s), 2.35-2.50 (3H, m), 2.90 (1H, vbr), 2.90-3.02 (2H, m), 3.08-3.18 (1H, m), 3.33 (2H, t), 3.47 (3H, br s), 6.42 (1H, d), 6.45 (1H, d).

### Description 35. N-(4-Fluoro-5-methyl-2-nitrophenyl)-1-[trans--4-(propyloxy-cyclohexyl]-4-piperidinamine (D35)

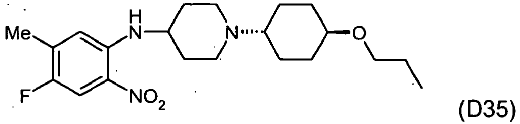

A stirred solution of 1-[*trans*-4-(propyloxy)cyclohexyl]-4-piperidinamine (D26, 250mg, 1.0mmole) and diisopropylethylamine (0.43ml, 2.4mmole) in dimethylformamide (4ml) at room temperature under argon was treated with 2,5-difluoro-4-nitrotoluene (208mg, 1.2mmole) and heated at 70°C for 18h. The resulting solution was concentrated under vacuum and the residue treated with 10% Na₂CO₃ solution (20ml) and extracted with dichloromethane (25ml). The extract was dried, concentrated under vacuum to -4ml and loaded onto a SCX cartridge (5g), which was eluted with dichloromethane followed by methanol to remove non-basic contaminants, then with 2M NH₃/methanol to remove the product. The product solution was concentrated under vacuum and the residue crystallised from methanol to afford the title compound as a dark orange solid (190mg, 48%).
¹H NMR □ (CDCl₃, 400 MHz): 0.91 (3H, t), 1.18-1.38 (4H, m), 1.50-1.72 (4H, m), 1.88-1.98 (2H, m), 2.00-2.15 (4H, m), 2.25-2.38 (1H, m), 2.30 (3H, s), 2.38-2.50 (2H, m), 2.83-2.93 (2H, m), 3.10-3.22 (1H, m), 3.41 (2H, t), 3.45-3.58 (1H, m), 6.63 (1H, d), 7.82 (1H, d), 8.06 (1H, d).

### Description 36. 4-Fluoro-5-methyl-N-{1-[trans-4-(propyloxy)cyclohexyl]-4-piperidinyl}-1,2-benzenediamine (D36)

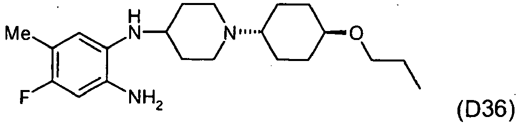

A stirred suspension of *N*-(4-fluoro-5-methyl-2-nitrophenyl)-1-[*trans*-4-(propyloxy)cyclohexyl]-4-piperidinamine (D35, 190mg, 0.46mmole) in ethanol (15ml) at room temperature under argon was treated with Raney nickel (-15mg) followed by dropwise addition of hydrazine hydrate (0.14ml, 4.6mmole). The mixture was stirred at room temperature for 3h, then filtered through Kieselguhr to remove the catalyst and the filtrate concentrated under vacuum to afford the title compound as a grey semi-solid (158mg, 90%).
¹H NMR □ (CDCl₃, 400 MHz): 0.91 (3H, t), 1.17-1.37 (4H, m), 1.40-1.52 (2H, m), 1.52-1.65 (2H, m), 1.88-1.97 (2H, m), 1.98-2.14 (4H, m), 2.16 (3H, s), 2.25-2.38 (3H, m), 2.3-3.0 (1H, vbr), 2.85-2.95 (2H, m), 3.04-3.20 (2H, m), 3.40 (2H, t), 3.45 (2H, br s), 6.41 (1H, d), 6.44 (1H, d).

### Description 37. N-[5-Bromo-2-nitro-4-(trifluoromethyl)phenyl]-1-[cis-4-(propyloxy)cyclohexyl]-4-piperidinamine (D37)

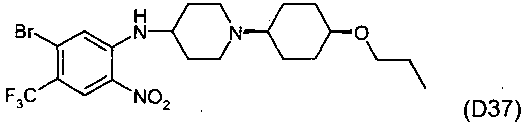

A stirred suspension of *cis*/*trans*-1-[4-(propyloxy)cyclohexyl]-4-piperidinamine dihydrochloride (D28, ∼3:1 *cis:trans* mixture, 1.0g, 3.2mmole) in dimethylformamide (10ml) at room temperature under argon was treated with diisopropylethylamine (2.0ml, 11mmole) and 1-bromo-5-fluoro-4-nitro-2-(trifluoromethyl)benzene (D2, 1.10g, 3.8mmole) and heated at 80°C for 2h. The resulting solution was concentrated under vacuum and the residue treated with 10% Na₂CO₃ solution (40ml) and extracted with dichloromethane. The extract was dried, concentrated under vacuum to ∼5ml and loaded onto a SCX cartridge (10g), which was eluted with dichloromethane followed by methanol to remove non-basic contaminants, then with 2M NH₃/methanol to remove the product. The product solution was concentrated under vacuum and the residue chromatographed on NH-silica column (100g) eluting with 0-20% ethyl acetate/hexane to obtain the title compound as the fastest eluting isomer. This was obtained as an orange solid (0.744g, 38%).
¹H NMR □ (CDCl₃, 400 MHz): 0.93 (3H, t), 1.33-1.45 (2H, m), 1.50-1.75 (8H, m), 1.94-2.02 (2H, m), 2.03-2.14 (2H, m), 2.33-2.42 (1H, m), 2.43-2.53 (2H, m), 2.85-2.95 (2H, m), 3.35 (2H, t), 3.45-3.60 (2H, m), 7.18 (1H, s), 8.30 (1H, d), 8.49 (1H, s).
Latter fractions contained a small amount of the trans isomer (108mg; >95% purity).

### Description 38. 5-Bromo-N-{1-[cis-4-(propyloxy)cyclohexyl]-4-piperidinyl}-4-(trifluoromethyl)-1,2-benzenediamine (D38)

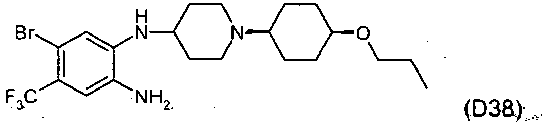

A stirred suspension of *N*-[5-bromo-2-nitro-4-(trifluoromethyl)phenyl]-1-[*cis*-4-(propyloxy)cyclohexyl]-4-piperidinamine (D37, 744mg, 1.46mmole) in ethanol (35ml) at room temperature under argon was treated with Raney nickel (-30mg) followed by dropwise addition of hydrazine hydrate (0.39ml, 12.5mmole). The mixture was maintained at room temperature for 18h, then filtered through Kieselguhr to remove the catalyst and the filtrate concentrated under vacuum to leave a pale red solid (700mg). A portion of this material (350mg) was purified by mass-directed automated H PLC to afford the title compound as a pale grey solid (190mg, 54%).
¹H NMR □(CDCl₃, 400 MHz): 0.94 (3H, t), 1.33-1.43 (2H, m), 1.43-1.75 (8H, m), 1.93-2.02 (2H, m), 2.02-2.12 (2H, m), 2.30-2.45 (3H, m), 2.88-2.98 (2H, m), 3.25 (3H, br s), 3.33 (2H, t), 3.48 (1H, m), 3.70 (1H, d), 6.81 (1H, s), 6.98 (1H, s).

### Description 39. N-[5-Bromo-2-nitro-4-(trifluoromethyl)phenyl]-1-[trans-4-(propyloxy)cyclohexyl]-4-piperidinamine (D39)

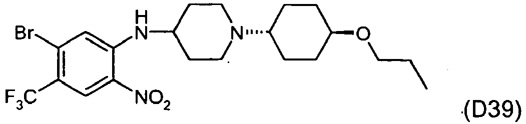

A stirred solution of 1-[*trans*-4-(propyloxy)cyclohexyl]-4-piperidinamine dihydrochloride (D27, 400mg, 1.3mmole) and diisopropylethylamine (0.81ml, 4.5mmole) in dimethylformamide (5ml) at room temperature under argon was treated with 1-bromo-5-fluoro-4-nitro-2-(trifluoromethyl)benzene (D2, 430mg, 1.5mmole) and heated at 70°C for 18h. The resulting solution was concentrated under vacuum and the residue treated with 10% Na₂CO₃ solution (20ml) and extracted with dichloromethane. The extract was dried, concentrated under vacuum to -4ml and loaded onto a SCX cartridge (5g), which was eluted with dichloromethane followed by methanol to remove non-basic contaminants, then with 2M NH₃/methanol to remove the product. The product solution was concentrated under vacuum and the residue chromatographed on NH-silica column (100g) eluting with 0-20% ethyl acetate/hexane to obtain the title compound a yellow solid (238mg, 47%).
¹H NMR (CDCl₃, 400 MHz): 0.92 (3H, t), 1.20-1.40 (4H, m), 1.52-1.80 (4H, m), 1.88-1.98 (2H, m), 2.02-2.16 (4H, m), 2.30-2.40 (1H, m), 2.40-2.52 (2H, m), 2.84-2.94 (2H, m), 3.12-3.20 (1H, m), 3.40 (2H, m), 3.50-3.60 (1H, m), 7.18 (1H, s), 8.29 (1H, d), 8.50 (1H, s).

### Description 40. 5-Bromo-N-{1-[trans-4-(propyloxy)cyclohexyl]-4-piperidinyl}-4-(trifluoromethyl)-1,2-benzenediamine (D40)

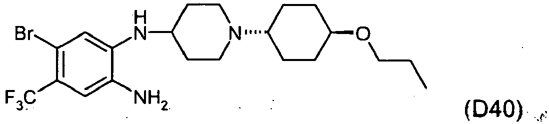

*N*-[5-bromo-2-nitro-4-(trifluoromethyl)phenyl]-1-[*trans*-4-(propyloxy)cyclohexyl]-4-piperidinamine (D39, 138mg, 0.3mmole) was dissolved in ethanol (3ml) and an aqueous suspension of Raney Nickel (1 ml) was added at room temperature. The mixture was stirred for 30 minutes before adding hydrazine monohydrate (0.12 ml, 15eq., 4.0 mmole) over 30 minutes. The reaction was left overnight. The reaction mixture was filtered through celite and the solvent was evaporated to yield the title compound as a brown solid (175 mgs, 100%). M⁺ + H = 480.

### Description 41. 8-[(1-Methylethyl)oxy]-1,4-dioxaspiro[4.5]decane (D41)

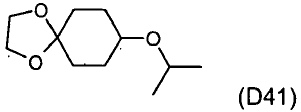

A mixture of 1,4-dioxaspiro[4.5]decan-8-ol (D3, 5.0g, 0.032mole), 2-iodopropane (25ml) and silver (I) oxide (14g, 0.060mole) at room temperature under argon was stirred in the dark for 6 days, followed by 6 days standing. The mixture was treated with diethyl ether (40ml) and filtered through Kieselguhr washing well with diethyl ether. The filtrate was concentrated under vacuum and the residue dissolved in hexane (150ml), washed with water (150ml), then dried (Na₂SO₄) and concentrated under vacuum to afford a colourless oil (4.89g) containing approx. 80% of the title compound together with 4-[(1-methylethyl)oxy]cyclohexanone from ketal hydrolysis. This mixture was used in the next step without purification.
¹H NMR □ (CDCl₃, 400 MHz): 1.13 (6H, d), 1.5-1.59 (2H, m), 1.60-1.74 (2H, m), 1.75-1.88 (4H, m), 3.43-3.50 (1H, m), 3.62-3.72 (1H, m), 3.90-4.00 (4H, m).

### Description 42. 4-[(1-Methylethyl)oxy]cyclohexanone (D42)

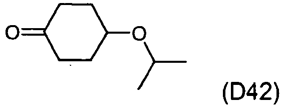

A solution of the mixture of 8-[(1-methylethyl)oxy]-1,4-dioxaspiro[4.5]decane and 4-[(1-methylethyl)oxy]cyclohexanone (∼4:1) (D41 , 4.89g, ∼0.025mole) in tetrahydrofuran (10ml) at room temperature under argon was treated with 5M HCl acid (50ml) and stirred well for 18h. The reaction mixture was treated with water (150ml) and extracted with dichloromethane (2 x 80ml). The combined extract was washed with brine, dried (Na₂SO₄) and concentrated under vacuum to afford the title compound as a colourless oil (3.9g, 100%).
¹H NMR □ (CDCl₃, 400 MHz): 1.19(6H, d), 1.88-2.08 (4H, m) 2.22-2.32 (2H, m), 2.54-2.65 (2H, m), 3.70-3.84 (2H, m).

### Description 43. 1,1-Dimethylethyl (1-{cis-4-[(1-methylethyl)oxy]cyclohexyl}-4-piperidinyl)carbamate (D43a) and 1,1-dimethylethyl (1-{trans-4-[(1-methylethyl)oxy]cyclohexyl}-4-piperidinyl)carbamate (D43b)

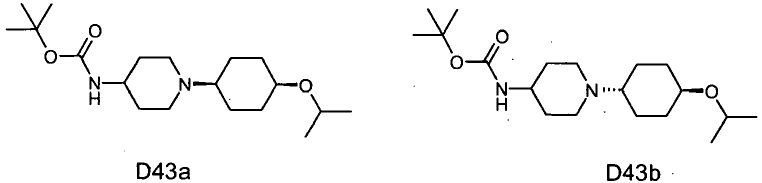

A stirred solution of 4-[(1-methylethyl)oxy]cyclohexanone (D42, 3.9g, 0.025 mole) in dichloromethane (100ml) at room temperature under argon was treated with 1,1-dimethylethyl 4-piperidinylcarbamate (5.0g, 0.025 mole), followed by portionwise addition over 10 minutes of sodium triacetoxyborohydride (7.4g, 0.035mole). Further dichloromethane (50ml) was added to aid stirring which was continued at room temperature for 18h. The reaction mixture was carefully treated with methanol (5ml) and stirred for 20 minutes, then 2% Na₂CO₃solution (200ml) was added and the mixture extracted with dichloromethane (2 x 150ml). The combined extract was washed with brine, then dried (Na₂SO₄) and concentrated under vacuum. The residual yellow oil (8.6g) was triturated with 60-80 petrol ether (80ml) which caused immediate crystallisation of a white solid. This was filtered off, washed with 60-80 petrol ether and dried, then recrystallised from 9:1 60-80 petrol ether/dichloromethane to afford the title compound *trans* isomer (D43b) as a white solid (0.85g).
¹H NMR □ (CDCl₃, 400 MHz): 1.12 (6H, d), 1.18-1.50 (6H, m), 1.44 (9H, s), 1.80-2.05 (6H, m), 2.20-2.32 (3H, m), 2.80-2.90 (2H, m), 3.15-3.26 (1H, m), 3.38-3.50 (1H, m), 3.64-3.73 (1H, m), 4.37-4.47 (1H, m).
Concentration under vacuum of the mother liquors from the first crystallisation afforded 7.3g of a yellow oil containing the title compound *cis* isomer (D43a) as -4:1 mixture with the *trans* isomer.

### Description 44. 1-{trans-4-[(1-Methylethyl)oxy]cyclohexyl}-4-piperidinamine dihydrochloride (D44)

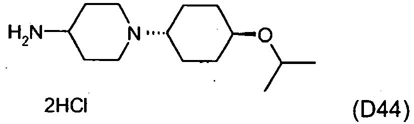

A stirred solution of 1.1-dimethylethyl.(1-{*trans*-4-[(1-methylethyl)oxy]cyclohexyl}-4-piperidinyl}carbamate (D43b, 0.85g, 2.5mmole) in dichloromethane (10ml) at room temperature under argon was treated with 4M HCl/dioxane (5ml) and maintained for 18h, then concentrated under vacuum. The residue was treated with diethyl ether (20ml), stirred for 5 minutes, then the solid filtered off, washed with diethyl ether and then dried to afford the title compound as a white solid (0.73g, 93%).
¹H NMR □ (d⁶DMSO, 400 MHz): 1.05 (6H, d), 1.08-1.22 (2H, m), 1.43-1.60 (2H, m), 1.93-2.20 (8H, m), 2.98-3.15 (3H, m), 3.20-3.55 (5H, m), 3.62-3.72 (1H, m), 8.46 & 8.60 (together 3H, 2 x brs), 10.84 & 10.95 (together 1H, 2 x brs).

### Description 45. trans-N-(4-Hydroxycyclohexyl)phthalimide (D45)

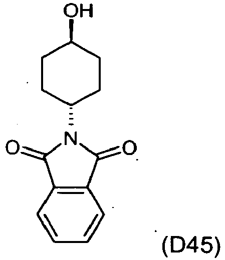

N-Ethoxycarbonylphthalimide (100g) was added to a mixture of *trans*-4-hydroxycyclohexylamine hydrochloride (69g), potassium carbonate (158g) and water (1l) at room temperature. After 3h the title compound was isolated by filtration, washing with water then ethyl acetate, 95g.

### Description 46. trans-N-(4-tert-Butyldimethylsilyloxycyclohexyl)phthalimide (D46)

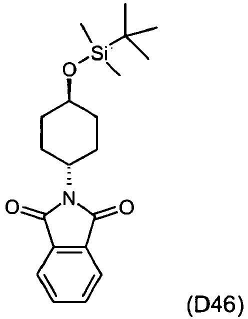

Tert-butyldimethylsilyl chloride (60g) was added in portions to a mixture of *trans*-N-(4-hydroxycyclohexyl)phthalimide (D45, 95g), imidazole (55g), and dimethylformamide (200ml) at 20-30°C (internal, ice cooling). After stirring for 3h more at 40°C the mixture was partitioned water / hexane. Drying and evaporation of the organic layer gave the title compound crystallised from pentane, 92g.

### Description 47. trans-N-(4-Ethoxycyclohexyl)phthalimide (D47)

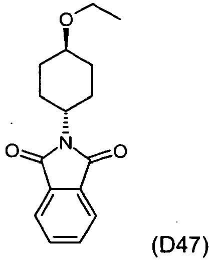

Acetaldehyde (10ml) in acetonitrile (50ml) was added over 30 minutes to a solution of 2-(*trans*-4-{[(1,1-dimethylethyl)(dimethyl)silyl]oxy}cyclohexylyl-1H-isoindole-1,3(2H)-dione (D46, 50.0g), bismuth tribromide (6.7g), triethylsilane (27ml) in acetonitrile (500ml) stirred at ice bath temperature and the mixture was allowed to warm to room temperature overnight. The mixture was filtered and the resulting grey solid and filtrate were worked up separately. The filtrate was evaporated and treated with hexane to give the title compound as a white solid (16.35g). The grey solid washed with dichloromethane, the dichloromethane extract was evaporated and the residue was stirred with hexane (200ml) to give a second crop of the title compound as a white solid (17.47g).

### Description 48. trans-4-Ethoxycyclohexylamine (D48)

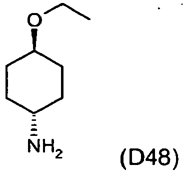

A solution of *trans*-N-(4-ethoxycyclohexyl)phthalimide (16.35g), hydrazine hydrate (12ml) in ethanol (300ml) and methanol (200ml) was stirred at reflux for 3 hours. The solvent was removed to give a slurry, which was treated with diethyl ether and filtered. The filtrate was evaporated to afford the title compound as a viscous oil contaminated with ether (8.16g).

### Description 49. 1-Ethyl-1-methyl-4-oxopiperidinium iodide (D49)

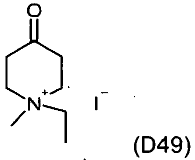

lodomethane (65ml) was added in portions to a solution of 1-ethyl-4-piperidone (100g) in acetone (11) at 20-30°C (internal, ice cooling). After stirring for 3h more the title compound was obtained by filtration, 189g.

### Description 50. trans-1-(4-Ethoxycyclohexyl)-4-piperidone (D50)

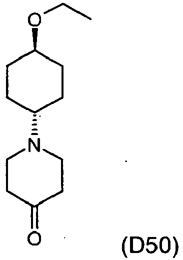

A mixture of 1-ethyl-4-piperidone methiodide (D49, 27g), *trans*-4-ethoxycyclohexylamine (D48, 8.16g), potassium carbonate (13.5g), water (100ml), and ethanol (200ml) was heated for 3 hours at 80°C, then cooled overnight. The mixture was partitioned with aqueous sodium bicarbonate and dichloromethane. The dichloromethane layer was separated, washed with brine and solvent removed to give the title compound as an amber coloured oil (13.2g).

### Description 51. trans-N-(4-Propoxycyclohexyl)phthalimide (D51)

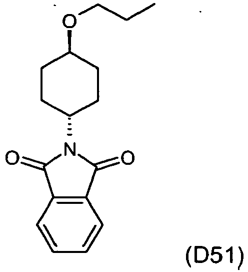

A solution of *trans*-N-(4-tert-butyldimethylsilyloxycyclohexyl)phthalimide (D46, 45g) in acetonitrile (500ml) at room temperature was treated sequentially with triethylsilane (24ml), bismuth tribromide (6g), and (dropwise) propanal (11ml) at 20-30°C (internal, ice cooling). After 30min more the solution was partitioned between aqueous sodium bicarbonate / ethyl acetate. Drying and evaporation of the organic layer gave the title compound crystallised from pentane, 20g.

### Description 52. trans-4-Propoxycyclohexylamine (D52)

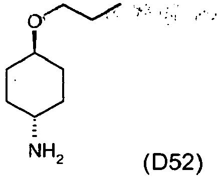

A mixture of *trans*-N-(4-propoxycyclohexyl)phthalimide (D51, 20g), hydrazine hydrate (15ml), and ethanol (400ml) was heated at 80°C for 2h then cooled and filtered. The filtrate was evaporated and the resulting residue redissolved in diethyl ether, filtered and again evaporated to give the title compound, 11g.

### Description 53. trans-1-(4-Propoxycyclohexyl)-4-piperidone (D53)

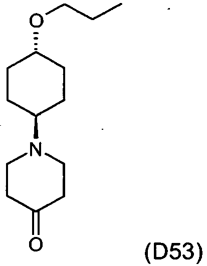

1-Ethyl-4-piperidone methiodide (D49, 26g) was added to a refluxing mixture of *trans*-4-propoxycyclohexylamine (D52, 11g), , potassium carbonate (1g), water (75ml), and ethanol (150ml) over 30min, and the mixture was then heated for another 30min at 80°C, then cooled, partitioned between aqueous sodium bicarbonate / dichloromethane, and purified by chromatography (40+M Biotage silica column, 0 to 10% methanol in dichloromethane containing 0.2M ammonia) to give the title compound, 8g.

### Description 54. cis/trans-1-Methyl-4-(methyloxy)cyclohexanecarboxylic acid (D54)

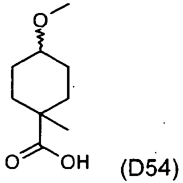

A stirred solution of diisopropylamine (16.1ml, 115 mmol, 2.1 eq.) in tetrahydrofuran (200ml) at 0°C under argon was treated with 2.5M n-butyllithium in hexane (1.05 eq., 110 mmol, 44ml) and it was then stirred at the same temperature for 10 minutes. The mixture was then treated with a solution of 4-(methyloxy)cyclohexanecarboxylic acid (1 eq., 55 mmol, 8.7g) in 50 ml of dry tetrahydrofuran. The resulting yellow solution was heated at 50°C for 2.0 hrs, then cooled to 0°C and treated with iodomethane (3 eq., 12 ml). The mixture was allowed to warm to room temperature and stir for 2 hrs. The mixture was partitioned between citric acid (10% aqueous solution) and Et₂O, the two phases were separated and the aqueous was extracted with Et₂O (2x). Organics were combined, dried (Na₂SO₄), filtered and concentrated under vacuum to leave a yellow solid (9.8g) a mixture of *cis:trans* isomers.
¹H NMR δ (d⁶DMSO, 400MHz): 1.078-2.018 (11 H, *cis*/*trans* isomers), 3.074 (1H, m single isomer), 3.193 (3H, s single isomer), 3.213 (3H, s single isomer), 3.606 (1H, s broad, single isomer), >12.5 (1H, s broad, *cisltrans* isomers).

### Description 55. trans-1-Methyl-4-(methyloxy)cyclohexanecarboxylic acid (D55)

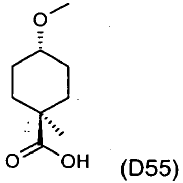

*cis*/*trans*-1-methyl-4-(methyloxy)cyclohexanecarboxylic acid (D54, 41.3 mmol, 7.7 g) was dissolved in thionyl chloride (15.3 eq., 631 mmol, 46 ml). The mixture was refluxed at 90°C for 4 hours. The solvent was evaporated and the crude product treated with toluene and concentrated in vacuo. The residual brown oil was treated with 5% Na₂CO₃ solution (400 ml) and tetrahydrofuran (40 ml) and the mixture was stirred at room temperature for 30 minutes. The aqueous solution was washed with Et₂O (2x) and the aqueous phase was acidified with 2M HCl acid and extracted with ethyl acetate (2x). The combined organics were dried over Na₂SO₄, filtered and the solvent was evaporated to afford the titled compound, 2.8 g, 40%.
¹H NMR δ (d⁶DMSO, 400MHz): 1.085 (3H, s), 1.419 (2H, m), 1.485 (4H, m), 1.701 (2H, m), 3.225 (4H, m), 3.342 (1H, s).

### Description 56. trans-1-Isocyanato-1-methyl-4(methyloxy)cyclohexane (D56)

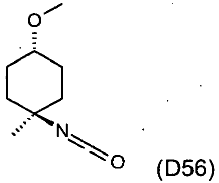

To a solution of *trans*-1-methyl-4-(methyloxy)cyclohexanecarboxylic acid (D55, 15.0 mmol, 2.8 g) in dry toluene (70 ml), Et₃N (1.3 eq., 19.6 mmol, 2.7 ml), and diphenylphosphoryl azide (1.0. eq., 15.0 mmol, 3.2 ml) were added at room temperature. The mixture was refluxed at 80°C for 2 hours. The mixture was cooled room temperature and poured onto 1 M NaOH (70 ml); the aqueous solution was extracted with EtOAc (2x). The organics were combined dried over Na₂SO₄, filtered and the solvent was evaporated to afford the crude compound. The crude product was then purified by chromatography (EtOAc-nhex) on silica column to afford the title compound, 1.33 g, 48%.
¹H NMR δ (d⁶DMSO, 400MHz): 1.327 (3H, s), 1.61 (8H, m), 3.197 (3H, s) 3.355 (1H, m).

### Description 57. trans-1-Methyl-4-(methyloxy)cyclohexanamine monohydrochloride (D57)

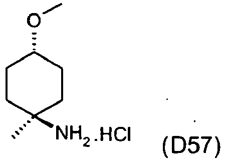

To a solution of *trans*-1-isocyanato-1-methyl-4-(methyloxy)cyclohexane (D56, 7.27 mmol, 1.33 g) in THF (30 ml), concentrated HCl (6 ml) was added at room temperature. The mixture was stirred for 4 hours at room temperature and concentrated HCl (a few drops) was added. The reaction was left overnight and the solvent evaporated to afford the titled compound, 0.9 g, 69%.
¹H NMR δ (d⁶DMSO, 400MHz): 1.260 (3H, s), 1.39 (2H, m), 1.632 (4H, m), 1.87 (2H, m), 3.157 (1H, m), 3.217 (3H, s).

### Description 58. 1-[trans-1-Methyl-4-(methyloxy)cyclohexyl]-4-piperidinone (D58)

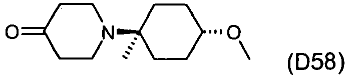

*Trans*-1-methyl-4-(methyloxy)cyclohexanamine monohydrochloride (D57, 7.69 mmol, 1.1 g), 1-ethyl-1-methyl-4-oxopiperidinium iodide (D49, 1.7 eq., 13.1 mmol), 3.5 g, K₂CO₃ (1.5 eq., 11.5 mmol, 1.5 g), water (40 ml) and ethanol (80 ml) were refluxed at 80°C until the starting material was not observed on TLC. The mixture was partitioned between NaHCO₃ and dichloromethane. Some product was lost due to mechanical spillage. The work up was repeated. The crude product was then purified by chromatography (MeOH-NH₃-dichloromethane) on silica column to afford the title compound, 650 mg, 35 %.
¹H NMR δ (d⁶DMSO, 400MHz) 0.85 (3H, s), 1.50 (8H, m)1.762 (2H, t), 2.301 (4H, t), 2.725 (4H, t), 3.207 (3H, s), 3.274 (1H, m).

### Description 59. 1-[trans-1-Methyl-4-(methyloxy)cyclohexyl]-4-piperidinamine (D59)

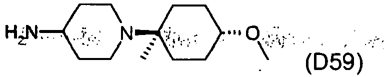

To a solution of 1-(*trans*-1-methyl-4-(methyloxy)cyclohexyl]-4-piperidinone (D58, 2.67 mmol, 650 mg) in 2M NH₃ in methanol (15.0 eq., 40.0 mmol, 20 ml), 10% Pd/C paste (65 mg) was added. The mixture hydrogenated at 50psi for 24 hours at room temperature. The mixture filtered through kieselguhr and washed with ethanol (100 ml). The solvent was evaporated to afford the crude product. The reaction was repeated on the crude product and left for 24 hours. The mixture was filtered through celite and washed with ethanol and the solvent was evaporated to afford the titled compound, 390 mg, 68%, M⁺ + H = 227.

### Description 60. cis/trans-Ethyl 4-{[(1,1-dimethylethyl)(dimethyl)silyl]oxy}cyclohexanecarboxylate (D60)

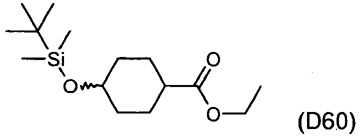

Chloro(1,1-dimethylethyl)dimethylsilane (115g) was added in portions over 1 hour to a solution of ethyl 4-hydroxycyclohexanecarboxylate (118g), imidazole (103g) and dimethylformamide (400ml) stirred under an atmosphere of argon. A small exotherm was observed resulting in the reaction mixture temperature increasing to -40°C. The mixture was stirred at room temperature overnight then poured into 10% citric acid solution (2000ml) and extracted with diethyl ether (2 x 800ml). The ether extracts were washed with water, brine and then dried (Na₂SO₄) and the solvent was removed to give the title compound as a oil (198.4g)
¹H NMR □(CDCl₃, 400 MHz): 0.01 (6H, m), 0.85 (9H, s), 1.2 (3H, m), 1.3-1.5 (2H, m), 1.6 (2H, m), 1.85-2 (3H, m), 2.15-2.3 (1H, m) 3.5 (0.4H, m) 3.86 (1H, m) 4.1 (1H, m).

### Description 61. cis/trans-Ethyl 4-(ethyloxy)cyclohexanecarboxylate (D61)

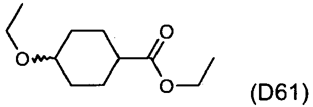

*cis*/*trans*-Ethyl 4-{[(1,1-dimethylethyl)(dimethyl)silyl]oxy}cyclohexanecarboxylate (D60, 35g, 122 mmol) was dissolved in acetonitile (250 ml) and Et₃SiH (1.2 eq., 0.146 mol, 23ml), BiBr₃ (4% mol, 4.9 mmol, 2.2 g) were added at room temperature followed by acetaldehyde (1.2 eq., 8.2 ml) that was slowly added at 25°C. The mixture was stirred at room temperature for 1 hour. The mixture was subsequently poured onto an aqueous saturated solution of. NaHCO₃ and the mixture obtained was then extracted with EtOAc (3x). Organics were combined, dried over Na₂SO₄, filtered and the solvent was evaporated to afford the crude mixture that was purified by silica chromatography (65i column, EtOAc-nhexane) to afford the title compound, 21 g, 87%.
¹H NMR □ (DMSO, 400 MHz): 1.1 (3H, m), 1.15 (3H, m), 1.492-3.212 (assume 10 H, set of broad signals and multiplets), 3.312 (2 H, m), 4.041 (2H, m).

### Description 62. cis/trans-4-(Ethyloxy)cyclohexanecarboxylic acid (D62)

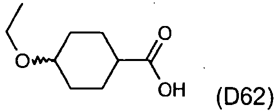

*cis*/*trans*-Ethyl. 4-(ethyloxy)cyclohexanecarboxylate (D61, 21g, -105 mmol) was dissolved in MeOH-tetrahydrofuran (100 ml-100 ml) and NaOH (5 eq., 0.5 mol, 40 ml, 12.5N aqueous solution) was slowly added at room temperature. The mixture was stirred at room temperature overnight; tetrahydrofuran /MeOH was then evaporated and the crude was washed with Et₂O. The aqueous was acidified and extracted with EtOAc (2x); organics were dried over Na₂SO₄, filtered and the solvent was evaporated to afford the title compound as a pale-yellow oil, 17.3 g, 96%.
¹H NMR □ (DMSO, 400 MHz): 1.1 (3H, m), 1.3-3.201 (assume 10 H, set of broad signals and multiplets), 3.417 (2H, m), 12.1 (1H, s broad).

### Description 63. cis/trans-4-(Ethyloxy)-1-methylcyclohexanecarboxylic acid (D63)

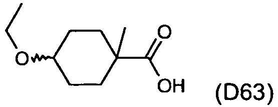

A stirred solution of diisopropylamine (2.3 eq., 0.231 mol, 33 ml) in tetrahydrofuran (300ml) at -20°C under argon was treated over 10 min with 2.5M n-butyllithium in hexane (2.3 eq., 93 ml), then allowed to warm to 0°C and stir for 15 mins. The mixture was recooled to - 10°C and treated over 10 min with a solution of *cis*/*trans*-4-(ethyloxy)cyclohexanecarboxylic acid (D62, 17.3g, 0.1 mol) in 50 ml of tetrahydrofuran. The resulting yellow solution was heated at 50°C for 2.5hrs, then cooled to 0°C and treated with iodomethane (3 eq., 0.3 mol, 19 ml). The mixture was allowed to warm to room temperature and stir for 20 hrs when a yellow precipitate had formed. The mixture was cooled to 10°C, treated with 10% citric acid solution (200ml), and then concentrated under vacuum. The residual mixture was diluted with water (200ml) and extracted with Et₂O (2x). The combined extracts were dried (Na₂SO₄) and concentrated under vacuum to leave the title compound as a yellow oil, 16.2 g, 87%, a mixture of *cis:trans* isomers.
¹H NMR □(DMSO, 400 MHz): 1.086 (assume 8H, m), 1.510 (3H, m), 1.698 (1H, m), 1.781 (1H, m), 2.005(1H, m), 3.191 (1/2H, m), 3.392 (2H, m), 3.606 (1/2H, m), 12.2 (1H, s br)

### Description 64. trans-4-(Ethyloxy)-1-methylcyclohexanecarboxylic acid (D64)

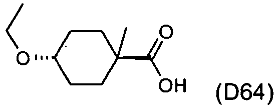

*cis*/*trans*-4-(Ethyloxy)-1-methylcyclohexanecarboxylic acid (D63, 16.2 g, 87.1 mmol) was dissolved in thionyl chloride (15 eq., 1.31 mol, 95 ml) at room temperature and it was subsequently heated to 85°C for 4 hrs. The mixture was then allowed to cool to room temperature and the thionyl chloride was azeotropically evaporated with toluene. The residue was dissolved in THF (100ml), treated with a 5% aqueous solution of Na₂CO₃ (500 ml) and stirred well at room temperature for 20 minutes. The aqueous residue washed with Et₂O (2), then acidified and extracted with EtOAc (3x). The combined extracts were dried (Na₂SO₄), filtered and concentrated under vacuum to leave the title compound, 6.5 g, 40%
¹H NMR □(DMSO, 400 MHz): 1.076 (6H, m), 1.505 (6H, m), 1.693 (2H, m), 3.331 (1H, m), 3.394 (2H, q), 12.1 (1H, s br).

### Description 65. trans-4-(Ethyloxy)-1-isocyanato-1-methylcyclohexane (D65)

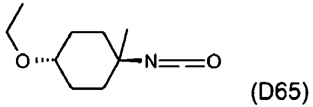

A stirred solution of *trans*-4-(ethyloxy)-methylcyclohexanecarboxylic acid (D64, 5.5g, 29.5mmol) in toluene (120ml) at room temperature under argon was treated with triethylamine (1.3 eq., 37.7 mmol, 5.3 ml) and diphenylphosphoryl azide (1eq., 29.5 mmol, 6.4 ml) and heated at 85°C for 1 hr. The mixture was allowed to cool to room temperature, then treated with 1 M NaOH solution (300ml) and extracted with Et₂O (3x). The combined extract was dried (Na₂SO₄), filtered and concentrated under vacuum to leave the title compound, 5 g, 94 %.
¹H NMR □(DMSO, 400 MHz): 1.092 (3H, t), 1.330 (3H, s), 1.487-1.691 (8H, m), 3.392 (2H, q), 3.477 (1H, m).

### Description 66. [trans-4-(Ethyloxy)-1-methylcyclohexyl]amine mono hydrochloride (D66)

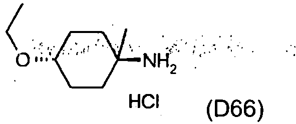

A solution of *trans*-4-(ethyloxy)-1-isocyanato-1-methylcyclohexane (D65, 5.0g, 27.3 mmol) in THF (100ml) was treated with 5M aqueous HCl acid (5.5 eq., 150 mmol, 30 ml) and stirred at room temperature under argon overnight, then concentrated under vacuum. The residual semi-solid was triturated with Et₂O to give a first batch of title compound as a white solid, 2.8 g. The mother liquors were evaporated, dissolved in THF again (50 ml) and treated with 5M aqueous HCl acid (15 ml) and the mixture stirred for over the weekend at room temperature. The solvent was then evaporated and the solid obtained was dried in the oven at 50°C before trituration with Et₂O. This final trituration afforded further 646 mg of title compound. The two batches were combined to afford 3.4 g, 66%.
¹H NMR □(DMSO, 400 MHz): 1.084 (3H, t), 1.256 (3H, s), 1.378 (2H, m), 1.619 (4H, m), 1.847 (2H, m), 3.243 (1H, m), 3.424 (2H, q), 8.090 (3H, br s).

### Description 67. 1-[trans-4-(Ethyloxy)-1-methylcyclohexyl]-4-piperidinone (D67)

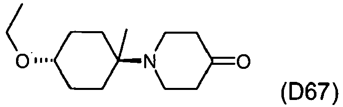

A stirred solution of [*trans*-4-(ethyloxy)-1-methylcyclohexyl]amine mono hydrochloride (D66, 3.4 g, 17.8 mmol) in a mixture of ethanol (216 ml) and water (108 ml) at room temperature under argon was treated with potassium carbonate (1.1 eq., 19.6 mmol, 2.7g) followed by 1-ethyl-1-methyl-4-oxopiperidinium iodide (D49, 1.5 eq., 26.7 mmol, 7.1g), then heated at 80°C for 2 hours. The mixture was allowed to cool to room temperature then the aqueous residue was treated with sat. NaHCO₃ solution and extracted with dichloromethane (3x). The combined extracts were dried (Na₂SO₄) and concentrated under vacuum to leave the crude compound which was chromatographed on silica gel (65i column) eluting with 0-10% MeOH/DCM to afford the title compound, 2.3 g 54%.
¹H NMR □(DMSO, 400 MHz): 0.855 (3H, s), 1.099 (3H, t), 1.421 (2H, m), 1.544 (4H, m), 1.793 (2H, m), 2.297 (4H, t), 2.726 (4H, t), 3.377-3.430 (3H, t + m).

### Description 68. 1-[trans-4-(Ethyloxy)-1-methylcyclohexyl]-4-piperidinamine dihydrochloride (D68)

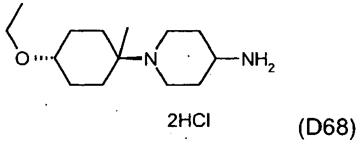

A solution of 1-[*trans*-4-(ethyloxy)-1-methylcyclohexyl]-4-piperidinone (D67, 2.03g 9.62 mmol) in 2M NH₃/MeOH (200ml) was treated with 10% Pd/C (510 mg) and shaken under hydrogen atmosphere at 50psi initial pressure overnight at room temperature. The mixture was filtered through Kieselguhr to remove catalyst and the filtrate concentrated under vacuum to leave the free base of the title compound. This was dissolved in MeOH (20 ml) and treated with 10 ml of HCl (1M solution of in Et₂O) for 10 min. Solvent was subsequently evaporated to afford the title compound as a white solid 2.8 g, complete conversion.
¹H NMR □(DMSO, 250 MHz, at 352.2K): 1.091 (6H, m br), 1.336 (2H, m), 1.695-1.865 (7H, m), 2.067 (2H, d br), 2.531 (1H, br), 3.187-3.317 (assume 6H, set of broad signals and multiplets), 3.434 (2H, q), 8.496 (2H, s br).

### Description 69. cis/trans-Ethyl 4-(propyloxy)cyclohexanecarboxylate (D69)

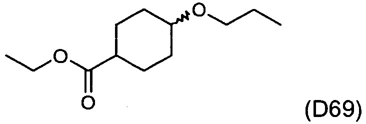

Propionaldehyde (6.4g) in acetonitrile (50ml) was added over 30 minutes to a solution of *cis*/*trans*-ethyl 4-{[(1,1-dimethylethyl)(dimethyl)silyl]oxy}cyclohexanecarboxylate (D60, 25.2g), bismuth tribromide (4.4g), triethylsilane (17.5ml) in acetonitrile (300ml) and the mixture was stirred for a further 1.5 hours. The solvent was partially removed then the residue was treated with ethyl acetate and saturated sodium bicarbonate solution. The organic layer was separated, washed with brine, dried with anhydrous sodium sulphate and the solvent was removed to give the title compound contaminated with silicone residues (39.1g).

### Description 70. cis/trans-4-(Propyloxy)cyclohexanecarboxylic acid (D70)

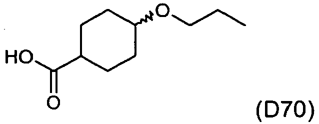

The product from Description 69 (39.1g) containing *cis*/*trans*-ethyl 4-(propyloxy)cyclohexanecarboxylate, 40% w/w sodium hydroxide solution (150ml) tetrahydrofuran (200ml) and methanol (150ml) was stirred for approx. 72 hours. The solvent was partially removed then the resulting mixture was treated with ethyl acetate and water. The aqueous layer was separated, acidified with concentrated hydrochloric acid and extracted with diethyl ether. The ether layer was washed with brine, dried with anhydrous sodium sulphate and the solvent was removed to give the title compound as a oil(15.42g).

### Description 71. cis/trans-1-Methyl-4-(propyloxy)cyclohexanecarboxylic acid (D71)

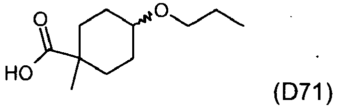

A stirred solution of diisopropylamine (24ml, 0.17mole) in tetrahydrofuran (300ml) at -20°C under argon was treated over 10 mins with 2.5M n-butyllithium in hexane (68ml, 0.17mole), then allowed to warm to 0°C and stir for 15 mins. The mixture was recooled to -10°C and treated over 10 mins with a solution of 4-(propyloxy)cyclohexanecarboxylic acid (D70, 13.8g, 0.074mole) in tetrahydrofuran. The resulting yellow solution was heated at 50°C for 2.5hrs, then cooled to 0°C and treated with iodomethane (13.8ml, 0.22mole). The mixture was allowed to warm to room temperature and stir for 20hrs when a yellow precipitate had formed. The mixture was cooled to 10°C, treated with 10% citric acid solution (200ml), then concentrated under vacuum to approx. 250ml volume. The residual mixture was diluted with water (200ml) and extracted with Et₂O (3x250ml). The combined extract was dried (Na₂SO₄) and concentrated under vacuum to leave a yellow oil (15.0g) which was approx. 60:40 mixture of cis:trans isomers.

### Description 72. trans-1-Methl-4-(propyloxy)cyclohexanecarboxylic acid (D72)

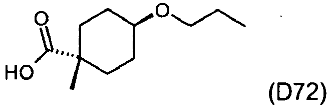

*cis*/*trans*-1-Methyl-4-(propyloxy)cyclohexanecarboxylic acid (D71, 13g, 0.070mole) was added to stirred thionyl chloride (50ml), 0.68mole) at 10°C and then allowed to warm to room temperature, followed by heating at 85°C for 3 hrs. The mixture was concentrated under vacuum and the residue concentrated twice with toluene to remove excess thionyl chloride. The residue was dissolved in THF (100ml), treated with dil. NaHCO₃ solution (250ml) and stirred well at room temperature for 24 hrs, followed by standing at room temperature for 3 days. The mixture from the same stage of a smaller scale reaction on 2g of *cis*/*trans*-1-methyl-4-(propyloxy)cyclohexanecarboxylic acid was combined at this time. The combined mixture was concentrated under vacuum to approx. 300ml and the aqueous residue washed with Et₂O (2x120ml), then acidified with 2M HCl acid and extracted with EtOAc (2x150ml). The combined extract was dried (Na₂SO₄) and concentrated under vacuum to leave the title compound as a pale yellow solid (5.1 g, 34%).
¹H NMR □(CDCl₃, 400 MHz): 0.92 (3H, t), 1.24 (3H, s), 1.54-1.74 (8H, m), 1.78-1.88 (2H, m); 3.33-3.40 (3H, m). 1H not discernible from spectrum.

### Description 73. trans-1-Isocyanato-1-methyl-4-(propyloxy)cyclohexane (D73)

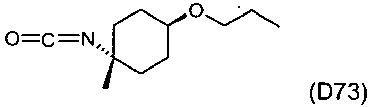

A stirred solution of *trans*-1-methyl-4-(propyloxy)cyclohexanecarboxylic acid (D72, 5.1g, 0.027mole) in toluene (120ml)) at room temperature under argon was treated with triethylamine (4.9ml, 0.035mole) and diphenylphosphoryl azide (5.8ml, 0.027mole) and heated at 85°C for 1 hr. The mixture was allowed to cool to room temperature, then treated with 1M NaOH solution (200ml) and extracted with Et₂O (2x150ml). The combined extract was dried (Na₂SO₄) and concentrated under vacuum to leave the title compound as a yellow oil (5.0g, 100%).
¹H NMR □(CDCl₃, 400 MHz): 0.91 (3H, t), 1.36 (3H, s), 1.50-1.60 (4H, m), 1.65-1.80 (6H, m), 3.33 (2H, t), 3.46-3.52 (1H, m).

### Description 74. [trans-1-Methyl-4-(propyloxy)cyclohexyl]amine hydrochloride (D74)

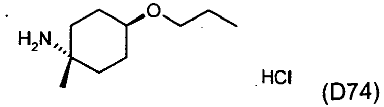

A solution of *trans*-1-isocyanato-1-methyl-4-(propyloxy)cyclohexane (D73, 5.0g, 0.027mole) in THF (100ml) was treated with 5M HCl acid (25ml) and stirred at room temperature under argon for 20hrs, then concentrated under vacuum and the residue azeotroped with toluene to remove traces of water. The residual semi-solid was triturated with Et₂O (120ml) to give a solid, which was filtered off, washed with Et₂O and dried at 50°C under vacuum to afford the title compound as a white solid (4.5g, 85%).
¹H NMR □(CDCl₃,400 MHz): 0.91 (3H, t), 1.47 (3H, s), 1-45-1.70 (4H, m), 1.75-2.00 (6H, m), 3.52 (2H, t), 3.40-3.48 (1H, m), 8.38 (3H, br s).

### Description 75. 1-[trans-1-Methyl-4-(propyloxy)cyclohexyl]-4-piperidinone (D75)

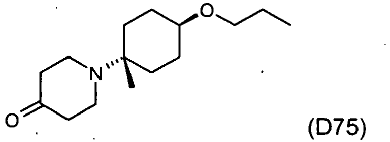

A stirred solution of [*trans*-1-methyl-4-(propyloxy)cyclohexyl]amine hydrochloride (D74, 4.5g, 0.022mole) in a mixture of ethanol (100ml) and water (60ml) at room temperature under argon was treated with potassium carbonate (3.31g 0.024mole) followed by 1-ethyl-1-methyl-4-oxopiperidinium iodide (D49, 8.91g; 0.033mole), then heated at 80°C for 2.5hrs. The mixture was allowed to cool, concentrated under vacuum to approx. 60ml, then the aqueous residue was treated with sat. NaHCO₃ solution and extracted with dichloromethane (3x80ml). The combined extract was dried (Na₂SO₄) and concentrated under vacuum to leave an orange oil (6.1g), which was chromatographed on silica gel eluting with 0-10% MeOH/ dichloromethane to afford the title compound as a yellow oil (3.45g, 63%).
¹H NMR □(CDCl₃, 400 MHz): 0.93 (3H, s + 3H, t), 1.48-1.72 (8H, m), 1.80-1.92 (2H, m), 2.41 (4H, t), 2.82 (4H, t), 3.35-3.45 (3H, t + m).

### Description 76. 1-[trans-1-Methyl-4-(propyloxy)cyclohexyl]-4-piperidinamine (D76)

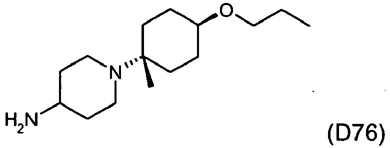

A solution of 1-[*trans*-1-methyl-4-(propyloxy)cyclohexyl]-4-piperidinone (D75, 2.0g, 0.0079mole) in 2M NH₃/MeOH (60ml) was treated with 10% Pd/C (150mg) and shaken under hydrogen atmosphere at 55psi initial pressure for 3.5 days at room temperature. The mixture was filtered through Kieselguhr to remove catalyst and the filtrate concentrated under vacuum to leave the title compound as a white solid (1.82g, 91%).
¹H NMR □(CDCl₃, 400 MHz): 0.91 (3H, t), 1.08 (3H, br s), 1.32-1.45 (2H, m), 1.5-1-60 (2H, m), 1.62-2.60 (assume 12H, set of broad signals), 2.70-3:10 (1H, br), 34.10-3.25 (2H, br), 3.30-3.45 (3H, m), 3.40-4.40 (2H, v br).

### Description 77. 5,6-Dimethyl-1-[1-(phenylmethyl)-1,2,3,6-tetrahydro-4-pyridinyl]-1,3-dihydro-2H-benzimidazol-2-one (D77)

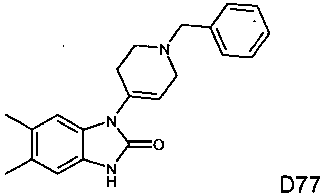

4,5-Dimethyl-1,2-benzenediamine (409mg, 3 mmole) and ethyl 4-oxo-1-(phenylmethyl)-3-piperidinecarboxylate (784mg, 3 mmole) were dissolved in xylene (20 ml) and heated under argon under reflux for 8h, then allowed to stand at room temperature overnight. The mixture was concentrated under reduced pressure, and the residue was recrystallised from ethylacetate containing a trace of methanol to give the title compound as white crystals (463mg, 46%). M⁺H = 334.3.

### Description 78. 5,6-Dimethyl-1-(4-piperidinyl)-1,3-dihydro-2H-benzimidazol-2-one (D78)

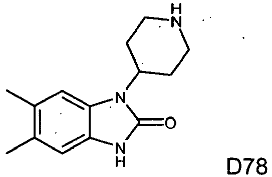

A solution of 5,6-dimethyl-1-[1-(phenylmethyl)-1,2,3,6-tetrahydro-4-pyridinyl]-1,3-dihydro-2H-benzimidazol-2-one (D77) (333mg, 1.0mmole) in ethanol (10 ml) and acetic acid (2 ml), was treated with Pd/charcoal (90 mg) and shaken under 50PSI of hydrogen pressure for 45hr. The catalyst was filtered off and the filtrate was concentrated under reduced pressure to leave a thick oil, which crystallised using a small amount of methanol. As material was a 1:1 mixture containing title compound and 5,6-dimethyl-1-(1,2,3,6-tetrahydro-4-pyridinyl)-1,3-dihydro-2*H*-benzimidazol-2-one, the solid was dissolved in ethanol and acetic acid, treated with 10% Pd/C catalyst and shaken under 50PSI hydrogen pressure over a weekend. After work-up the material still contained some 5,6-dimethyl-1-(1,2,3,6-tetrahydro-4-pyridinyl)-1,3-dihydro-2*H*-benzimidazol-2-one. This was used without purification.

### Description 79. 1-Bromo-4-fluoro-2-methyl-5-nitrobenzene (D79)

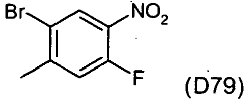

A soltution of 2-bromo-5-fluorotoluene (3.8g) in concentrated sulfuric acid (40ml) was treated portionwise with potassium nitrate (2.0g) at 0-20°C (internal). After 2h more at the same temperature the mixture was poured onto ice and extracted with hexane. Drying and evaporation gave the title compound, 4.0g.

### Description 80. trans-N-(4-Bromo-5-methyl-2-nitrophenyl)-1-(4-ethoxycyclohexyl)-4-piperidinamine (D80)

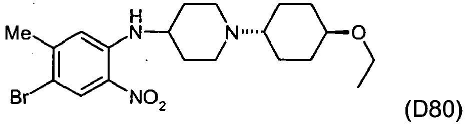

A stirred solution of 1-bromo-4-fluoro-2-methyl-5-nitrobenzene (D79, 0.23g) in dimethylformamide (5ml) at room temperature under argon was treated with diisopropylethylamine (0.7ml) and *trans*-1-[4-(ethoxy)cyclohexyl]-4-piperidinamine dihydrochloride (D20, 0.3g) and heated at 80°C for 18h. The cooled reaction was diluted with ethyl acetate and washed three times with water then dried, evaporated and chromatographed (Biotage KP-NH^{™}-silica column eluting with 0-15% ethyl acetate/hexane) to give the title compound (0.15g).

### Description 81. trans-4-Bromo-5-methyl-N-[1-(4-ethoxycyclohexyl)-4-piperidinyl]-1,2-benzenediamine (D81)

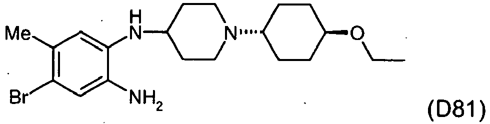

A stirred suspension of *trans*-*N*-(4-bromo-5-methyl-2-nitrophenyl)-l-(4-ethoxycyclohexyl)-4-piperidinamine (D80,150mg) in ethanol (10ml) at 50°C was treated with Raney nickel (2.0ml of 10% aqueous suspension) followed by dropwise addition of hydrazine hydrate (0.17ml). The mixture was heated at the same temperature for 1h, then filtered through Kieselguhr and the filtrate evaporated and re-evaporated from toluene to afford the title compound, 130mg.

### Description 82. N-(4-Chloro-5-methyl-2-nitrophenyl)-1-[trans-4-(ethyloxy)cyclohexyl]-4-piperidinamine (D82)

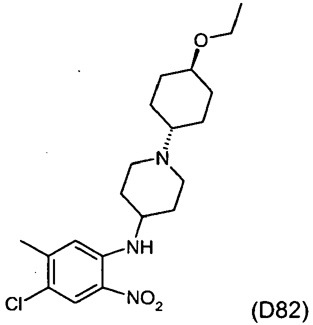

A stirred mixture of 1-chloro-4-fluoro-2-methyl-5-nitrobenzene (D1, 310mg, 1.6mmole) in dimethylformamide(7ml) at room temperature under argon was treated with diisopropylethylamine (0.71 ml, 4.0mmole) and 1-(*trans*-4-ethoxycyclohexyl)piperidin-4-amine dihydrochloride (D20, 400mg, 1.34mmole) and heated at 85°C for 3hrs. The mixture was concentrated under vacuum and the residue treated with 10% Na₂CO₃ solution and extracted with dichloromethane. The extract was dried, concentrated under vacuum and the residue chromatographed on silica gel eluting with 0-10% methanol/dichloromethane to afford the title compound as an orange solid (425mg, 80%). Recrystallisation from methanol (∼15ml) afforded a crystalline orange solid (320mg). MH⁺ 396.
¹H NMR □(CDCl₃, 400 MHz): 1.17-1.38 (4H, m), 1.20 (3H, t), 1.59-1.71 (2H, m), 1.90-1.97 (2H, m), 2.03-2.17 (4H, m), 2.30-2.50 (3H, m), 2.36 (3H, s), 2.83-2.93 (2H, m), 3.13-3.23 (1H, m), 3.48-3.59 (3H, m), 6.70 (1H, s), 8.08 (1H, d), 8.16 (1H, s).

### Description 83. 4-Chloro-N-{1-[trans-4-(ethyloxy)cyclohexyl]-4-piperidinyl}-5-methyl-1,2-benzenediamine (D83)

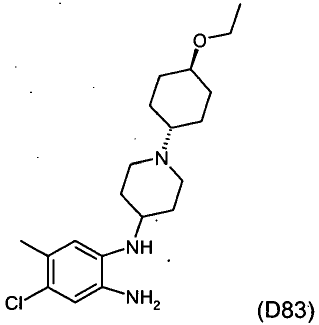

A stirred solution of *N*-(4-chloro-5-methyl-2-nitrophenyl)-1-[trans-4-(ethyloxy)cyclohexyl]-4-piperidinamine (D82, 0.32g, 0.81 mmole) in ethanol (20ml) at room temperature under argon was treated with Raney Nickel (∼20mg) followed by a dropwise addition of hydrazine hydrate (0.25ml, 8.4mmole) and the mixture stirred at room temperature for 3. hrs. The Raney Nickel was removed by filtration through Kieselguhr and the filtrate concentrated under vacuum to afford the title compound as a pale green solid (0.295g, 100%yield). MH⁺=366.
¹H NMR δ (CDCl₃, 400MHz): 1.15-1.88 (4H, m), 1.19 (3H, t), 1.40-1.53 (2H, m), 1.90-1.98 (2H, m), 2.00-2.15 (4H, m), 2.25 (3H, s), 2.25-2.40 (3H, m), 2.89 (2H, br d), 3.05-3.35 (5H, m), 3.51 (2H, q), 6.47 (1H, s), 6.70 (1H, s).

### Description 84. 1-Chloro-5-fluoro-2-methyl-4-nitrobenzene (D84)

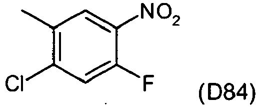

A solution of 2-chloro-4-fluorotoluene (4.0g) in concentrated sulfuric acid (30ml) was treated portionwise with potassium nitrate (2.8g) at <20°C (internal). After 1h more stirring with ice cooling the mixture was poured onto ice and extracted with diethyl ether. Drying and evaporation gave the title compound, 4.5g.

### Description 85. trans-N-(5-Chloro-4-methyl-2-nitrophenyl)-1-(4-ethoxycyclohexyl)-4-piperidinamine (D85)

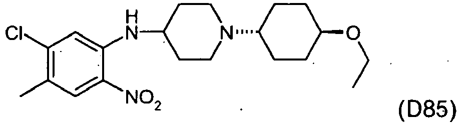

A stirred solution of 1-chloro-5-fluoro-2-methyl-4-nitrobenzene (D84, 0.20g) in dimethylformamide (5ml) at room temperature under argon was treated with diisopropylethylamine (0.7ml) and *trans*-1-[4-(ethoxy)cyclohexyl]-4-piperidinamine dihydrochloride (D20, 0.3g) and heated at 50°C for 18h. The cooled reaction was diluted with ethyl acetate and washed three times with water then dried, evaporated and the residue crystallised from diethyl ether to give the title compound (0.10g).

### Description 86. trans-5-Chloro-4-methyl-N-[1-(4-ethoxycyclohexyl)-4-piperidinyl]-1,2-benzenediamine (D86)

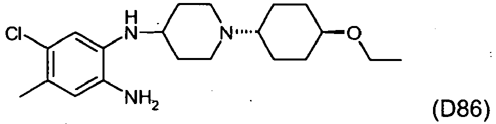

A stirred suspension of *trans*-*N*-(5-chloro-4-methyl-2-nitrophenyl)-l-(4-ethoxycycl ohexyl)-4-piperidinamine (D85,100mg) in ethanol (10ml) at 50°C was treated with Raney nickel (1.0ml of 10% aqueous suspension) followed by dropwise addition of hydrazine hydrate (0.5ml). The mixture was heated at the same temperature for 1h, then filtered through Kieselguhr and the filtrate evaporated and re-evaporated from toluene then diethyl ether to afford the title compound, 95mg.

### Description 87. N-[5-Chloro-2-nitro-4-(trifluoromethyl)phenyl]-1-[trans-4-(ethyloxy)cyclohexyl]-4-piperidinamine (D87)

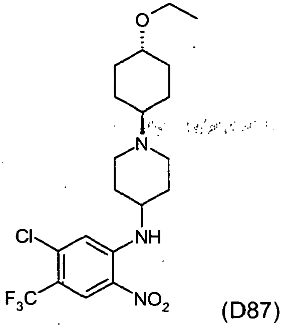

To a solution of 1,5-dichloro-2-nitro-4-(trifluoromethyl)benzene (1.0 eq., 460 mg) in dry dimethylformamide (4ml), diisopropylethylamine (2eq., 3.54 mmol, 0.604ml) and 1-[*trans-*4-(ethyloxy)cyclohexyl]-4-piperidinamine dihydrochloride (D20, 1eq., 1.77 mmol, 400 mgs) were added at room temperature and the mixture was stirred at 45°C for 24 hours. The crude mixture was then cooled to room temperature and the crude was poured onto water/brine; the aqueous solution was extracted with EtOAc (3x); organics were alternatively washed with brine and water (1x). The organics were combined dried over Na₂SO₄, filtered and the solvent was evaporated to afford the crude product. The crude product was passed through an SCX cartridge and then purified by chromatography (EtOAc-nhex) on silica column followed by another column chromatography (20%EtOAc - 80% nhex) on Biotage KP-NH^{™}-silica column to yield the title compound, 184 mgs, 23%. M⁺ + H = 450.

### Description 88. - [2-Amino-5-chloro-4-(trifluoromethyl)phenyl]{1-[trans-4-(ethyloxy)cyclohexyl]-4-piperidinyl}amine (D88)

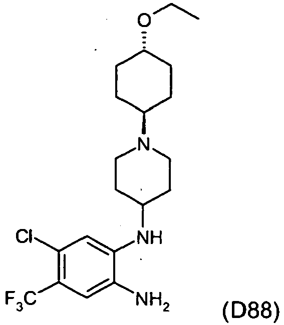

N-[5-Chloro-2-nitro-4-(trifluoromethyl)phenyl]-1-[*trans*-4-(ethyloxy)cyclohexyl]-4-piperidinamine (D87, 0.409 mmol, 184 mgs) was dissolved in EtOH (15 ml) and an aqueous suspension of Raney Nickel (0.6ml of a 50% suspension) was added at room temperature. The mixture was stirred at 40°C before adding hydrazine monohydrate (15eq., 6.132 mmol, 0.30 ml) over 20 minutes. The reaction mixture was filtered through a celite and the solvent was evaporated to yield the title compound, 128 mgs, 75%. M^{+*} + H = 420.

### Description 89. trans-N-(4-Bromo-5-methyl-2-nitrophenyl)-1-(4-propoxycyclohexyl)-4-piperidinamine (D89)

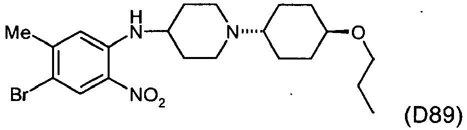

A stirred solution of 1-bromo-4-fluoro-2-methyl-5-nitrobenzene (D79, 0.23g) in dimethylformamide (5ml) at room temperature under argon was treated with diisopropylethylamine (0.7ml) and *trans*-1-[4-(propoxy)cyclohexyl]-4-piperidinamine dihydrochloride (D27, 0:31g) and heated at 80°C for 18h. The cooled reaction was diluted with ethyl acetate and washed three times with water then dried, evaporated and chromatographed (Biotage KP-NH^{™}-silica column eluting with 0-15% ethyl acetate/hexane) to give the title compound (0.14g).

### Description 90. trans-4-Bromo-5-methyl-N-[1-(4-propoxycyclohexyl)-4-piperidinyl]-1,2-benzenediamine (D90)

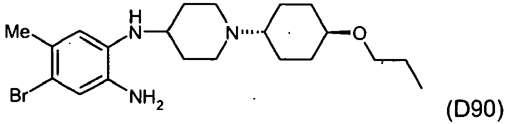

A stirred suspension of *trans*-*N*-(4-bromo-5-methyl-2-nitrophenyl)-1-(4-propoxycyclohexyl)-4-piperidinamine (D89,140mg) in ethanol (10ml) at 50°C was treated with Raney nickel (1.9ml of 10% aqueous suspension) followed by dropwise addition of hydrazine hydrate (0.16ml). The mixture was heated at the same temperature for 1h, then filtered through Kieselguhr and the filtrate evaporated and re-evaporated from toluene to afford the title compound, 140mg.

### Description 91. N-[5-Chloro-2-nitro-4-(trifluoromethyl)phenyl]-1-[trans-4-(propyloxy)cyclohexyl]-4-piperidinamine (D91)

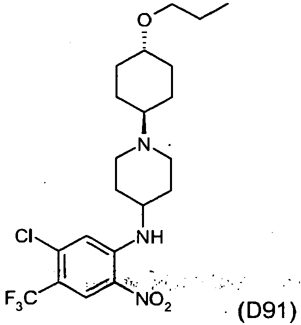

To a solution of 1,5-dichloro-2-nitro-4-(trifluoromethyl)benzene (1.0 eq., 430 mg) in dry dimethylformamide (4ml), diisopropylethylamine (2eq., 3.34 mmol, 0.60 ml) and 1-[*trans-*4-(propyloxy)cyclohexyl]-4-piperidinamine dihydrochloride (D27, 1eq., 1.67 mmol, 400 mgs) were added at room temperature and the mixture was stirred at 45°C for 24 hours. The crude mixture was then cooled to room temperature and the crude was poured onto water/brine; the aqueous solution was extracted with EtOAc (3x); organics were alternatively washed with brine and water. The organics were combined dried over Na₂SO₄, filtered and the solvent was evaporated to afford the crude product. The crude product was passed through an SCX cartridge and then purified by chromatography (MeOH-NH₃-dichloromethane) on silica column followed by another column chromatography (20%EtOAc - 80% nhex) on Biotage KP-NH^{™}-silica column to yield the title compound;174 mgs, 23%. M⁺ + H = 464.

### Description 92. 2-Amino-5-chloro-4-(trifluoromethyl)phenyl]{1-[trans-4-(propyloxy)cyclohexyl]-4-piperidinyl}amine (D92)

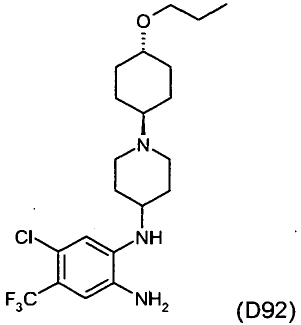

*N*-[5-Chloro-2-nitro-4-(trifluoromethyl)phenyl]-1-[*trans*-4-(propyloxy)cyclohexyl]-4-piperidinamine (D91, 0.375 mmol, 174 mgs) was dissolved in EtOH (15 ml) and an aqueous suspension of Raney Nickel (0.7ml of a 50% suspension) was added at room temperature. The mixture was stirred at 40°C before adding hydrazine monohydrate (15eq., 5.625 mmol, 0.175 ml) over 20 minutes. The reaction was left overnight. The reaction mixture was filtered through a celite and the solvent was evaporated to yield the title compound; 158 mgs, 98%. M⁺ + H = 434.

### Description 93. N-(4-Fluoro-5-methyl-2-nitrophenyl)-1-{trans-4-[(1-methylethyl)oxy]cyclohexyl}-4-piperidinamine (D93)

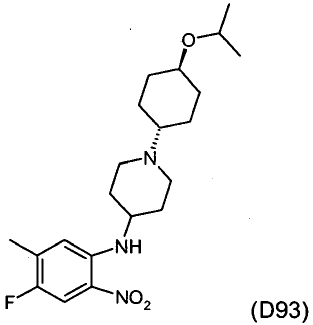

A stirred suspension of 1-{*trans*-4-[(1-methylethyl)oxy]cyclohexyl}-4-piperidinamine dihydrochloride (D44, 250mg, 0.80mmole) in dimethylformamide (4ml) at room temperature under argon was treated with diisopropylethylamine (0.62ml, 3.5mmole) followed by 2,5-difluoro-4-nitrotoluene (173mg, 1.0mmole) and heated at 80°C for 20hrs. The mixture was concentrated under vacuum and the residue treated with 10% Na₂CO₃ solution (25ml) and extracted with EtOAc (25ml). The extract was dried (Na₂SO₄), concentrated under vacuum and the residue chromatographed on silica gel (20g) eluting with 0-10% methanol/dichloromethane to afford the title compound as an orange solid (220mg, 70%). MH⁺ 394.
¹H NMR □(CDCl₃, 400 MHz): 1.14 (6H, d), 1.20-1.38 (4H, m), 1.60-1.72 (2H, m), 1.89-1.96 (2H, m), 2.00-2.12 (4H, m), 2.29 (3H, s), 2.30-2.50 (3H, m), 2.83-2.93 (2H, m), 3.20-3.30 (1H, m), 3.47-3.59 (1H, m), 3.66-3.76 (1H, m), 6.63 (1H, d), 7.82 (1H, d), 8.06 (1H, d).

### Description 94. 4-Fluoro-5-methyl-N-(1-{trans-4-[(1-methylethyl)oxy]cyclohexyl}-4-piperidinyl)-1,2-benzenediamine (D94)

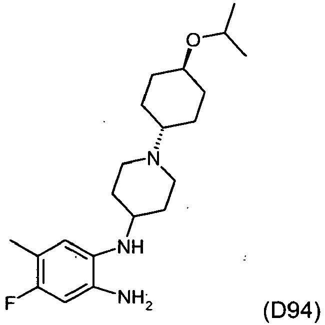

A stirred solution of *N*-(4-fluoro-5-methyl-2-nitrophenyl)-1-{*trans*-4-[(1-methylethyl)oxy]cyclohexyl}-4-piperidinamine (D93, 220mg, 0.56mmole) in ethanol (15ml) at room temperature under argon was treated with Raney Nickel (-20mg) followed by hydrazine hydrate (0.18ml, 6.0mmole) and maintained for 3 hrs. The mixture was filtered through Kieselguhr and the filtrate concentrated under vacuum to afford the title compound as a pale yellow solid (205mg, 100%yield).
¹H NMR δ (CDCl₃, 400MHz): 1.13 (6H, d), 1.20-1.38 (4H, m), 1.43-1.60 (2H, m), 1.90-1.96 (2H, m), 2.04 (4H, br d), 2.15 (3H, s), 2.30-2.42 (3H, m), 2.50-3.00 (1H, v br), 3.47 (2H, br d), 3.05-3.17 (1H, m), 3.19-3.30 (1H, m), 3.47 (2H, br s), 3.65-3.75 (1H, m), 6.40-6.48 (2H, m).

### Description 95. N-(4-Fluoro-5-methyl-2-nitrophenyl)-1-[trans-1-methyl-4-(methyloxy)cyclohexyl]-4-piperidinamine (D95)

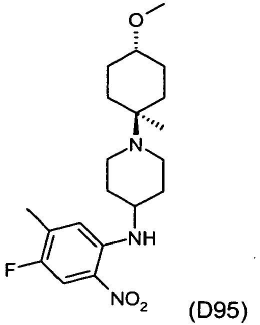

To a solution of 1,4-difluoro-2-methyl-5-nitrobenzene (2.6 eq., 2.19mmol, 380 mg) in dry dimethylformamide (12 ml), diisopropylethylamine (2eq., 1.68 mmol, 0.29 ml) and 1-[*trans*-1-methyl-4-(methyloxy)cyclohexyl]-4-piperidinamine (D59, 1eq., 0.841 mmol, 190 mgs) were added at room temperature and the mixture was refluxed at 80°C for 24 hours. The crude mixture was then cooled to room temperature and the crude was poured onto water (50 ml) and basified with 2M NaOH to pH 10. The aqueous solution was extracted with EtOAc (3x) and the organics were combined, dried over Na₂SO₄, filtered and the solvent was evaporated, to afford the crude product. The crude product was passed through an SCX cartridge and then purified by chromatography (MeOH-NH₃-dichloromethane) on silica column afford the title compound, 125 mgs, 40%, M⁺ + H = 380.

### Description 96. (2-Amino-4-fluoro-5-methylphenyl){1-[trans-1-methyl-4-(methyloxy)cyclohexyl]-4-piperidinyl}amine (D96)

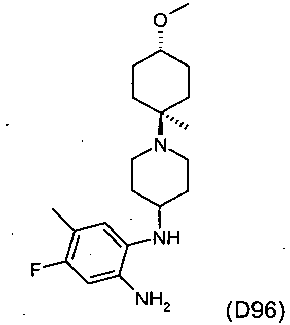

*N-*(4-Fluoro-5-methyl-2-nitrophenyl)-1-[*trans*-1-methyl-4-(methyloxy)cyclohexyl]-4-piperidinamine (D95, 0.329 mmol, 125 mg) was dissolved in EtOH (3 ml) and an aqueous suspension of Raney Nickel (1 ml of a 50% suspension) was added at room temperature. The mixture was stirred for 30 minutes at 50°C before adding hydrazine monohydrate (15eq., 4.93 mmol, 0.15 ml). The reaction was left for 1 hour and then the mixture was filtered through a celite and the solvent was evaporated to afford the crude compound. The crude product was purified by chromatography (MeOH-NH₃-dichloromethane) on silica column to afford the title compound, 66 mgs, 60%, M⁺ + H = 350.

### Description 97. 1-[trans-4-(Ethyloxy)-1-methylcyclohexyl]-N-(4-fluoro-5-methyl-2-nitrophenyl)-4-piperidinamine (D97)

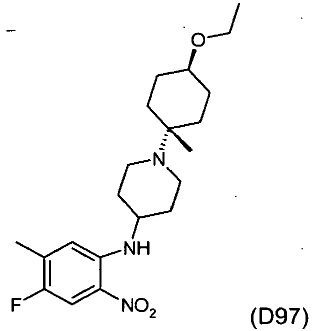

A stirred suspensions of 1-{*trans*-4-(ethyloxy)-1-methylcyclohexyl]-4-piperidinamine (D68 free base, 160mg, 0.67mmole) in dimethylformamide (3ml) at room temperature under argon was treated with diisopropylethylamine (0.18ml), 1.0mmole) followed by 2,5-difluoro-4-nitrotoluene (147mg, 0.85mmole) and heated at 80°C for 16hrs. The mixture was concentrated under vacuum and the residue treated with 10% Na₂CO₃ solution and extracted with dichloromethane. The extract was dried (Na₂SO₄), concentrated under vacuum and the residue chromatographed on silica gel (10g) eluting with 0-10% methanol/dichloromethane. The title compound crystallised from MeOH as an orange solid (107mg, 41%). MH⁺ 394.
¹H NMR □(CDCl₃, 400 MHz): 0.93 (3H, s), 1.21 (3H, t), 1.42-1.72 (8H, m), 1.80-1.90 (2H, m), 2.02-2.12 (2H, m), 2.28-2.40 (2H, m), 2.29 (3H, s), 2.90-3.00 (2H, m), 3.35-3.43 (1H, m), 3.43-3.53 (3H, m), 6.64 (1H, d), 7.82 (1H, d), 8.05 (1H, d).

### Description 98. N-1-[trans-4-(Ethyloxy)-1-methylcyclohexyl]-4-piperidinyl}-4-fluoro-5-methyl-1,2-benzenediamine (D98)

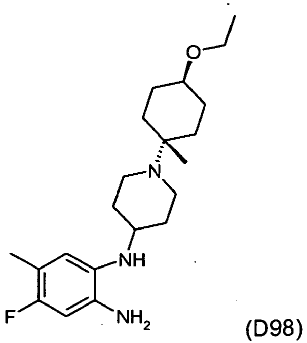

A stirred solution of 1-[*trans*-4-(ethyloxy)-1-methylcyclohexyl]-*N*-(4-fluoro-5-methyl-2-nitrophenyl)-4-piperidinamine (D97, 107mg, 0.28mmole) in ethanol (10ml) at room temperature under argon was treated with Raney Nickel (-20mg) followed by hydrazine hydrate (0.10ml, 3.2mmole) and maintained for 2 hrs. The mixture was filtered through Kieselguhr to remove catalyst and the filtrate concentrated under vacuum to afford the title compound as a pale yellow oil (90mg, 91%yield). MH⁺ 364.
¹H NMR δ (CDCl₃, 400MHz): 0.91 (3H, s), 1.20 (3H, t), 1.34-1.58 (6H, m), 1.60-1.70 (2H, m), 1.80-1.90 (2H, m), 2.02 (2H, br d), 2.15 (3H, s), 2.18-2.28 (2H, m), 2.40-2.90 (1H, vbr), 2.95 (2H, br d), 3.04-3.14 (1H, m), 3.3-3.53 (5H, m), 6.40-6.50 (2H, m).

### Description 99. N-(4-Chloro-5-methyl-2-nitrophenyl)-1-[trans-4-(ethyloxy)-1-methylcyclohexyl]-4-piperidinamine (D99)

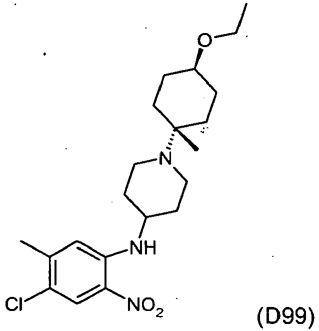

A stirred solution of 1-[*trans*-4-(ethyloxy)-methylcyclohexyl]-4-piperidinamine (D68 free base, 170mg, 0.71 mmole) in dimethylformamide (4ml) at room temperature under argon was treated with diisopropylethylamine (0.18ml, 1.0mmole) followed by 1-chloro-4-fluoro-2-methyl-5-nitrobenzene (D84, 190mg, 1.0mmole) and heated at 80°C for 20hrs. The mixture was concentrated under vacuum and the residue treated with 10% Na₂CO₃ solution and extracted with dichloromethane. The extract was dried, concentrated under vacuum and the residue chromatographed on silica gel (10g) eluting with 0-10% methanol/dichloromethane to afford the title compound as an orange solid (210mg, 72%). MH⁺ 410.
¹H NMR □(CDCl₃, 400 MHz): 0.94 (3H, s), 1.20 (3H, t), 1.40-1.70 (8H, m), 1.80-1.90 (2H, m), 2.07 (2H, br d), 2.28-2.40 (2H, m), 2.37 (3H, s), 2.90-3.00 (2H, m), 3.35-3.42 (1H, m), 3.42-3.55 (3H, m), 6.71 (1H, s), 8.06 (1H, d), 8.16 (1H, s).

### Description 100. 4-Chloro-N-{1-[trans-4-(ethyloxy)-1-methylcyclohexyl]-4-piperidinyl}-5-methyl-1,2-benzenediamine (D100)

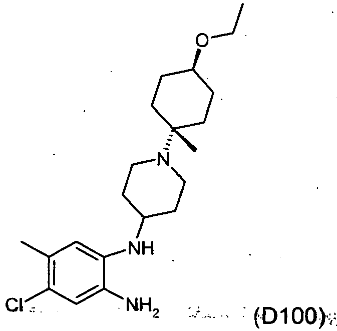

A stirred solution of *N*-(4-chloro-5-methyl-2-nitrophenyl)-[*trans*-4-(ethyloxy)-1-methylcyclohexyl]-4-piperidinamine (D99, 210mg, 0.51mmole) in ethanol (15ml) at room temperature under argon was treated with Raney Nickel (-20mg) followed by hydrazine hydrate (0.17ml), 3.5mmole) and maintained for 1 hr. The mixture was filtered through Kieselguhr to remove catalyst and the filtrate concentrated under vacuum to afford the title compound as a pale yellow oil (195mg, 100%yield). MH⁺ 380.
¹H NMR δ (CDCl₃, 400MHz): 0.94 (3H, s), 1.20 (3H, t), 1.40-1.60 (6H, m), 1.60-1.72 (2H, m), 1.80-1.92 (2H, m), 2.00-2.12 (2H, m), 2.20-2.35 (2H, m), 2.25 (3H, s), 2.90-3.03 (2H, m), 3.10-3.30 (3H, m), 3.32-3.40 (1H, m), 3.48 (2H, q), 6.47 (1H, s), 6.70 (1H, s). 1H not discernible from spectrum.

### Description 101. N-(4-Fluoro-5-methyl-2-nitrophenyl)-1-[cis-1-methyl-4-(propyloxy)cyclohexyl]-4-piperidinamine (D101)

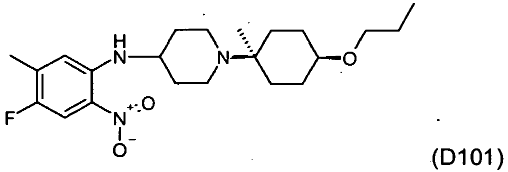

A microwave vessel was charged with 2,5-difluoro-4-nitrotoluene (0.254g), 1-[*cis*-1-methyl-4-(propyloxy)cyclohexyl]-4-piperidinamine (D119, 0.2g), dimethylformamide (3ml) and diisopropylethylamine (1.0ml) and heated at 200°C for 20 minutes in a microwave reactor. The cooled reaction was diluted with dichloromethane and washed with saturated sodium bicarbonate solution then the solvent was removed and the residue was chromatographed on silica gel eluted with 0-5% dichloromethane - methanolic ammonia to give the title as a yellow orange solid (0.274g). MH⁺ = 408, 409.

### Description 102. 4-Fluoro-5-methyl-N-{1-[cis-1-methyl-4-(propyloxy)cyclohexyl]-4-piperidinyl}-1,2-benzenediamine (D102)

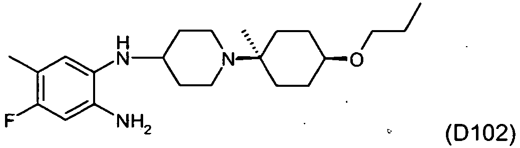

A solution of *N*-(4-fluoro-5-methyl-2-nitrophenyl)-1-[cis-1-methyl-4-(propyloxy)cyclohexyl]-4-piperidinamine (D101, 0.274g) in ethanol (15ml) was added over 10 min to a solution of tin (II) chloride (0.8g) in conc hydrochloric acid (8ml) stirred at 60°C. The mixture was heated at 60°C, for 2hr then poured into a mixture of dilute sodium hydroxide solution and dichloromethane. The dichloromethane layer was separated, washed with brine and dried with hydromatrix and the solvent was removed to give the title compound as a gum (0.15g). MH⁺ = 378.

### Description 103. N-(4-Fluoro-5-methyl-2-nitrophenyl)-1-[trans-1-methyl-4-(propyloxy)cyclohexyl]-4-piperidinamine (D103)

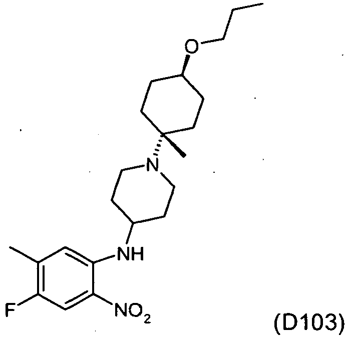

A stirred mixture of 1,4-difluoro-2-methyl-5-nitrobenzene (170mg, 0.98mmole), 1-[*trans*-1-methyl-4-(propoxy)cyclohexyl]-4-piperidinamine (D76 free base, 250mg, 0.98mmole) and diisopropylethylamine (0.2ml, 1.17mmole) in dimethylformamide (20ml) was heated at 80°C overnight under an argon atmosphere. The mixture was washed with sat. NaHCO₃ solution then extracted with dichloromethane. The organic phase was eluted through an SCX cartridge, washed with methanol and the compound was collected by elution with 2M NH₃ in MeOH. The solvent was removed under vacuum to leave the title compound as an orange solid (313mg, 78.5%). MH⁺= 408.

### Description 104. 4-Fluoro-5-methyl-N-{1-[trans-1-methyl-4-(propyloxy)cyclohexyl]-4-piperidinyl}-1,2-benzenediamine (D104)

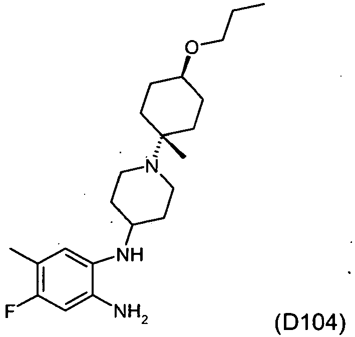

A stirred solution of *N*-(4-fluoro-5-methyl-2-nitrophenyl)-1-[*trans*-1-methyl-4-(propyloxy)cyclohexyl]-4-piperidinamine (D103, 313mg, 0.77mmole) in ethanol (30ml) at room temperature under argon was treated with Raney Nickel (100mg) and dropwise addition of hydrazine hydrate (1ml) and maintained for 1 hr. The solution was filtered through Kieselguhr and the filtrate concentrated under vacuum to leave the title compound as a colourless oil (280mg, 96%). MH⁺ = 378.

### Description 105. N-(4-Chloro-5-methyl-2-nitrophenyl)-1-[cis-1-methyl-4-(propyloxy)cyclohexyl]-4-piperidinamine (D105)

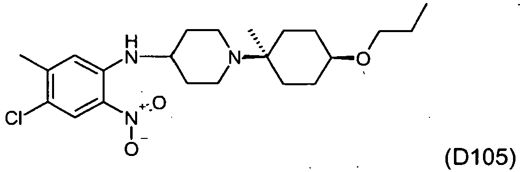

A microwave vessel was charged with 2-chloro-5-fluoro-4-nitrotoluene (0.22g), 1-[*cis*-1-methyl-4-(propyloxy)cyclohexyl]-4-piperidinamine (D119, 0.254g), dimethylformamide (3ml) and diisopropylethylamine (1.0ml) and heated at 200°C for 20 minutes in a microwave reactor. The cooled reaction was diluted with dichloromethane and washed with saturated sodium bicarbonate solution then the solvent was removed and the residue was chromatographed on silica gel eluted with 0-5% dichloromethane - methanolic ammonia to give the title as a yellow solid (0.18g). MH⁺ = 424, 426.

### Description 106. 4-Chloro-5-methyl-N-{1-[cis-1-methyl-4-(propyloxy)cyclohexyl]-4-piperidinyl}-1,2-benzenediamine (D106)

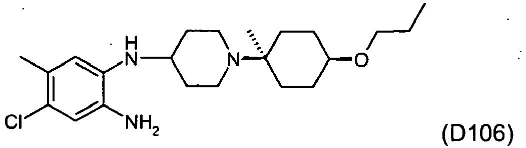

To a solution of *N*-(4-chloro-5-methyl-2-nitrophenyl)-1-[*cis*-1-methyl-4-(propyloxy)cyclohexyl]-4-piperidinamine (D105, 0.18g) in ethanol (30ml) and methanol (20ml), stirred at 40°C was added Raney-nickel (∼1ml) and hydrazine hydrate (1ml). The mixture was stirred for 20 min, filtered and the filtrate was evaporated to give the title compound as a gum (0.167g). MH⁺=394.

### Description 107. N-(4-Chloro-5-methyl-2-nitrophenyl)-1-[trans-1-methyl-4-(propyloxy)cyclohexyl]-4-piperidinamine (D107)

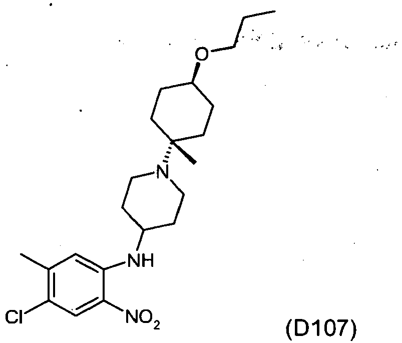

A stirred mixture of 1-chloro-4-fluoro-2-methyl-5-nitrobenzene (185mg, 0.98mmole), 1-[*trans*-1-methyl-4-(propoxy)cyclohexyl]-4-piperidinamine (D76 free base, 250mg, 0.98mmole) and diisopropylethylamine (0.2ml, 1.17mmole) in dimethylformamide (20ml) was heated at 80°C for 1.5hrs under an argon atmosphere. The mixture was washed with sat. NaHCO₃ solution then extracted with dichloromethane. The organic phase was eluted through an SCX cartridge, washed with methanol and the compound was collected by elution with 2M NH₃ in MeOH. The solvent was removed under vacuum to leave the title compound as an orange solid (340mg, 82%). MH⁺ = 424.

### Description 108. 4-Chloro-5-methyl-N-{1-[trans-1-methyl-4-(propyloxy)cyclohexyl]-4-piperidinyl}-1,2-benzenediamine (D108)

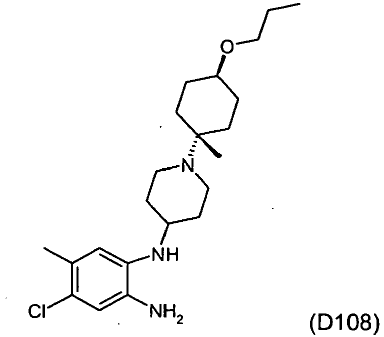

A stirred solution of *N*-(4-chloro-5-methyl-2-nitrophenyl)-1-[trans-1-methyl-4-(propyloxy)cyclohexyl]-4-piperidinamine (D107, 340mg, 0.8mmole) in ethanol (30ml) at room temperature under argon was treated with Raney Nickel (50mg) and dropwise addition of hydrazine hydrate (1ml) and maintained for 1 hr. The solution was filtered through Kieselguhr and the filtrate concentrated under vacuum to leave the title compound as a pale yellow oil (300mg, 95%). MH⁺ = 394.

### Descrtption:109. N-[5-Bromo-2--nitro-4-(trifluoromethyl)phenyl]-1-[trans-1-methyl-4-(propyloxy)cyclohexyl]-4-piperidinamine (D109)

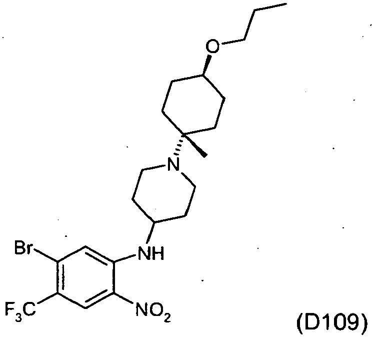

A stirred mixture of 1-bromo-5-fluoro-4-nitro-2-(trifluoromethyl)benzene (282mg, 0.98mmole), 1-[*trans*-1-methyl-4-(propoxy)cyclohexyl]-4-piperidinamine (D76 free base, 250mg, 0.98mmole) and diisopropylethylamine (0.2ml, 1.17mmole) in dimethylformamide (20ml) was heated at 50°C for 1 hr under an argon atmosphere. The mixture was washed with sat. NaHCO₃ solution then extracted with dichloromethane. The organic phase was eluted through an SCX cartridge, washed with methanol and the compound was collected by elution with 2M NH₃ in MeOH. The solvent was removed under vacuum to leave the title compound as a pale yellow solid (450mg, 88%). MH⁺ = 523, 524.
¹H NMR δ (CDCl₃, 400MHz): 0.90-0.97 (6H, s + t), 1.4-1.70 (assume 10H, m), 1.78-1.86 (2H, m), 2.02-2.13 (2H, m), 2.30-2.40 (2H, m), 2.90-3.00 (2H, m), 3.30-3.40 (3H, q + m), 3.45-3.58 (1H, m), 7.19 (1H, s), 8.28 (1H, d), 8.50 (1H, s).

### Description 110. 5-Bromo-N-{1-[trans-1-methyl-4-(propyloxy)cyclohexyl]-4-piperidinyl}-4-(trifluoromethyl)-1,2-benzenediamine (D110)

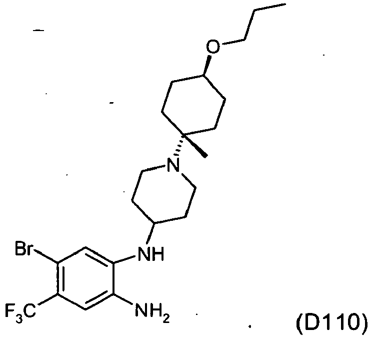

A stirred solution of *N*-[5-bromo-2-nitro-4-(trifluoromethyl)phenyl]-1-[*trans*-1-methyl-4-(propyloxy)cyclohexyl]-4-piperidinamine (D109, 450mg, 0.86mmole) in ethanol (50ml) at room temperature under argon was treated with Raney Nickel (50mg) and dropwise addition of hydrazine hydrate (1 ml) and maintained for 45 mins. The solution was filtered through Kieselguhr and the filtrate concentrated under vacuum to leave the title compound as a red oil (420mg, 91% purity). MH⁺ = 493, 494.

### Description 111. cis/trans-Methyl 4-(propyloxy)cyclohexanecarboxylate (D111)

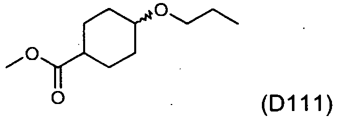

*cis*/*trans*-4-(Propyloxy)cyclohexanecarboxylic acid (D70, 13.3g), concentrated sulfuric acid (10ml) and methanol (100ml) was heated at reflux for 3 hours then allowed to cool overnight. The solvent was partially removed, then the residue was poured onto a mixture of ice and dichloromethane. The organic layer was separated, washed consecutively with saturated sodium bicarbonate and brine, then dried with anhydrous sodium sulphate and the solvent was removed to give the title compound as an oil (13.42g).

### Description 112. cis/trans-Methyl 1-methyl-4-(propyloxy)cyclohexanecarboxylate (D112)

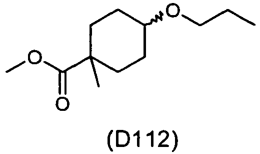

A stirred solution of diisopropylamine (11.2ml) in tetrahydrofuran (180ml) at -78°C under argon was treated over 10 mins with 2.5M n-butyllithium in hexane (32ml), then stirred for a further 30 minutes when a solution of *cis*/*trans*-methyl 4-(propyloxy)cyclohexane carboxylate (D111, 13.4g) in tetrahydrofuran (30ml) was added over 10 minutes. The mixture was stirred at -78°C for 1 hour when methyl iodide (4.9ml) was added and the mixture was stirred for 2 hours then allowed to warm to room temperature overnight. The mixture was poured into 10% citric acid solution then extracted with hexane. The hexane layer was washed with saturated sodium bicarbonate solution, brine then dried (Na₂SO₄) and the solvent was removed to give the title compound as an oil (13.7g), mainly the *cis* isomer.

### Description 113. cis/trans-1-Methyl-4-(propyloxy)cyclohexanecarboxylic acid (D113)

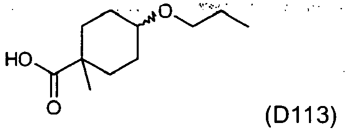

*cis*/*trans*-Methyl 1-methyl-4-(propyloxy)cyclohexanecarboxylate (D112, 13.7g), 50% w/w sodium hydroxide solution (25ml), tetrahydrofuran (75ml) and methanol (25ml) was refluxed for 4 hours then allowed to cool to room temperature overnight. The solvent was partially removed then the resulting mixture was poured onto ice and extracted with hexane. The aqueous layer was separated, acidified with concentrated hydrochloric acid and extracted with diethyl ether. The ether layer was washed with brine, dried with anhydrous sodium sulphate and the solvent was removed to give the title compound as an oil (12.13g).

### Description 114. cis-(1,1-Dimethylethyl)(diphenyl)silyl 1-methyl-4-(propyloxy)cyclohexanecarboxylate (D114)

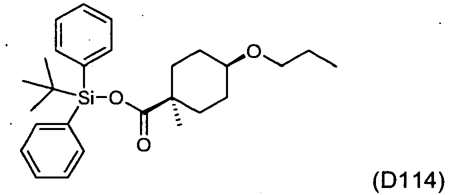

A solution of chloro(1,1-dimethylethyl)diphenylsilane (12.8g), *cis*/*trans*-1-methyl-4-(propyloxy)cyclohexanecarboxylic acid (D113, 9.2g) and imidazole (6.25g) in dimethylformamide (50ml) was stirred at 80°C for 2 hours, then at room temperature over the weekend. The mixture was then poured into 10% citric acid solution and extracted with hexane. The extracts were washed with water, brine and then dried (Na₂SO₄) and the solvent was removed and the residue was chromatographed on silica gel eluted with 0-5% ethyl acetate/hexane to give the title compound (8.59g).

### Description 115. cis-1-Methyl-4-(propyloxy)cyclohexanecarboxylic acid (D115)

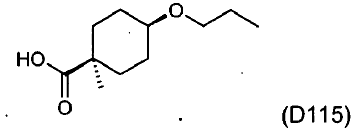

A solution of *cis*-(1,1-dimethylethyl)(diphenyl)silyl 1-methyl-4-(propyloxy)cyclohexanecarboxylate (D114, 8.59g), tetrabutylammonium fluoride solution in tetrahydrofuran (1M, 30ml) and tetrahydrofuran (100ml) was stirred at room temperature for 2 days. The mixture was then treated with citric acid solution and extracted twice with diethyl ether. The organic extracts were combined and extracted twice with sodium hydroxide solution (2M). These extracts were washed with ether and the ether was discarded. The aqueous was acidified and extracted with ether. The ether layer was dried (Na₂SO₄) and solvent removed to leave the title compound as a white solid (3.76g).

### Description 116. cis-1-Isocyanato-1-methyl-4-(propyloxy)cyclohexane (D116)

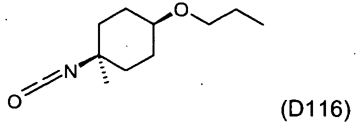

A stirred solution of *cis*-1-methyl-4-(propyloxy)cyclohexanecarboxylic acid (D115, 3.76g) in toluene (50ml) at room temperature under argon was treated with triethylamine (3.8ml) and diphenylphosphoryl azide (3.9ml) and heated at 100°C for 20 minutes. The mixture was allowed to cool to room temperature, washed with sodium bicarbonate solution, brine and dried hydromatrix to give the title compound as a oil (3.82)

### Description 117. cis-1-Methyl-4-(propyloxy)cyclohexanamine hydrochloride (D117)

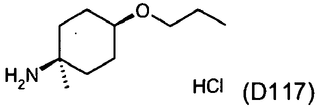

A solution of *cis*-1-isocyanato-1-methyl-4-(propyloxy)cyclohexane (D116, 3.82g), water (20ml) and 4M hydrogen chloride in dioxane (20ml) was stirred at room temperature for 4 hours, then the solvent was removed to give the title compound as a sticky solid (3.36g).

### Description 118. 1-[cis-1-Methyl-4-(propyloxy)cyclohexyl]-4-piperidinone (D118)

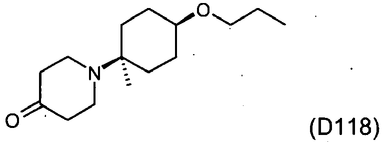

A solution of [*cis*-1-methyl-4-(propyloxy)cyclohexyl]amine hydrochloride (D117, 3.36g) in ethanol (50ml) and water (25ml) together with potassium carbonate (3.8g) and 1-ethyl-1-methyl-4-oxopiperidinium iodide (D49, 6.7) was heated at reflux for 2 hours. The solvent was partially removed and then the aqueous residue was treated with sat. NaHCO₃ solution and extracted twice with dichloromethane. The combined extracts were washed with brine, dried hydromatrix and the solvent was removed to afford the title compound as a oil (2.92g).

### Description 119. 1-[cis-1-Methyl-4-(propyloxy)cyclohexyl]-4-piperidinamine (D119)

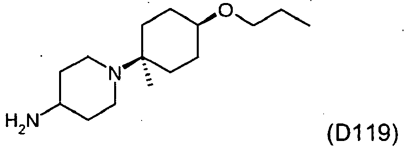

A solution of 1-[*cis*-1-methyl-4-(propyloxy)cyclohexyl]-4-piperidinone (D118, 2.92g) in 2M NH₃/MeOH (150ml) was treated with 10% Pd/C (2g) and shaken under hydrogen atmosphere at 50psi initial pressure for 1 overnight. The mixture was filtered to remove catalyst and the filtrate concentrated under vacuum to leave the title compound as an oil (2.81g).

### Description 120. (2-Amino-4,5-dimethylphenyl){1-[trans-4-(ethyloxy)-1-methylcyclohexyl]-4-piperidinyl}amine (D120)

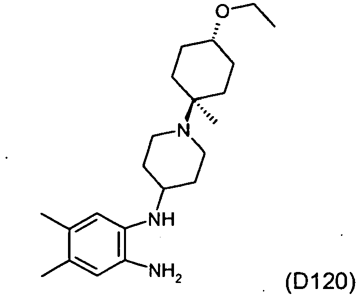

4,5-Dimethyl-1,2-benzenediamine (100mg, 0.74mmol) and 1-[*trans*-4-(ethyloxy)-1-methylcyclohexyl]-4-piperidinone (D67, 176mg, 0.74mmol) were dissolved in isopropyl acetate (5ml) at room temperature under argon. Trifluoroacetic acid (0.1ml, 1.48mmol) was added, followed by the addition of sodium triacetoxyborohydride (188mg, 0.89mmol) over approximately one minute. The mixture was stirred at room temperature under argon for 2 hours. Further sodium triacetoxyborohydride (188mg, 0.89mmol) added and stirring continued for 30 minutes. More isopropyl acetate (2ml) was added, followed by sodium triacetoxyborohydride (313mg, 1.48mmol) and the mixture was then stirred under argon at room temperature overnight. Aqueous sodium hydroxide (0.5M) was added until solution reached pH 10, then the layers were separated. The aqueous layer was extracted with ethyl acetate, and the combined organic layers were washed with brine, dried over Na₂SO₄ and concentrated under vacuum to give orange crystals (200mg). These crystals were purified by silica chromatography (Biotage 12s column, gradient 0-10% NH₃/MeOH/DCM over 10 column volumes followed by 10 column volumes at 10%) to afford the title compound (35mg). MH⁺ = 360.

### Description 121. (2-Amino-4,5-difluorophenyl){1-[trans-4-(ethyloxy)-1-methylcyclohexyl]-4-piperidinyl}amine (D121)

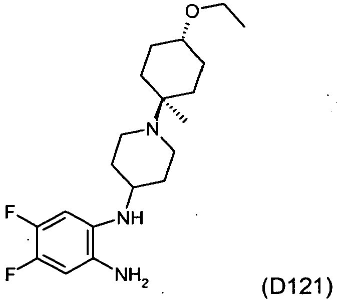

4,5-Difluoro-1,2-benzenediamine (380mg, 2.6mmol) and 1-[*trans*-4-(ethyloxy)-1-methylcyclohexyl]-4-piperidinone (D67, 500mg, 2.1 mmol) were dissolved in isopropyl acetate (20ml) under argon at room temperature. Trifluoroacetic acid (0.3ml, 4.44mmol) was added, followed by addition of sodium triacetoxyborohydride 2.3g, 10.5mmol). The mixture was stirred under argon at room temperature for 30 minutes, followed by a further 3 hours of the same. Water (≈10ml) was added, followed by portionwise addition of sodium hydroxide until the mixture reached a pH of approximately 10. The layers were separated and the aqueous layer was extracted with ethyl acetate. The combined organic layers were washed with brine, dried over sodium sulphate and concentrated under vacuum to give the title compound as brown/yellow solid (1.3g). MH⁺ = 368.

### Description 122. N-(4-Chloro-2-nitrophenyl)-1-[trans-1-methyl-4-(methyloxy)cyclohexyl]-4-piperidinamine (D122)

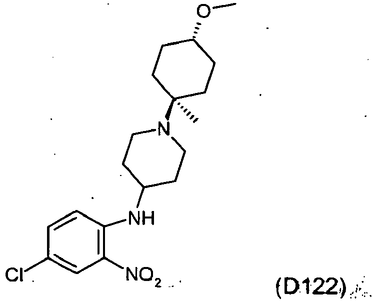

Diisopropylethylamine (0.52mL, 3.06mmole) and 5-chloro-2-fluoro-1-nitrobenzene (359mg, 2.05mmole) were added to a solution of 1-[*trans*-4-(methyloxy)-1-methylcyclohexyl]-4-piperidinamine dihydrochloride (diHCl salt of D59, 302mg, 1.01 mmole) in N,N-dimethylformamide (5ml) at r.t. The reaction was heated at 110°C in a microwave reactor for 30min and then the mixture was poured on to sat. NaHCO₃ solution (50mL) and extracted with EtOAc (3x). The combined organics were washed sequentially with brine, H₂O and brine, dried (Na₂SO₄) and concentrated via rotary evaporation. The crude residue was purified by SCX (5g) and then chromatographed (silica, CH₂Cl₂-0.5% NH₃ /9.5% MeOH /90% CH₂Cl₂) to yield the title compound (190mg, 49%) as an orange solid. M(CI-35)⁺ 382, M(CI-37)H⁺ 384.

### Description 123. (2-Amino-4-chlorophenyl){1-[trans-1-methyl-4-(methyloxy)cyclohexyl]-4-piperidinyl}amine (D123)

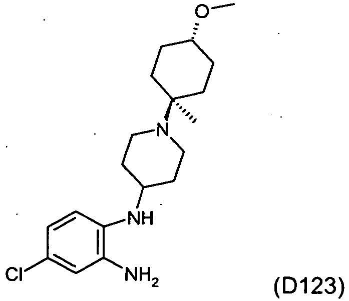

*N*-(4-Chloro-2-nitrophenyl)-1-[*trans*-1-methyl-4-(methyloxy)cyclohexyl]-4-piperidinamine (D122, 190mg, 0.50mmole) was suspended in EtOH (10mL) under argon. The reaction was stirred vigorously and Raney Nickel (0.25mL, 10% sol. in H₂O) was added, followed by the hydrazine hydrate (150µL, 4.82mmole). A second portion of Raney Nickel (0.25mL, 10% sol. in H₂O) was added and the reaction was stirred vigorously for 30min. The mixture was filtered through Kieselguhr using EtOH followed by MeOH and EtOAc and concentrated by rotary evaporation. The crude residue was taken up in EtOH and concentrated twice and then in CH₂Cl₂ and concentrated twice to give the title compound (84mg, 48%) as a brown solid. M(CI-35)⁺ 352, M(CI-37)H⁺ 354.

### Description 124. N-(4-Chloro-2-nitrophenyl)-1-[trans-4-(ethyloxy)-1-methylcyclohexyl]-4-piperidinamine (D124)

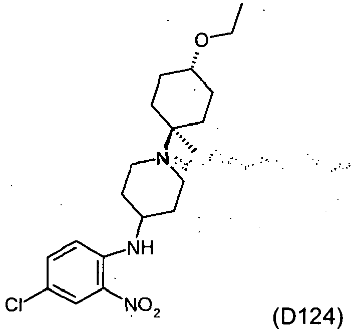

Diisopropylethylamine (0.52mL, 3.06mmole) and 5-chloro-2-fluoro-1-nitrobenzene (365mg, 2.08mmole) were added to a solution of 1-[*trans*-4-(ethyloxy)-1-methylcyclohexyl]-4-piperidinamine dihydrochloride (D68, 317mg, 1.01mmole) in N,N-dimethylformamide (5ml) at r.t. The reaction was heated at 110°C in a microwave reactor for 30min and then the mixture was poured on to sat. NaHCO₃ solution (50mL) and extracted with EtOAc (3x). The combined organics were washed sequentially with brine, H₂O and brine, dried (Na₂SO₄) and concentrated via rotary evaporation. The crude residue was purified by SCX (5g) and then chromatographed (silica, CH₂Cl₂-0.5% NH₃ /9.5% MeOH /90% CH₂Cl₂) to yield the title compound (117mg, 29%) as an orange solid. M(CI-35)⁺ 396, M(CI-37)H⁺ 398.

### Description 125. (2-Amino-4-chlorophenyl){1-[trans-4-(ethyloxy)-1-methylcyclohexyl]-4-piperidinyl}amine (D125)

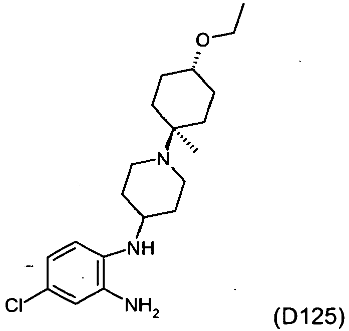

*N*-(4-Chloro-2-nitrophenyl)-1-[*trans*-4-(ethyloxy)-1-methylcyclohexyl]-4-piperidinamine (D124, 117mg, 0.29mmole) was suspended in EtOH (10mL) under argon. The reaction was stirred vigorously and Raney Nickel (0.25mL, 10% sol. in H₂O) was added, followed by the hydrazine hydrate (90µL, 2.89mmole). A second portion of Raney Nickel (0.25mL, 10% sol. in H₂O) was added and the reaction was stirred vigorously for 30min. The mixture was filtered through Kieselguhr using EtOH and concentrated by rotary evaporation. The crude residue was azeotroped with toluene (2x) to give the title compound (102mg, 96%) as a pale brown solid. M(CI-35)⁺ 366, M(CI-37)H⁺ 368.

### Description 126. N-(5-Chloro-4-cyano-2-iodophenyl)-N'-{1-[trans-4-(ethyloxy)cyclohexyl]-4-piperidinyl}urea (D126)

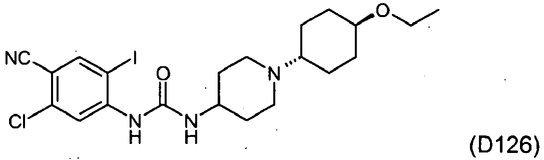

A stirred solution of 4-amino-2-chloro-5-iodobenzonitrile (J. Med. Chem. 2001, 44 (23), 3866) (440mg, 1.60mmole) in dioxane (5ml) at room temperature under argon was treated with solid triphosgene (160mg, 0.54mmole), maintained at room temperature for 15 minutes, then heated at 95°C for 20 minutes. The mixture was allowed to cool, concentrated under vacuum and the residue dissolved in dichloromethane (10ml) and treated with a solution of 1-(*trans*-4-ethoxycyclohexyl)piperidin-4-amine (D20, 360mg, 1.60mmole) in dichloromethane (5ml). The mixture was stirred at room temperature for 18 hrs when a precipitate had formed. This was filtered off and dried to give a white solid (560mg). The material proved very insoluble in MeOH, so it was dissolved in DMSO and loaded on to an SCX cartridge (2g) which was washed with MeOH and then eluted with 2M NH₃/MeOH to remove part of the relatively insoluble material. The ammonia solution was concentrated under vacuum to leave the title compound as a white solid (300mg). MH⁺ 531.
¹H NMR δ (d⁶DMSO, 400MHz): 1.06 (3H, t), 1.04-1.40 (6H, m), 1.73 (2H, br d), 1.82 (2H, br d), 1.99 (2H, br d), 2.18-2.30 (3H, m), 2.75 (2H, br d), 3.08-3.18 (1H, m), 3.37-3.48 (3H, m + q), 7.64 (1H, d), 7.93 (1H, s), 8.37 (2H, s).

### Example 1. 5-Fluoro-6-methyl-1-{1-[cis-4-(methyloxy)cyclohexyl]-4-piperidinyl}-1,3-dihydro-2H-benzimidazol-2-one hydrochloride (E1)

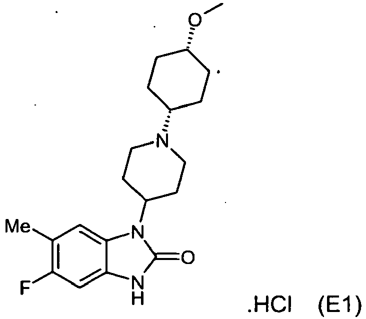

A stirred solution of (2-amino-4-fluoro-5-methylphenyl){1-[*cis*-4-(methyloxy)cyclohexyl]-4-piperidinyl}amine (D9, 0.33g, 1.04 mmole) and diisopropylethylamine (0.28ml, 1.56 mmole) in dichloromethane (30ml) at 0°C under argon was treated with solid triphosgene (123mg, 0.42 mmole) and maintained at 0°C for 2h. The mixture was treated with water followed by dil. NaHCO₃ solution and extracted with dichloromethane (3x20ml). The combined extract was dried with Na₂SO₄ and concentrated under vacuum to leave the title compound as a white solid (337mg, 95% yield). A portion of this material (260mg) was dissolved in methanol/diethyl ether (1:3 mixture, 10ml), treated with 1M HCl/diethyl ether (5ml) and concentrated under vacuum to give the hydrochloride salt as a white solid (300mg). MH⁺ = 362 and 363.
¹H NMR (HCl salt) δ (CD₃OD, 400MHz): 1.55 (2H, m), 1.8-1.9 (4H, m), 2.1-2.2 (4H, m), 2.3 (3H, s), 2.8-2.9 (2H, m), 3.3-3.4 (6H, m), 3.5 (1H, s), 3.65 (2H, m), 4.5 (1H, m), 6.8 (1H, d), 7.2 (1H, m).

### Example 2. 5-Fluoro-6-methyl-1-{1-[trans-4-(methyloxy)cyclohexyl]-4-piperidinyl}-1,3-dihydro-2H-benzimidazol-2-one hydrochloride (E2)

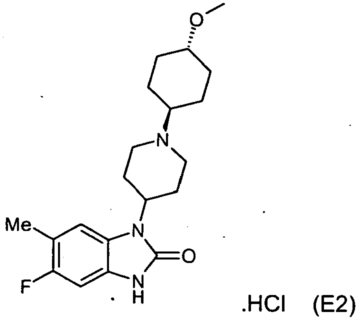

A stirred solution of (2-amino-4-fluoro-5-methylphenyl){1-[*trans*-4-(methyloxy)cyclohexyl]-4-piperidinyl}amine (D10, 0.25g, 0.78 mmole) and diisopropylethylamine (0.21 ml, 1.17 mmole) in dichloromethane (25ml) at 0°C under argon was treated with solid triphosgene (92mg, 0.31 mmole) and maintained at 0°C for 2h. The mixture was treated with water followed by dil. NaHCO₃ solution and extracted with dichloromethane. The combined extract was dried with Na₂SO₄ and concentrated under vacuum to leave the title compound as a white solid (173mg, 62% yield). This material was dissolved in methanol/diethyl ether (1:3 mixture, 10ml), treated with 1M HCl/diethyl ether (5ml) and concentrated under vacuum to give the hydrochloride salt as a white solid (190mg).
¹H NMR (HCl salt) δ (CD₃OD, 400MHz): 1.3-1.4 (2H, m), 1.6-1.7 (2H, m), 2.1-2.3 (6H, m), 2.3 (3H, s), 2.8-2.9 (2H, m), 3.3-3.4 (7H, m), 3.65 (2H, m), 4.5 (1H, m), 6.8 (1H, d), 7.2 (1H, d).
MH⁺ = 362 and 363.

### Example 3. 5-Chloro-6-methyl-1-{1-[cis-4-(methyloxy)cyclohexyl]-4-piperidinyl}-1,3-dihydro-2H-benzimidazol-2-one hydrochloride (E3)

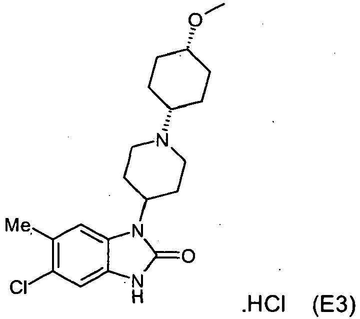

A stirred solution of (2-amino-4-chloro-5-methylphenyl){1-[*cis*-4-(methyloxy)cyclohexyl]-4-piperidinyl}amine (D12, 0.22g, 0.63 mmole) and diisopropylethylamine (0.17ml, 0.95 mmole) in dichloromethane (20ml) at 0°C under argon was treated with solid triphosgene (76mg, 0.25 mmole) and maintained at 0°C for 30 minutes. The mixture was treated with water followed by dil. NaHCO₃ solution and extracted with dichloromethane (3x 20ml). The combined extract was dried with Na₂SO₄ and concentrated under vacuum to leave the title compound as a white solid (227mg, 96% yield). This material was dissolved in dichloromethane (10ml), treated with 1M HCl/diethyl ether (5ml) and concentrated under vacuum to give the hydrochloride salt as a pale yellow solid.
¹H NMR (HCl salt) δ (CD₃OD, 400MHz): 1.5 (2H, m), 1.8-2.0 (4H, m), 2.1-2.2 (4H, m), 2.4 (3H, s), 2.8-2.9 (2H, m), 3.3-3.4 (6H, m), 3.5 (1H, s), 3.7 (2H, m), 4.5-4.6 (1H, m), 7.1 (1H, s), 7.3 (1H, s).
MH⁺ = 378, 379, 380 and 381.

### Example 4. 5-Chloro-6-methyl-1-{1-[trans-4-(methyloxy)cyclohexyl]-4-piperidinyl}-1,3-dihydro-2H-benzimidazol-2-one hydrochloride (E4)

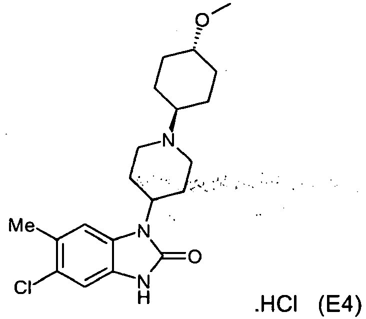

A stirred solution of (2-amino-4-chloro-5-methylphenyl){1-[*trans*-4-(methyloxy)cyclohexyl]-4-piperidinyl}amine (D13, 0.13g, 0.36 mmole) and diisopropylethylamine (0.1ml, 0.54 mmole) in dichloromethane (15ml) at 0°C under argon was treated with solid triphosgene (43mg, 0.14 mmole) and maintained at 0°C for 30min. The mixture was treated with water followed by dil. NaHCO₃ solution and extracted with dichloromethane (3x 20ml). The combined extract was dried with Na₂SO₄ and concentrated under vacuum to leave a white solid. Residue was a mixture of benzimidazolone (80%) and starting material (20%): residue was chromatographed on silica (5g of silica for 150mg of crude: 0% to 5% 0.4M NH₃ in methanol/dichloromethane 8CV) to afford 80mg of title compound, (59% yield). This material was dissolved in dichloromethane (10ml); treated with 1M HCl/diethyl ether (5ml) and concentrated under vacuum to give the hydrochloride salt as a white solid.
¹H NMR (HCl salt) δ (CD₃OD, 400MHz): 1.3-1.4 (2H, m), 1.6-1.7 (2H, m), 2.1 (2H, m), 2.2-2.3 (4H, m), 2.4 (3H,s), 2.8-2.9 (2H, m), 3.2-3.4 (7H, m), 3.65 (2H, m), 4.55 (1H, m), 7.0 (1H, s), 7.3 (1H, s).
MH⁺ = 378, 379, 380 and 381.

### Example 5. 6-Bromo-1-{1-[cis-4-(methyloxy)cyclohexyl]-4-piperidinyl}-5-(trifluoromethyl)-1,3-dihydro-2H-benzimidazol-2-one hydrochloride (E5)

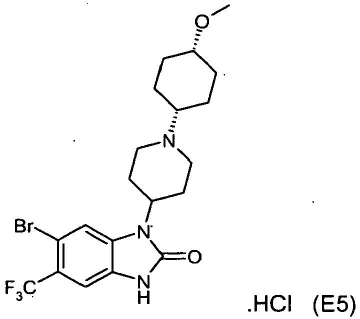

A stirred solution of 2-amino-5-bromo-4-(trifluoromethyl)phenyl]{1-[*cis*-4-(methyloxy) cyclohexyl]-4-piperidinyl}amine (D15, 480mg, 1.04 mmole) and diisopropylethylamine (0.29ml, 1.59 mmole) in dichloromethane (35ml) at 0°C under argon was treated with solid triphosgene (127mg, 0.42 mmole) and maintained at 0°C for 1h. The mixture was treated with water followed by dil. NaHCO₃ solution and extracted with dichloromethane (3x20ml). The combined extract was dried with Na₂SO₄ and concentrated under vacuum to leave a beige solid (mix: 40%, of compound + impurity). The residue was chromatographed on silica column (550mg of crude for 2x40g of silica: 3CV 5% to 5%, 10CV 5% to 15%, 3CV 15% to 15% 0.4M NH₃ in methanol/dichloromethane) to give the title compound as a white solid (130mg). This material was dissolved in dichloromethane (10ml), treated with 1 M HCl/diethyl ether (5ml) and concentrated under vacuum to give the hydrochloride salt as a white solid (150mg, 29% yield).
¹H NMR (HCl salt) δ (CD₃OD, 400MHz): 1.5-1.6 (2H, m), 1.8-1.9 (4H, m), 2.1-2.2 (4H, m), 2.65-2.9 (2H, m), 3.2-3.4 (6H, m), 3.5 (1H, s), 3.7 (2H, m), 4.55 (1H, m), 7.4 (1H, s), 7.8 (1H, s).
MH⁺ = 477, 478 and 479.

### Example 6. 6-Bromo-1-{1-[trans-4-(methyloxy)cyclohexyl]-4-piperidinyl}-5-(trifluoromethyl)-1,3-dihydro-2H-benzimidazol-2-one hydrochloride (E6)

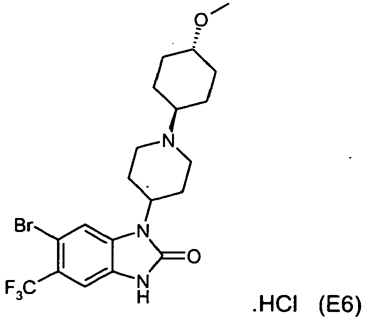

A stirred solution of [2-amino-5-bromo-4-(trifluoromethyl)phenyl]{1-[*trans*-4-(methyloxy) cyclohexyl]-4-piperidinyl}amine (D16, 212g, 0.47mmole) and diisopropylethylamine (0.13ml, 0.7mmole) in dichloromethane (20ml) at 0°C under argon was treated with solid triphosgene (60mg, 0.19mmole) and maintained at 0°C for 1h. The mixture was treated with water followed by dil. NaHCO₃ solution and extracted with dichloromethane. The combined extract was dried with Na₂SO₄ and concentrated under vacuum to leave a pale yellow solid (220mg: mixture of compound (55%) and subcompounds). The residue was purified with MDAP to afford the title compound as a white solid (60mg, 27%yield). This material was dissolved in dichloromethane (5ml), treated with 1M HCl/diethyl ether (1ml) and concentrated under vacuum to give the hydrochloride salt as a white solid.
¹H NMR (HCl salt) δ (CD₃OD, 400MHz): 1.3-1.4 (2H, m), 1.6-1.7 (2H, m), 2.1-2.2 (4H, m), 2.3 (2H, m), 2.75-2-85 (2H, m), 3.2-3.3 (4H, m), 3.4 (3H, s), 3.7 (2H, m), 4.6 (1H, m), 7.4 (1H, s), 7.7 (1H, s).
MH⁺ = 477, 478 and 479.

### Example 7. 1-{1-[trans-4-(Ethyloxy)cyclohexyl]-4-piperidinyl}-5-fluoro-6-methyl-1,3-dihydro-2H-benzimidazol-2-one hydrochloride (E7)

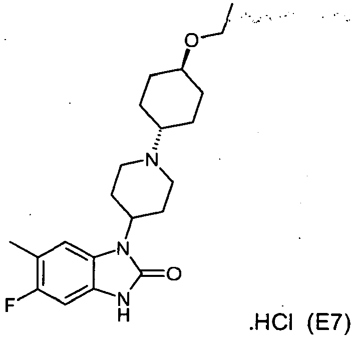

A stirred solution of *N*-{1-[*trans*-4-(ethyloxy)cyclohexyl)-4-piperidinyl}-4-fluoro-5-methyl-1,2-benzenediamine (D22, 74mg, 0.212mmole) and diisopropylethylamine (0.061 ml), 0.337mmole) in dichloromethane (10ml) at 0°C under argon was treated with solid triphosgene (25mg, 0.085mmole) and maintained at 0°C for 1 h. The mixture was treated with water (10ml) followed by NaHCO₃ solution (10ml) and extracted with dichloromethane (2 x 10ml). The combined extract was dried (Na₂SO₄) and concentrated under vacuum. The residue was triturated with 1:1 ethyl acetate/diethyl ether (5ml), allowed to stand for 20 minutes, then the solid filtered off, washed with cold 1:1 ethyl acetate/diethyl ether and dried to afford the title compound as a white solid (57mg, 72%). This was dissolved in dichloromethane (2ml) and methanol (0.4ml), treated with 1M HCl/diethyl ether (0.25ml) and concentrated under vacuum. The residue was triturated with diethyl ether and the solid obtained filtered off, washed with diethyl ether and dried to afford the hydrochloride salt as a white solid.
¹H NMR (HCl salt) □ (d⁶DMSO, 400 MHz): 1.09 (3H, t), 1.12-1.28 (2H, m), 1.47-1.62 (2H, m), 1.80-1.90 (2H, m), 2.03-2.22 (4H, m), 2.24 (3H, s), 2.80-2.98 (2H, m), 3.13-3.30 (4H, m), 3.40-3.68 (4H, m), 4.50-4.65 (1H, m), 6.79 (1H, d), 7.70 (1H, d), 10.80 (1H, br m), 10.98 (1H, s).

### Example 8. 5-Chloro-6-methyl-1-{1-[cis-4-(propyloxy)cyclohexyl]-4-piperidinyl}-1,3-dihydro-2H-benzimidazol-2-one hydrochloride (E8)

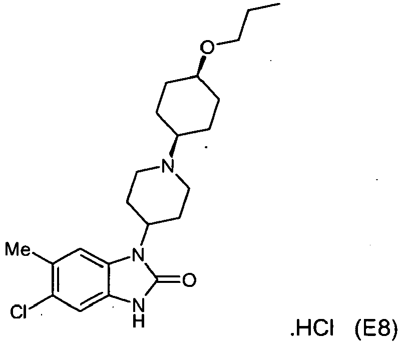

A stirred solution of 4-chloro-5-methyl-*N*-{1-[*cis*-4-(propyloxy)cyclohexyl]-4-piperidinyl}-1,2-benzenediamine (D30, 630mg, 1.66mmole) and diisopropylethylamine (0.44ml, 2.49mmole) in dichloromethane (40ml) at 0°C under argon was treated with solid triphosgene (198mg, 0.66mmole) and maintained at 0°C for 40 minutes. The mixture was treated with water (20ml) followed by dil. NaHCO₃ solution (25ml) and extracted with dichloromethane (2 x 30ml). The combined extract was dried (Na₂SO₄) and concentrated under vacuum to leave a yellow oil, which crystallised from ethyl acetate (15ml) to afford the title compound as a beige solid (347mg, 52%). A portion of this material (300mg) was dissolved in dichloromethane (7ml), treated with 1M HCl/diethyl ether (1ml) and concentrated under vacuum to give the hydrochloride salt as a white solid.
¹H NMR (HCl salt) □(d⁶DMSO, 400 MHz): 0.90 (3H, t), 1.38-1.58 (4H, m), 1.62-1.77 (2H, m), 1.80-2.05 (6H, m), 2.34 (3H, s), 2.82-2.97 (2H, m), 3.17-3.40 (6H, m), 3.45-3.55 (2H, m), 4.50-4.62 (1H, m), 7.00 (1H, s), 7.83 (1H, s), 10.78 (1H, br s), 11.00 (1H, s).

### Example 9. 5-Chloro-6-methyl-1-{1-[trans-4-(propyloxy)cyclohexyl]-4-piperidinyl}-1,3-dihydro-2H-benzimidazol-2-one hydrochloride (E9)

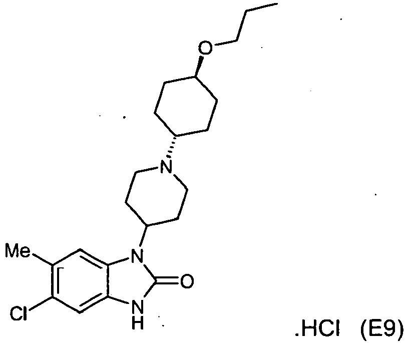

A stirred solution of 4-chloro-5-methyl-*N*-{1-[*trans*-4-(propyloxy)cyclohexyl]-4-piperidinyl}-1,2-benzenediamine (D32, 360mg, 0.95mmole) and diisopropylethylamine (0.25ml, 1.42mmole) in dichloromethane (25ml) at 0°C under argon was treated with solid triphosgene (113mg, 0.38mmole) and maintained at 0°C for 45 minutes. The mixture was treated with water (15ml) followed by sat. NaHCO₃ solution (10ml) and extracted with dichloromethane. The extract was dried (Na₂SO₄) and concentrated under vacuum. Trituration of the residue with 1:1 ethyl acetate/diethyl ether produced a solid which was filtered off, washed with 1:1 ethyl acetate/diethyl ether and dried at 50°C under vacuum to afford the title compound as a white solid (260mg, 68%). A portion of this material (210mg) was dissolved in dichloromethane (5ml), treated with 1 M HCl/diethyl ether (0.7ml) and concentrated under vacuum to give the hydrochloride salt as a white solid.
¹H NMR (HCl salt) □ (d⁶DMSO, 400 MHz): 0.86 (3H, t), 1.15-1.28 (2H, m), 1.42-1.62 (4H, m), 1.80-1.90 (2H, m), 2.05-2.25 (4H, m), 2.34 (3H, s), 2.84-3.00 (2H, m), 3.12-3.30 (4H, m), 3.30-3.40 (2H, m), 3.45-3.52 (2H, m), 4.53-4.66 (1H, m), 6.70 (1H, s), 7.86 (1H, s), 10.95 (1H, br s), 10.98 (1H, s).

### Example 10. 5-Fluoro-6-methyl-1-{1-[cis-4-(propyloxy)cyclohexyl]-4-piperidinyl}-1,3-dihydro-2H-benzimidazol-2-one hydrochloride (E10)

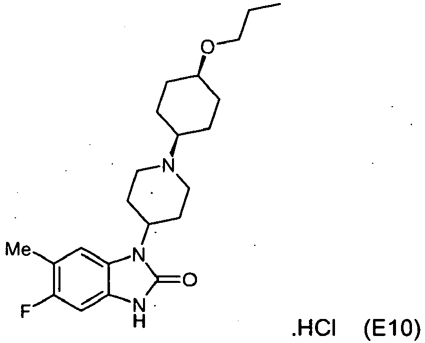

A stirred solution of 4-fluoro-5-methyl-*N*-{1-[*cis*-4-(propyloxy)cyclohexyl]-4-piperidinyl}-1,2-benzenediamine (D34, 290mg, 0.80mmole) and diisopropylethylamine (0.21 ml, 1.20mmole) in dichloromethane (20ml) at 0°C under argon was treated with solid triphosgene (95mg, 0.32mmole) and maintained at 0°C for 1 h. The mixture was treated with water (10ml) followed by dil. NaHCO₃ solution (15ml) and extracted with dichloromethane (2 x 20ml). The combined extract was dried (Na₂SO₄) and concentrated under vacuum. The residue was triturated with diethyl ether to afford the title compound as a white solid (157mg, 51%). A portion of this material (117mg) was dissolved in dichloromethane (2ml) and methanol (0.2ml), treated with 1 M HCl/diethyl ether (0.45ml) and concentrated under vacuum to give the hydrochloride salt as a white solid.
¹H NMR (HCl salt) □ (d⁶DMSO, 400 MHz): 0.90 (3H, t), 1.38-1.58 (4H, m), 1.62-1.77 (2H, m), 1.80-2.05 (5H, m), 2.23 (3H, s), 2.82-3.00 (2H, m), 3.18-3.40 (6H, m), 3.44-3.55 (3H, m), 4.50-4.65 (1H, m), 6.69 (1H, d), 7.71 (1H, d), 10.78 (1H, br s), 10.95 (1H, s).

### Example 11. 5-Fluoro-6-methyl-1-{1-[trans-4-(propyloxy)cyclohexyl]-4-piperidinyl}-1,3-dihydro-2H-benzimidazol-2-one hydrochloride (E11)

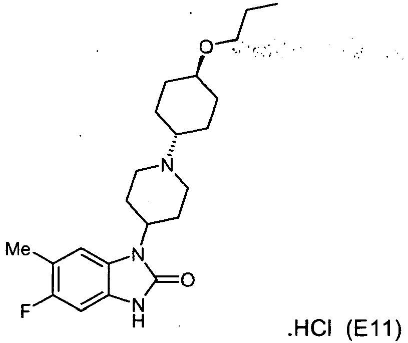

A stirred solution of 4-fluoro-5-methyl-*N*-{1-[*trans*-4-(propyloxy)cyclohexyl]-4-piperidinyl}-1,2-benzenediamine (D36, 158mg, 0.44mmole) and diisopropylethylamine (0.12ml, 0.66mmole) in dichloromethane (15ml) at 0°C under argon was treated with solid triphosgene (52mg, 0.18mmole) and maintained at 0°C for 40 minutes. The mixture was treated with water (10ml) followed by dil. NaHCO₃ solution (15ml) and extracted with dichloromethane (2 x 15ml). The combined extract was dried (Na₂SO₄) and concentrated under vacuum. Trituration of the residue with 1:1 ethyl acetate/diethyl ether (8ml) produced a white solid which was filtered off, washed with cold 1:1 ethyl acetate/diethyl ether and dried at 50°C under vacuum to afford the title compound as a white solid (89mg, 53%). This material was dissolved in dichloromethane (1ml) and methanol (0.3ml), treated with 1 M HCl/diethyl ether (0.34ml) and the solvent evaporated off under a stream of air to leave the hydrochloride salt as a white solid.
¹H NMR (HCl salt) □(d⁶DMSO, 400 MHz): 0.86 (3H, t), 1.14-1.30 (2H, m), 1.43-1.63 (4H, m), 1.80-1.90 (2H, m), 2.05-2.22 (4H, m), 2.24 (3H, s), 2.82-2.98 (2H, m), 3.14-3.30 (4H, m), 3.30-3.40 (2H, m), 3.44-3.54 (2H, m), 4.5-4.62 (1H, m), 6.79 (1H, d), 7.71 (1H, d), 10.86 (1H, br s), 10.95 (1H, s).

### Example 12. 6-Bromo-1-{1-[cis-4-(propyloxy)cyclohexyl]-4-piperidinyl}-5-(trifluoromethyl)-1,3-dihydro-2H-benzimidazol-2-one hydrochloride (E12)

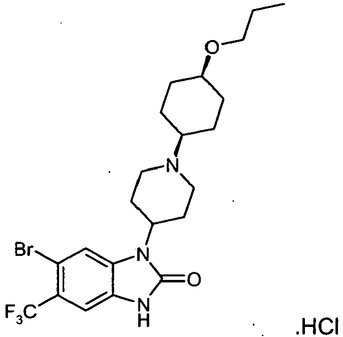

A stirred solution of 5-bromo-*N*-{1-[*cis*-4-(propyloxy)cyclohexyl]-4-piperidinyl}-4-(trifluoromethyl)1,2-benzenediamine (D38, 190mg, 0.40mmole) and diisopropylethylamine (0.107ml, 0.60mmole) in dichloromethane (15ml) at 0°C under argon was treated with solid triphosgene (47mg, 0.16mmole) and maintained at 0°C for 1.5h. The mixture was treated with water (10ml) followed by dil. NaHCO₃ solution and extracted with dichloromethane (2 x 15ml). The combined extract was dried (Na₂SO₄) and concentrated under vacuum. The residual solid was treated with ethyl acetate (15ml), stirred for 10 minutes and the remaining solid filtered off, washed with ethyl acetate and dried to afford the title compound as a white solid (124mg, 62%). This material was dissolved in dichloromethane (2ml) and methanol (0.5ml), treated with 1 M HCl/diethyl ether (0.37ml) and then concentrated under vacuum to leave the hydrochloride salt as a white solid.
¹H NMR (HCl salt) δ (d⁶DMSO, 400 MHz): 0.90 (3H, t), 1.401.58 (4H, m), 1.62-1.78 (2H, m), 1.85-2.05 (6H, m), 2.70-2.88 (2H, m), 3.16-3.40 (5H, m), 3.46-3.58 (3H, m), 4.56-4.68 (1H, m), 7.34 (1H, s), 8.08 (1H, s), 10.38 (1H, br s), 11.50 (1H, s).

### Example 13.6-Bromo-1-{1-[trans-4-(propyloxy)cyclohexyl]-4-piperidinyl}-5-(trifluoromethyl)-1,3-dihydro-2H-benzimidazol-2-one hydrochloride (E13)

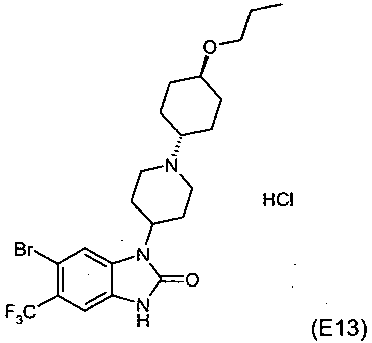

To a solution of 5-bromo-*N*-{1-[*trans*-4-(propyloxy)cyclohexyl]-4-piperidinyl}-4-(trifluoromethyl)-1,2-benzenediamine (D40, 170mg, 0.4mmole) in dichloromethane (10ml), diisopropylethylamine (0.09ml, 1.5eq., 0.5 mmole) was added at room temperature. The mixture was cooled to 0°C and bis(trichloromethyl)carbonate (40mg, 0.4eq., 0.14mmole) was added. After 30 minutes the mixture was poured onto water/brine; the aqueous solution was extracted with dichloromethane (2x) and the organics were alternatively washed with brine and water (2x). The organics were combined, dried over Na₂SO₄, filtered and the solvent was evaporated. The product was treated with HCl (3 ml of a 1M solution in diethyl ether) to yield the title product (40 mgs, 22%). M⁺ - H = 504.
¹H NMR (free base) δ (d⁶DMSO, 400MHz) 0.85 (3H, t), 1.13 (2H, q), 1.28 (2H, q), 1.46 (2H, m), 1.66 (2H, d), 1.78 (2H, d), 2.00 (2H, d), 2.29 (4H, m), 2.90 (2H, s, br), 3.12 1H, m), 3.33 (m), 4.12 (1H, m), 7.29 (1H, s), 7.69 (1H, s), 10.35 (1H, s).

### Examples 14 and 15. cis and trans 1-{1-[4-(Ethyloxy)cyclohexyl]-4-piperidinyl}-5,6-dimethyl-1,3-dihydro-2H-benzimidazol-2-one (cis = E14; trans = E15)

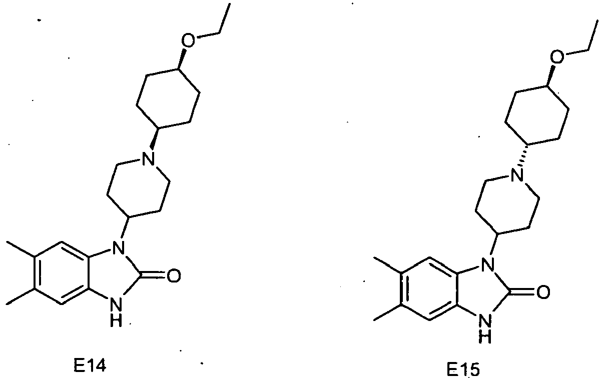

A stirred solution of containing a mixture of 5,6-dimethyl-1-(4-piperidinyl)-1,3-dihydro-2H-benzimidazol-2-one and 5,6-dimethyl-1-(1,2,3,6-tetrahydro-4-pyridinyl)-1,3-dihydro-2*H-*benzimidazol-2-one (D78, 220mg, 0.90mmole) in DCM (8ml) at room temperature under argon was treated with 4-ethoxycyclohexanone (D18, 156mg, 1.10mmole) followed by sodium triacetoxyborohydride (317mg, 1.50mmole) and maintained for 18hrs. Further 4-ethoxycyclohexanone (100mg) and sodium triacetoxyborohydride (150mg) was added and stirring maintained for 4 days. The reaction mixture was concentrated under vacuum and the residue treated with 10% Na₂CO₃ solution and extracted with EtOAc. The extract was dried and concentrated under vacuum. The residue was chromatographed on silica gel (20g) eluting with 0-10% MeOH/dichloromethane to give a yellow oil, which was dissolved in a mixture of EtOH (15ml) and acetic acid (1ml), treated with 10% Pd/C (20mg) and shaken under 50psi hydrogen pressure for 20hrs. The catalyst was removed by filtration through Kieselguhr and the filtrate concentrated under vacuum. The residue was treated with 10% Na₂CO₃ solution (10ml) and extracted with EtOAc (2x10ml). The combined extract was dried (Na₂SO₄) and concentrated under vacuum to leave a beige solid containing a mixture of cis and *trans* isomers. This was chromatographed on silica gel (40g) eluting with 10-13% MeOH/dichloromethane to afford partial separation. Fractions containing the higher rf material were combined, concentrated under vacuum and the residue crystallised from EtOAc to afford the title compound *trans* isomer (E15) as a white solid (7.7mg). MH+ 372.
¹H NMR δ (CDCl₃, 400MHz): 1.20-1.40 (4H, m), 1.21 (3H, t), 1.80-1.90 (2H, m), 1.90-2.00 (2H, m), 2.10-2.20 (2H, m), 2.25 (3H, s), 2.27 (3H, s), 2.35-2.50 (5H, m), 3.00-3.13 (2H, m), 3.15-3.25 (1H, m), 3.53 (2H, q), 4.27-4.40 (1H, m), 6.90 (1H, s), 7.09 (1H, s), 9.53 (1H, s).
Fractions containing the lower rf material were combined and concentrated under vacuum to afford the title compound cis isomer (E14) as a white solid (27mg). MH+ 372.
¹H NMR δ (CDCl₃, 400MHz): 1.20 (3H, t), 1.36-1.50 (2H, m), 1.55-2.05 (8H, m), 2.25 (3H, s), 2.28 (3H, s), 2.30-2.50 (5H, m), 3.10 (2H, br s), 3.46 (2H, q), 3.48-3.55 (1H, m), 4.35 (1H, br s), 6.90 (1H, s), 7.11 (1H, br s), 9.52 (1H, s).

### Example 16. 5-Bromo-6-methyl-1-[1-(trans-4-ethoxycyclohexyl)-4-piperidinyl]-1,3-dihydro-2H-benzimidazol-2-one hydrochloride (E16)

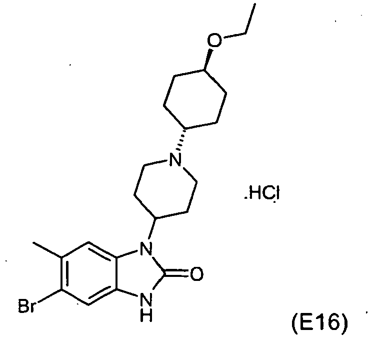

A solution of *trans*-4-bromo-5-methyl-*N*-[1-(4-ethoxycyclohexyl)-4-piperidinyl]-1,2-benzenediamine (D81, 130mg) in dichloromethane (6ml) at 0°C was treated with triphosgene (40mg) then diisopropylethylamine (0.08ml), and then maintained at 0°C for 1h. The mixture was partitioned between dichloromethane and aqueous sodium bicarbonate. Drying, evaporation and chromatography (10g silica, 0-10% methanol in dichloromethane) gave the title compound isolated as the hydrochloride salt from diethyl ether, 70mg.
**1HNMR (HCl salt) □ (d⁶DMSO):1.1** (3H, t), 1.2(2H, m), 1.55 (2H, m), 1.9 (2H, m) 2.15 (4H, m), 2.35 (3H, s), 2.8 (2H, m), 3.1-3.5 (11H, m), 4.6 (1H, m), 7.1 (1H, s), 7.7 (1H, s), 10.5 (1H, br s), 11.0 (1H, s), MH+ 436 and 438.

### Example 17. 5-Chloro-1-{1-[trans-4-(ethyloxy)cyclohexyl]-4-piperidinyl}-6-methyl-1,3-dihydro-2H-benzimidazol-2-one hydrochloride (E17)

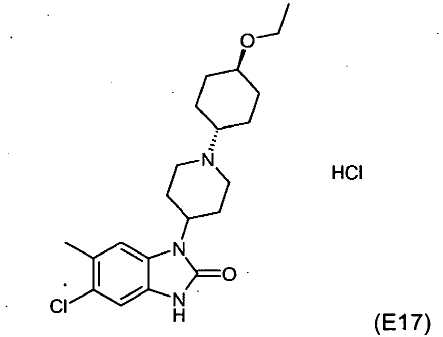

A stirred solution of 4-chloro-*N*-{1-[*trans*-4-(ethyloxy)cyclohexyl]-4-piperidinyl}-5-methyl-1,2-benzenediamine (D83, 195mg, 0.81mmole) in dichloromethane (25ml) at 0°C under argon was treated with diisopropylethylamine (0.21ml, 1.20mmole) followed by the addition of solid triphosgene (95mg, 0.32mmole) and maintained at 0°C for 45mins. The mixture was treated with water (10ml) followed by dil. NaHCO₃ solution (15ml), stirred well for 15mins, then extracted with dichloromethane (2x25ml). The combined extract was dried (Na₂SO₄) and concentrated under vacuum to leave a beige solid, which was recrystallised from a mixture of EtOAc (20ml) and MeOH (3ml) to afford the free base of the title compound as a white solid (220mg, 70%). This was dissolved in dichloromethane (4ml) and MeOH (12ml), treated with 1 M HCl/Et₂O (0.8ml) and concentrated under vacuum. The residue was triturated with Et₂O (15ml) to give a white solid which was filtered off, washed with Et₂O and dried to afford the title compound as a white solid. M⁺ + H = 392.
¹H NMR δ (d⁶DMSO, 400MHz): 1.09 (3H, t), 1.13-1.30 (2H, m), 1.48-1.62 (2H, m), 1.80-1.90 (2H, m), 2.06-2.22 (4H, m), 2.34 (3H, s), 2.83-2.96 (2H, m), 3.14-3.30 (4H, m), 3.42-3.55 (4H, m), 4.52-4.65 (1H, m), 7.00 (1H, s), 7.85 (1H, s), 10.90 (1H, br s), 10.98 (1H, s).

### Example 18. 6-Chloro-5-methyl-1-[1-(trans-4-ethoxycyclohexyl)-4-piperidinyl]-1,3-dihydro-2H-benzimidazol-2-one hydrochloride (E18)

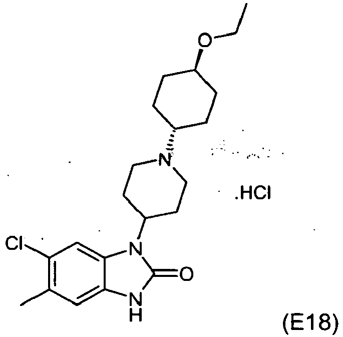

A solution of trans-5-chloro-4-methyl-*N*-[1-(4-ethoxycyclohexyl)-4-piperidinyl]-1,2-benzenediamine (D86, 95mg) in dichloromethane (3ml) at 0°C was treated with triphosgene (30mg) then diisopropylethylamine (0.1ml), and then maintained at 0°C for 30min. The mixture was partitioned between dichloromethane and aqueous sodium bicarbonate. Drying, evaporation and crystallisation from diethyl ether gave the title compound which was isolated as the hydrochloride salt from diethyl ether, 75mg.
1HNMR (HCl salt) □ (d⁶DMSO): 1.1 (3H, t), 1.2 (2H, m), 1.55 (2H, m), 1.9 (2H, m), 2.15 (4H, m), 2.35 (3H, s), 2.8 (2H, m), 3.1-3.5 (13H, m), 4.6 (1H, m), 6.9 (1H, s), 7.6 (1H, d, J=5Hz), 10.2 (1H, br s), 11.0 (1H, s), MH+ 392 and 394.

### Example 19. 6-Chloro-1-{1-[4-(ethyloxy)cyclohexyl]-4-piperidinyl}-5-methyl-1,3-dihydro-2H-benzimidazol-2-one monohydrochloride (E19)

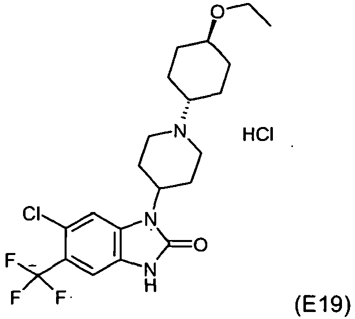

To a solution of [2-chloro-4-(trifluoromethyl)phenyl]{1-[*trans*-4-(ethyloxy)cyclohexyl]-4-piperidinyl}amine (D88, 0.30 mmol, 128 mgs) in dichloromethane (10 ml), diisopropylethylamine (1.5eq., 0.45 mmol, 0.08 ml) was added at room temperature. The mixture was cooled to 0°C and bis(trichloromethyl) carbonate (0.4eq., 0.10 mmol, 43 mgs) was added. After 60 minutes, water was added and the mixture passed through a hydrophobic filter and then purified by chromatography (MeOH-NH₃-dichloromethane) on silica column. The solvent was evaporated to yield the free base of the title compound, 48 mgs, 36%, M⁺ +H= 446. This was subsequently converted into the title compound using 2ml HCl solution (1M in Et₂O) to afford 65 mg, M⁺ + H = 446.
¹H NMR δ (CD₃OD, 400MHz, monohydrochloride salt) 1.179 (3H, t), 1.392 (2H, m), 1.655 (2H, q), 2.187 (6H, q), 2.87 (2H, q), 3.290 (5H, obs), 3.563 (2H, q), 3.67 (2H, d), 4.583 (1H, m), 7.405 (1H, s), 7.614 (1H, s).

### Example 20. 5-Bromo-6-methyl-1-[1-(trans-4-propoxycyclohexyl)-4-piperidinyl]-1,3-dihydro-2H-benzimidazol-2-one hydrochloride (E20)

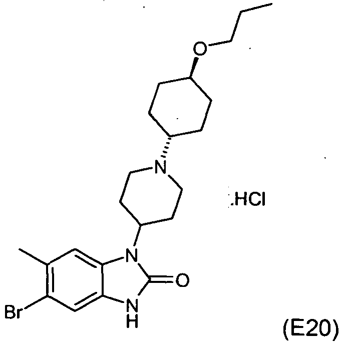

A solution of *trans*-4-bromo-5-methyl-*N*-[1-(4-propoxycyclohexyl)-4-piperidinyl]-1,2-benzenediamine (D90, 140mg) in dichloromethane (6ml) at 0°C was treated with triphosgene (35mg) then diisopropylethylamine (0.07ml), and then maintained at 0°C for 1h. The mixture was partitioned between dichloromethane and aqueous sodium bicarbonate. Drying, evaporation and chromatography (10g silica, 0-10% methanol in dichloromethane) gave the title compound isolated as the hydrochloride salt from diethyl ether, 55mg.
1HNMR (HCl salt) □ (d⁶DMSO): 0.85 (3H, t), 1.2 (2H, m), 1.5 (4H, m), 1.85 (2H, m), 2.15 (4H, m), 2.35 (3H, s), 2.8 (2H, m), 3.1-3.5 (13H, m), 4.6 (1H, m), 7.1 (1H, s), 7.7 (1H, s), 10.5 (1H, br s), 10.95 (1H, s), MH+ 450 and 452.

### Example 21. 6-Chloro-1-{1-[trans-4-(propyloxy)cyclohexyl]-4-piperidinyl}-5-(trifluoromethyl)-1,3-dihydro-2H-benzimidazol-2-one monohydrochloride (E21)

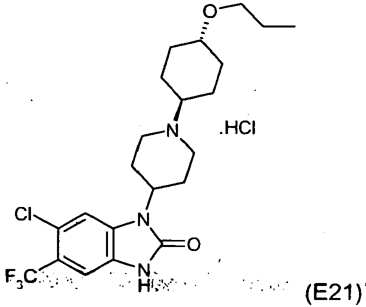

To a solution of 2-amino-5-chloro-4-(trifluoromethyl)phenyl]{1-[*trans*-4-(propyloxy)cyclohexyl]-4-piperidinyl}amine (D92, 0.40 mmol, 158 mgs) in dichloromethane (10 ml), diisopropylethylamine (1.5eq., 0.50 mmol, 0.09 ml) was added at room temperature. The mixture was cooled to 0°C and bis(trichloromethyl) carbonate (0.4eq., 0.10 mmol, 43 mgs) was added. After 30 minutes, the mixture was poured onto water/brine; the aqueous solution was extracted with dichloromethane. The solvent was evaporated to yield the free base of the title compound, 60 mgs, 36%, M⁺ + H = 460. This was subsequently converted into the title compound using 2ml HCl solution (1M in Et₂O) to afford 48 mg. M⁺ + H = 460.
¹H NMR δ (CD₃OD, 400MHz, monohydrochloride salt): 0.937 (3H, q), 1.176 (2H, t), 1.339 (4H, q), 1.595 (4H, m) 2.186 (6H, m), 2.819 (2H, q), 3.48 (3H, m), 3.665 (2H, d), 4.580 (1H, t), 7.405 (1H, s), 7.61 (1H, s)

### Example 22. 5-Fluoro-6-methyl-1-(1-{trans-4-[(1-methylethyl)oxy]cyclohexyl}-4-piperidinyl)-1,3-dihydro-2H-benzimidazol-2-one hydrochloride (E22)

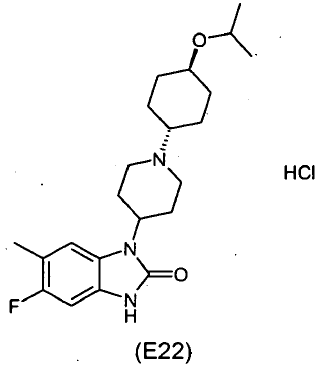

A stirred solution of 4-fluoro-5-methyl-*N*-(1-{*trans*-4-[(1-methylethyl)oxy]cyclohexyl}-4-piperidinyl)-1,2-benzenediamine (D94, 205mg, 0.56mmole) in dichloromethane (15ml) at 0°C under argon was treated with diisopropylethylamine (0.15ml, 0.84mmole) followed by the addition of solid triphosgene (65mg, 0.22mmole) and maintained for 1hr. The mixture was treated with dil. NaHCO₃ solution (20ml), stirred well for 5mins, then extracted with dichloromethane. The extract was dried (Na₂SO₄) and concentrated under vacuum to leave a beige solid, which was recrystallised from a mixture of EtOAc (15ml) and dichloromethane (5ml) to afford the free base of the title compound as a white solid (150mg, 68%). This was dissolved in dichloromethane (4ml) and MeOH (12ml), treated with 1 M HCl/Et₂O (0.55ml) and concentrated under vacuum. The residue was triturated with Et₂O (10ml) to give a white solid which was filtered off, washed with Et₂O and dried to afford the title compound as a white solid. MH⁺ = 390.
¹H NMR δ (d⁶DMSO, 400MHz): 1.07 (6H, d), 1.13-1.30 (2H, m), 1.50-1.65 (2H, m), 1.83 (2H, br d), 2.00 (2H, br d), 2.17 (2H, br d), 2.23 (3H, s), 2.85-3.00 (2H, m), 3.15-3.40 (4H, m), 3.49 (2H, br d), 3.63-3.77 (1H, m), 4.52-4.66 (1H, m), 6.79 (1H, d), 7.78 (1H, d), 10.95 (1H, s), 11.00 (1H, br m).

### Example 23. 5-Fluoro-6-methyl-1-{1-[trans-1-methyl-4-(methyloxy)cyclohexyl]-4-piperidinyl}-1,3-dihydro-2H-benzimidazol-2-one monohydrochloride (E23)

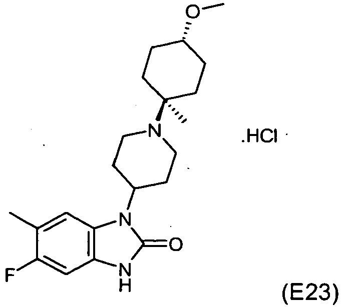

To a solution of (2-amino-4-fluoro-5-methylphenyl){1-[*trans*-1-methyl-4-(methyloxy)cyclohexyl]4-piperidinyl}amine (D96, 0.189 mmol, 66 mgs) in dichloromethane (5 ml), diisopropylethylamine (1.5eq., 0.284 mmol, 0.05 ml) was added at room temperature. The mixture was cooled to 0°C and bis(trichloromethyl) carbonate (0.4eq., 0.08 mmol, 22 mgs) was added. After 60 minutes, the mixture was quenched with brine and basified with 2M NaOH to Ph 10. The aqueous solution was extracted with EtOAc (2x); The organics were combined dried over Na₂SO₄, filtered and the solvent was evaporated to afford the crude product. The crude product was purified by chromatography (MeOH-NH₃-dichloromethane) on silica column to afford the free base of the title compound, 31 mgs, 40%, M⁺ + H = 376. This was subsequently converted into the title compound using 1ml of HCl solution (1 M in Et₂O) to afford 16 mg.
¹H NMR δ (d⁶DMSO, 400MHz) 1.283 (5H, m), 1.939 (8H, m), 2.242 (3H, d), 2.886 (2H, q), 3.097 (1H, m), 3.163 (3H, m) 3.259 (3H, s), 3.645 (2H, d), 4.078 (1H, m), 4.585 (1H, m), 6.795 (1H, d), 7.682 (1H, d), 10.2 (1H, s br), 10.9 (1H, s).

### Example 24. 1-{1-[trans-4-(Ethyloxy)-1-methylcyclohexyl]-4-piperidinyl}-5-fluoro-6-methyl-1,3-dihydro-2H-benzimidazol-2-one hydrochloride (E24)

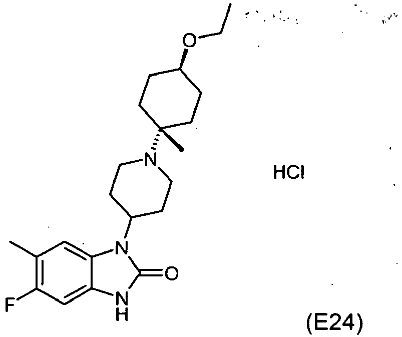

A stirred solution of *N*-{1-[*trans*-4-(ethyloxy)-1-methylcyclohexyl]-4-piperidinyl}-4-fluoro-5-methyl-1,2-benzenediamine (D98, 90mg, 0.248mmole) in dichloromethane (10ml) at 0°C under argon was treated with diisopropylethylamine (0.066ml, 0.372mmole) followed by the addition of solid triphosgene (29mg, 0.099mmole) and maintained for 2hr. The mixture was treated with dil. NaHCO₃ solution (10ml) and extracted with dichloromethane (2x10ml). The extract was dried (Na₂SO₄), concentrated under vacuum and the residue crystallised from 1:1 Et₂O/EtOA to afford the free base of the title compound as a white solid (77mg, 80%). This was dissolved in DCM (3ml) and MeOH (4ml), treated with 1M HCl/Et₂O (0.30ml) and concentrated under vacuum. The residue was triturated with Et₂O to give a solid which was filtered off, washed with Et₂O and dried to afford the title compound as a white solid (59mg). MH⁺ = 390.
¹H NMR δ (d⁶DMSO, 400MHz): 1.10 (3H, t), 1.24-1.40 (2H, m), 1.33 (3H, s), 1.85 (2H, br d), 1.90-2.05 (assume 5H, m), 2.40 (3H, s), 2.88-3.02 (2H, m), 3.10-3.26 (4H, m), 3.47 (2H, q), 3.64 (2H, br d), 4.54-4.67 (1H, m), 6.79 (1H, d), 7.77 (1H, d), 10.45 (1H, m), 10.94 (1H, s).

### Example 25. 5-Chloro-1-{1-[trans-4-(ethyloxy)-1-methylcyclohexyl]-4-piperidinyl}-6-methyl-1,3-dihydro-2H-benzimidazol-2-one hydrochloride (E25)

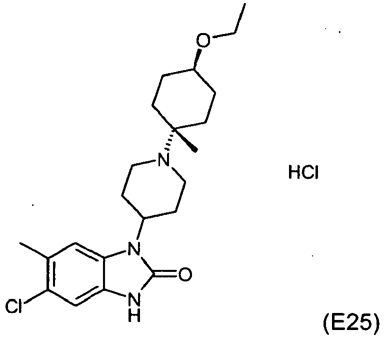

A stirred solution of 4-chloro-*N*-{1-[*trans*-4-(ethyloxy)-1-methylcyclohexyl]-4-piperidinyl}-5-methyl-1,2-benzenediamine (D100, 195mg, 0.51mmole) in dichloromethane (12ml) at 0°C under argon was treated with diisopropylethylamine (0.136ml, 0.76mmole) followed by the addition of solid triphosgene (60mg, 0.20mmole) and maintained for 40mins. The mixture was treated with dil. NaHCO₃ solution (10ml) and extracted with dichloromethane (2x15ml). The combined extract was dried (Na₂SO₄) and concentrated under vacuum to leave a solid which was recrystallised from EtOAc/dichloromethane to afford the free base of the title compound as a white solid (60mg, 29%). This was dissolved in dichloromethane (2ml) and MeOH (2ml), treated with 1 M HCl/Et₂O (0.25ml) and concentrated under vacuum. The residue was triturated with Et₂O to give a solid which was filtered off, washed with Et₂O and dried to afford the title compound as a white solid (65mg). MH⁺ = 406.
¹H NMR δ (d⁶DMSO, 400MHz): 1.10 (3H, t), 1.22-1.40 (5H, m), 1.82-2.05 (assume 7H, m), 2.34 (3H, s), 2.80-2.97 (2H, m), 3.12-3.25 (3H, m), 3.30-3.42 (assume 1H, m), 3.47 (2H, q), 3.65 (2H, br d), 4.52-4.65 (1H, m), 6.70 (1H, s), 7.79 (1H, s), 10.20 (1H, br m), 10.96 (1H, s).

### Example 26. 5-Fluoro-6-methyl-1-{1-[cis-1-methyl-4-(propyloxy)cyclohexyl]-4-piperidinyl}-1,3-dihydro-2H-benzimidazol-2-one hydrochloride (E26)

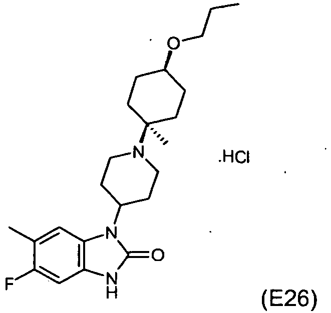

Triphoshene (0.047g) was added to a solution of 4-fluoro-5-methyl-*N*-{1-[*cis*-1-methyl-4-(propyloxy)cyclohexyl]-4-piperidinyl}-1,2-benzenediamine (D102, 0.15g,), dichloromethane (10ml) and diisopropylethylamine (0.2ml) stirred at ice bath temperature. The solution was stirred to room temperature overnight then washed with saturated sodium bicarbonate solution, brine and the solvent was removed. The residue was chromatographed on silica gel eluted with dichloromethane - methanolic ammonia 0-5%, to give the free base of the title as a white solid. This was dissolved in dichloromethane, treated with hydrogen chloride in ether and the solvent was removed to give the title compound as a white solid (0.12g). MH⁺ = 404.
¹H NMR □ (d⁶DMSO, 400 MHz): 0.90 (3H, t), 1.35 (3H, s), 1.55 (4H, m), 1.73 (2H, m), 1.85-2.0 (7H, m), 2.25 (3H, s), 2.8 (2H, m), 3.25 (obs, m), 3.46 (2H, m), 4.56 (1H, m), 6.81 (1H, d), 7.50 (1H, m) 9.63 (1H, m), 10.92 (1H, s).
¹⁹F NMR □ (d⁶DMSO) -125.67ppm.

### Example 27. 5-Fluoro-6-methyl-1-{1-[trans-1-methyl-4-(propyloxy)cyclohexyl]-4-piperidinyl}-1,3-dihydro-2H-benzimidazol-2-one hydrochloride (E27)

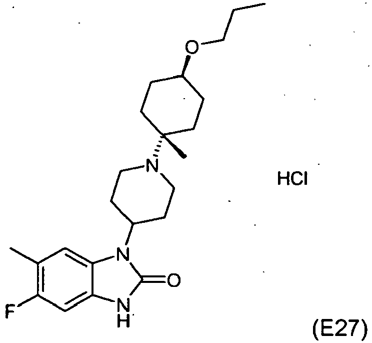

A stirred mixture of 4-fluoro-5-methyl-*N*-{1-[*trans*-1-methyl-4-(propyloxy)cyclohexyl]-4-piperidinyl}-1,2-benzenediamine (D104, 280mg, 0.74mmole) in dichloromethane (20ml) was treated with diisopropylethylamine (0.4ml, 2.2mmole) and portionwise addition of triphosgene (88mg, 0.30mmole) at 0°C under argon for 15 minutes. The mixture was allowed to warm to room temperature, then washed with sat. NaHCO₃ solution and extracted with dichloromethane. The extract was dried (Na₂SO₄) and concentrated under vacuum, then crystallised from pentane and collected by filtration to afford the free base of the title compound (120mg, 40%). This was dissolved in dichloromethane (10ml), treated with 1 M HCl/Et₂O (5ml) and concentrated under vacuum to afford the title compound as a white solid (123mg). MH⁺= 404 ¹H NMR δ (d⁶DMSO, 400MHz): 0.87 (3H, t), 1.20-1.40 (2H, m), 1.33 (3H, s), 1.40-1.57 (2H, m), 1.86 (2H, br d), 1.90-2.07 (5H, m), 2.24 (3H, s), 2.82-3.00 (2H, m), 3.10-3.37 (3H, m), 3.30-3.45 (assume 3H, m), 3.62 (2H, br d), 4.53-4.65 (1H, m), 6.79 (1H, d), 7.75 (1H, d), 10.4 (1H, br m), 10.92 (1H, s).

### Example 28. 5-Chloro-6-methyl-1-{1-[cis-1-methyl-4-(propyloxy)cyclohexyl]-4-piperidinyl}-1,3-dihydro-2H-benzimidazol-2-one hydrochloride (E28)

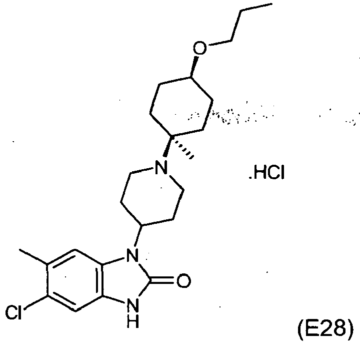

Triphoshene (0.05g) was added to a solution of 4-fluoro-5-methyl-*N*-{1-[*cis*-1-methyl-4-(propyloxy)cyclohexyl]-4-piperidinyl}-1,2-benzenediamine (D106, 0.167g,), dichloromethane (10ml) and diisopropylethylamine (0.2ml) stirred at ice bath temperature. The solution was stirred to room temperature overnight then washed with saturated sodium bicarbonate solution, brine and the solvent was removed. The residue was chromatographed on silica gel eluted with dichloromethane - methanolic ammonia 0-5%. The product was dissolved in dichloromethane, treated with hydrogen chloride in ether and the solvent was removed to give the title compound as a white solid (0.102g). MH⁺ = 420.
¹H NMR □ (d⁶DMSO, 400 MHz): 0.90 (3H, t), 1.36 (3H , s), 1.55 (4H, m), 1.70 (2H, m), 1.85-2.2 (7H, m), 2.34 (3H, s), 2.9 (2H, m), 3.2 (2H, m), 3.35 (obs, m), 3.49 (1H, m), 3.67 (2H, m), 4.59 (1H, m), 7.0 (1H, s), 7.76 (1H, s) 10.0 (1H, m), 11.0 (1H, s).

### Example 29. 5-Chloro-6-methyl-1-{1-[trans-1-methyl-4-(propyloxy)cyclohexyl]-4-piperidinyl}-1,3-dihydro-2H-benzimidazol-2-one (E29)

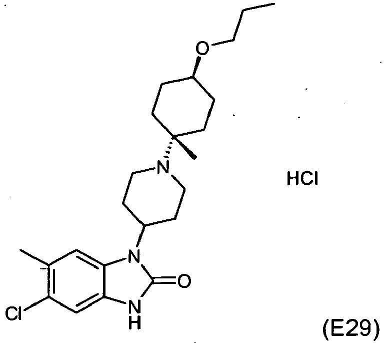

A stirred mixture of 4-chloro-5-methyl-*N*-{1-[*trans*-1-methyl-4-(propyloxy)cyclohexyl]-4-piperidinyl}-1,2-benzenediamine (D108, 300mg, 0.76mmole) in dichloromethane (25ml) was treated with diisopropylethylamine (0.4ml, 2.2mmole) and portionwise addition of triphosgene (90mg, 0.30mmole) at 0°C under argon for 15 minutes. The mixture was allowed to warm to room temperature, then washed with sat. NaHCO₃ solution and extracted with dichloromethane. The extract was dried (Na₂SO₄) and concentrated under vacuum, then crystallised from pentane and collected by filtration to afford the free base of the title compound (175mg, 55%). This was dissolved in dichloromethane (10ml), treated with 1 M HCl/Et₂O (5ml) and concentrated under vacuum to afford the title compound as a white solid (181 mg). MH⁺=420.
¹H NMR δ (d⁶DMSO, 400MHz): 0.87 (3H, t), 1.22-1.40 (5H, m), 1.42-1.55 (2H, m), 1.85 (2H, br d), 1.85-2.06 (5H, m), 2.34 (3H, s), 2.85-3.00 (2H, m), 3.10-3.25 (3H, m), 3.30-3.45 (assume 3H, m), 3.65 (2H, br d), 4.55-4.70 (1H, m), 6.99 (1H, s), 7.88 (1H, s), 10.45 (1H, br m), 10.96 (1H, s).

### Example 30. 6-Bromo-1-{1-[trans-1-methyl-4-(propyloxy)cyclohexyl]-4-piperidinyl}-5-(trifluoromethyl)-1,3-dihydro-2H-benzimidazol-2-one hydrochloride (E30)

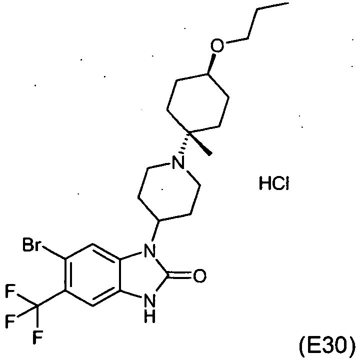

A stirred mixture of 5-bromo-*N*-{1-[*trans*-1-methyl-4-(propyloxy)cyclohexyl]-4-piperidinyl}-4-(trifluoromethyl)-1,2-benzenediamine (D110, 420mg, 0.85mmole) in dichloromethane (40ml) was treated with diisopropylethylamine (0.45ml, 2.55mmole) and portionwise addition of triphosgene (101mg, 0.34mmole) at 0°C under argon for 10 minutes. The mixture was allowed to warm to room temperature, then washed with NaHCO₃ solution and extracted with dichloromethane. The extract was dried (Na₂SO₄) and concentrated under vacuum, then crystallised from pentane and collected by filtration to afford the free base of the title compound (290mg, 66%). This was dissolved in dichloromethane (10ml), treated with 1 M HCl/Et₂O (5ml) and concentrated under vacuum to afford the title compound as a white solid (180mg). MH⁺ = 520.
¹H NMR δ (d⁶DMSO, 400MHz): 0.86 (3H, t), 1.20-1.40 (5H, s + m), 1.42-1.54 (2H, m), 1.85-2.07 (8H, m), 2.83-3.00 (2H, m), 3.10-3.28 (3H, m), 3.30-3.43 (assume 2H), 3.67 (2H, br d), 4.61-4.74 (1H, m), 7.34 (1H, s), 8.27 (1H, s), 10.6 (1H, br m), 11.54 (1H, s).

### Example 31. 1-{1-[trans-4-(Ethyloxy)-1-methylcyclohexyl]-4-piperidinyl}-5,6-dimethyl-1,3-dihydro-2H-benzimidazol-2-one hydrochloride (E31)

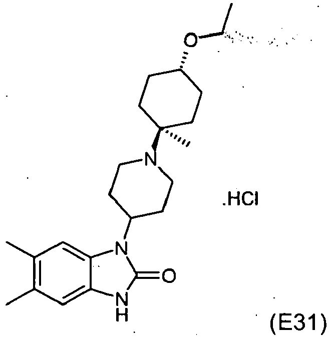

(2-Amino-4,5-dimethylphenyl){1-[*trans*-4-(ethyloxy)-1-methylcyclohexyl]-4-piperidinyl}amine (D120, 30mg, 0.084mmol) stirred under argon in tetrahydrofuran (5ml) at ice bath temperature was treated with diisopropylethylamine (89µl, 0.42mmol), followed by addition of triphosgene (12.5mg, 0.042mmol). The mixture was allowed to warm to room temperature, and then stirred under argon at this temperature over the weekend. A solid formed in the solution. Minimal methanol was added to dissolve the solid, and the solution was loaded onto a 2g SCX cartridge. The product was eluted with NH₃ in methanol (2M) and the solvent was then removed. The resulting solid was dissolved in a small amount of ethanol, and then concentrated under vacuum to give orange crystals (25mg). The crystals were dissolved in dichloromethane (≈2ml) and 1 M hydrogen chloride in diethyl ether (0.5ml) was added. The solvent was then removed, and the resulting solid dried in a vacuum oven giving the title compound as a brown solid (20mg). MH⁺ = 386.
¹HNMR δ DMSO, 400MHz): 1.1 (3H, t), 1.2-1.4 (obs), 1.827 (2H, d), 1.91-2.05 (obs), 2.19 (3H, s), 2.23 (3h, s), 2.92 (2H, q), 3.15-3.3 (3H, m), 3.47 (2H, q), 3.63(2H, d), 4.54-4.6 (1H, m), 6.76 (1H, s), 7.612 (1H, s), 10.4 (1H, m), 10.7 (1H, s).

### Example 32. 1-{1-[trans-4-(Ethyloxy)-1-methylcyclohexyl]-4-piperidinyl}-5,6-difluoro-1,3-dihydro-2H-benzimidazol-2-one hydrochloride (E32)

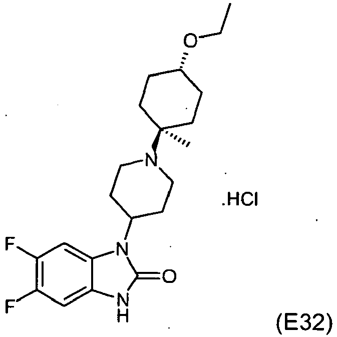

*N*-{1-[*trans*-4-(Ethyloxy)-1-methylcyclohexyl]-4-piperidinyl}-4,5-difluoro-1,2-benzenediamine (D121, 650mg, 1.7mmol) stirred under argon in dichloromethane at ice bath temperature was treated with diisopropylethylamine (1ml, 4.7mmol), followed by addition of triphosgene (155mg, 0.52mmol). The mixture was allowed to warm to room temperature, and then stirred under argon at this temperature overnight. Solvent removed to give dark brown crystals (600mg). These cyrstals were purified using a Waters Xbridge chromatography column eluting with a gradient using aqueous ammonium bicarbonate (10mmolar) adjusted to pH10 with ammonia and acetonitrile as the mobile phase to give the pure product. Once obtained, this was dissolved in dichloromethane and 1 M hydrogen chloride in diethyl ether was added. The solvent was then blown off and the product was triturated in diethyl ether. The solvent was removed and the resulting solid was dried to give the title compound as a fawn coloured solid (48mg). MH⁺ = 394.
¹HNMR δ DMSO, 400MHz): 1.09 (assume 3H, m), 1.23-1.36 (obs), 1.86-2.0 (obs), 2.86 (2H, q), 3.17 (3H, m), 3.47 (2H, q), 3.65 (2H, d), 4.62 (1H, m), 7.1 (1H, m), 7.96 (1H, m), 10.35 (1H, m), 11.15 (1H, s).

### Example 33. 5-Chloro-1-{1-[trans-1-methyl-4-(methyloxy)cyclohexyl]-4-piperidinyl}-1,3-dihydro-2H-benzimidazol-2-one hydrochloride (E33)

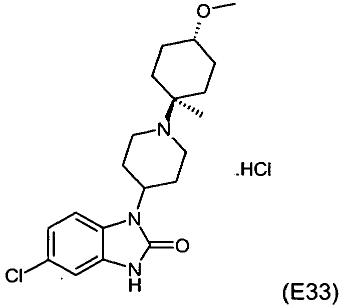

Diisopropylethylamine (120µL, 0.71mmole) was added to a solution of (2-amino-4-chlorophenyl){1-[*trans*-1-methyl-4-(methyloxy)cyclohexyl]-4-piperidinyl}amine (D123, 84mg, 0.24mmole) in CH₂Cl₂ (10mL) at room temperature under argon. The reaction was cooled to 0°C and triphosgene (28mg, 0.09mmole) was added portion-wise. The reaction was stirred overnight before the addition of 2M NaOH (10mL). The mixture was partitioned and the aqueous layer extracted with CH₂Cl₂ (2x). The combined organics were dried (Na₂SO₄) and reduced in volume by rotary evaporation before being chromatographed (silica, CH₂Cl₂-0.5% NH₃ /9.5% MeOH /90% CH₂Cl₂) to give the free base of the title **compound as a yellow solid. The free base was suspended in** MeOH (2mL) and then 1 M HCl in Et₂O (0.3mL) was added. The mixture was stirred for 30min and then filtered, with the precipitate being washed with Et₂O (2x) to give the title compound (51 mg, 52%) as a beige solid. M(CI-35)⁺ 378, M(CI-37)H⁺ 380.
¹H NMR δ (DMSO-*d*₆, 400 MHz): 1.24-1.39 (5 H, m), 1.83-2.08 (8 H, m), 2.86 (2 H, m), 3.05-3.21 (3 H, m), 3.21 (3 H, s), 3.64 (2 H, m), 4.62 (1H, m), 7.04 (2 H, m), 7.73 (1H, d, *J* 9), 10.24 (1 H, m), 11.12 (1H, s).

### Example 34. 5-Chloro-1-{1-[trans-4-(ethyloxy)-1-methylcyclohexyl]-4-piperidinyl}-1,3-dihydro-2H-benzimidazol-2-one hydrochloride (E34)

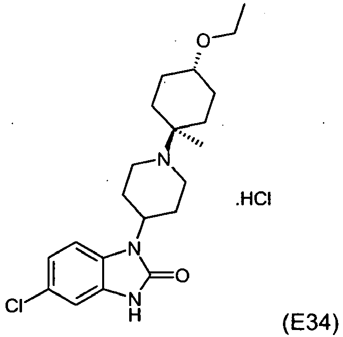

(2-Amino-4-chlorophenyl){1-[*trans*-4-(ethyloxy)-1-methylcyclohexyl]-4-piperidinyl}amine (D125, 102mg, 0.28mmole) in CH₂Cl₂ (10mL) at room temperature under argon. The reaction was cooled to 0°C and triphosgene (33mg, 0.11mmole) was added portion-wise. The reaction was stirred overnight before the addition of 2M NaOH (10mL). The mixture was partitioned and the aqueous layer extracted with CH₂Cl₂ (2x). The combined organics were dried (Na₂SO₄) and reduced in volume by rotary evaporation before being chromatographed (silica, CH₂Cl₂-0.5% NH₃ /9.5% MeOH /90% CH₂Cl₂) to give the free base of the title compound as a yellow solid. The free base was suspended in MeOH (2mL) and then 1M HCl in Et₂O (0.5mL) was added. The mixture was stirred for 2h and then filtered, with the precipitate being washed with Et₂O (2x) to give the title compound (77mg, 64%) as a cream solid. M(CI-35)⁺ 392, M(CI-37)H⁺ 394.
¹H NMR δ (DMSO-*d*₆, 400 MHz): 1.10 (3 H, t, *J* 7), 1.26-1.39 (5 H, m), 1.79 (2 H, m), 1.89-2.05 (6 H, m), 2.75 (2 H, m), 3.14-3.25 (3 H, m), 3.47 (2 H, q, *J* 7), 3.65 (2 H, m), 4.60 (1H, m), 7.03 (1H, d, *J* 2), 7.08 (1H, m), 7.52 (1H, d, *J* 8), 9.54 (1H, m), 11.12 (1 H, s).

### Example 35. 6-Chloro-3-{1-[trans-4-(ethyloxy)cyclohexyl]-4-piperidinyl}-2-oxo-2,3-dihydro-1H-benzimidazole-5-carbonitrile (E35)

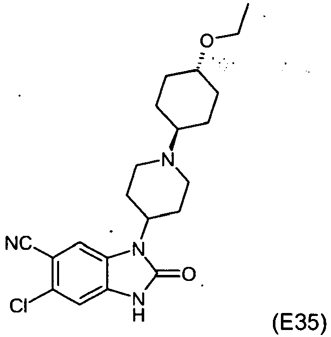

A solution of 1,1'-bis(diphenylphosphino)ferrocene (6.4mg, 0.0116mmole) in dioxane (5ml) at room temperature under argon was treated with *N*-(5-chloro-4-cyano-2-iodophenyl)-*N*'-{1-[*trans*-4-(ethyloxy)cyclohexyl]-4-piperidinyl}urea (D126, 103mg, 0.194mmole) and stirred for 10 minutes, then sodium t-butoxide (56mg, 0.582mmole) was added and the mixture heated at 95°C for total of 20 hours. The mixture was cooled, concentrated under vacuum and the residue treated with 10% Na₂CO₃ solution and extracted with dichloromethane. The extract was dried (Na₂SO₄) and concentrated under vacuum. The residue was initially purified by passage through an SCX cartridge, then further purified by MDAP to afford the title compound as a white solid (5mg). MH⁺ 403.
¹H NMR δ (CDCl₃, 400MHz): 1.21 (3H, t), 1.20-1.42 (4H, m), 1.84 (2H, brd), 1.95 (2H, br d), 2.14 (2H, br d), 2.26-2.50 (5H, m), 3.10 (2H, br d), 3.15-3.26 (1H, m), 3.52 (2H, q), 4.22-4.37 (1H, m), 7.23 (1H, s), 7.56 (1H, s), 10.30 (1H, br s).

### Example 36. 5,6-Dimethyl-1-{1-(trans-1-methyl-4-(methyloxy)cyclohexyl]-4-piperidinyl}-1,3-dihydro-2H-benzimidazol-2-one hydrochloride (E36)

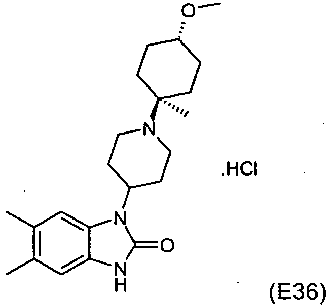

The title compound can be prepared from 4,5-dimethyl-1,2-benzenediamine and 1-[*trans-*1-methyl-4-(methyloxy)cyclohexyl]-4-piperidinone (D58) using a similar procedure to that described in Description 120 and Example 31.

### Example 37. 5,6-Diftuoro-1-{1-[trans-1-methyl-4-(methyloxy)cyclohexyl]-4-piperidinyl}-1,3-dihydro-2H-benzimidazol-2-one hydrochloride (E37)

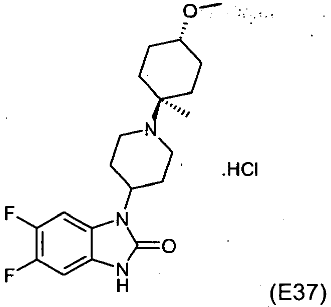

The title compound can be prepared from 4,5-difluoro-1,2-benzenediamine and 1-[*trans*-1-methyl-4-(methyloxy)cyclohexyl]-4-piperidinone (D58) using a similar procedure to that described in Description 121 and Example 32.

### Example 38. 6-chloro-3-{1-[trans-1-methyl-4-(methyloxy)cyclohexyl]-4-piperidinyl}-2-oxo-2,3-dihydro-1H-benzimidazole-5-carbonitrile (E38)

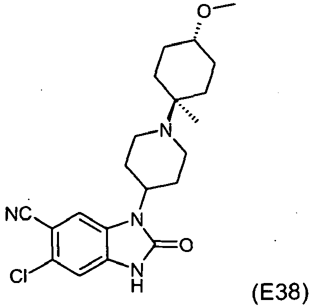

The title compound can be prepared from 4-amino-2-chloro-5-iodobenzonitrile (J. Med. Chem. 2001, 44 (23), 3866) and 1-[*trans*-1-methyl-4-(methyloxy)cyclohexyl]-4-piperidinamine (D59) using a similar procedure to that described in Description 126 and Example 35.

### Example 39. 6-Chloro-3-{1-[trans-4-(ethyloxy)-1-methylcyclohexyl]-4-piperidinyl}-2-oxo-2,3-dihydro-1H-benzimidazole-5-carbonitrile (E39)

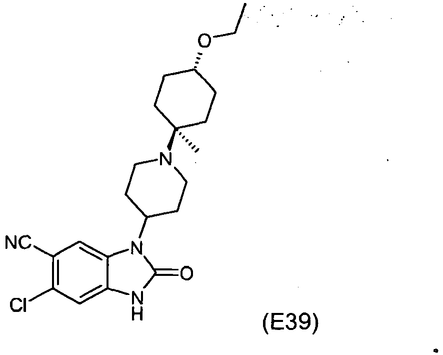

The title compound can be prepared from 4-amino-2-chloro-5-iodobenzonitrile (J. Med. Chem. 2001, 44 (23), 3866) and 1-[*trans*-4-(ethyloxy)-1-methylcyclohexyl]-4-piperidinamine (D68) using a similar procedure to that described in Description 126 and Example 35.

## Claims

1. A compound of formula (I) or a salt or solvate thereof: 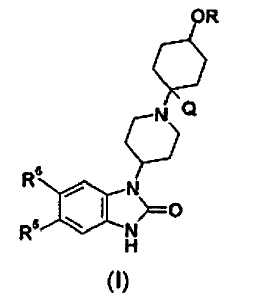 wherein:
- R⁵ is selected from halogen, cyano, C₁₋₆alkyl, C₁₋₆alkyl substituted with one or more fluorine atoms, C₁₋₆alkoxy, and C₁₋₆alkoxy substituted with one or more fluorine atoms;
- R⁸ is selected from hydrogen, halogen, cyano, C₁₋₆alkyl, C₁₋₆alkyl substituted with one or more fluorine atoms, C₁₋₆alkylsulfonyl, C₃₋₆cycloalkyl, C₃₋₆cycloalkyl substituted with one or more fluorine atoms, C₁₋₆alkoxy and C₁₋₆alkoxy substituted with one or more fluorine atoms;
- R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted with one or more fluorine atoms; and
Q is hydrogen or C₁₋₆alkyl.

2. A compound as claimed in claim 1, wherein R⁵ is selected from halogen, cyano, C₁₋₄alkyl, C₁₋₄alkyl substituted with one, two or three fluorine atoms, C₁₋₄alkoxy, and C₁₋₄alkoxy substituted with one, two or three fluorine atoms.

3. A compound as claimed in claim 1 or claim 2, wherein R⁶ is selected from hydrogen, halogen, cyano, C₁₋₄alkyl, C₁₋₄alkyl substituted with one, two or three fluorine atoms, C₁₋₄alkylsulfonyl, C₃₋₆cycloafkyl, C₃₋₆cycloalkyl substituted with one or more fluorine atoms, C₁₋₄alkoxy and C₁₋₄alkoxy substituted with one, two or three fluorine atoms.

4. A compound as claimed in claim 1, 2 or 3, wherein R is selected from C₁₋₄alkyl, C₃₋₆ cycloalkyl, C₃₋₆cycloalkylC₁₋₄alkyl and C₂₋₄alkynyl, any alkyl or cycloalkyl group being optionally substituted with one, two or three fluorine atoms.

5. A compound as claimed in any of claims 1-4, wherein Q is selected from hydrogen, methyl, ethyl and propyl.

6. A compound as claimed in claim 1 which is selected from
1. 5-Fluoro-6-methyl-1-{1-[*cis-*4-(methyloxy)cyclohexyl]-4-piperidinyl}-1,3-dihydro-2*H-*benzimidazol-2-one
2. 5-Fluoro-6-methyl-1-{1-[*trans*-4-(methyloxy)cyclohexyl]-4-piperidinyl}-1,3-dihydro-2*H-*benzimidazol-2-one
3. 5-Chloro-6-methyl-1-{1-[*cis*-4-(methyloxy)cyclohexyl]-4-piperidinyl}-1,3-dihydro-2*H-*benzimidazol-2-one
4. 5-Chloro-6-methyl-1-{1-[*trans*-4-(methyloxy)cyclohexyl]-4-piperidinyl}-1,3-dihydro-2*H-*benzimidazol-2-one
5. 6-Bromo-1-{1-[*cis-*4-(methyloxy)cyclohexyl]-4-piperidinyl}-5-(trifluorcmethyl)-1,3-dihydro-2*H*-benzimidazol-2-one
6. 6-Bromo-1-{1-[*trans*-4-(methyloxy)cyclohexyl]-4-piperidinyl}-5-(trifluoromethyl)-1,3-dihydro-2*H*-benzimidazol-2-one
7. 1-{1-[*trans*-4-(Ethyloxy)cyclohexyl]-4-piperidinyl}-5-fluoro-6-methyl-1,3-dihydro-2*H-*benzimidazol-2-one
8. 5-Chloro-6-methyl-1-{1-[*cis*-4-(propyloxy)cyclohexyl]-4-piperidinyl}-1,3-dihydro-2*H-*benzimidazol-2-one
9. 5-Chloro-6-methyl-1-{1-[*trans*-4-(propyloxy)cyclohexyl]-4-piperidinyl}-1, 3-dihydro-2*H-*benzimidazol-2-one
10. 5-Fluoro-6-methyl-1-{1-[*cis*-4-(propyloxy)cyclohexyl]-4-piperidlnyl}-1,3-dihydro-2*H-*benzimidazol-2-one
11. 5-Fluoro-6-methyl-1-{1[*trans*-4-(propyloxy)cyclohexyll-4-piperidinyl}-1,3-dihydro-2*H-*benzimidazol-2-one
12. 6-Bromo-1-{1-[*cis*-4-(propyloxy)cyclohexyl]-4-piperidinyl}-5-(trifluoromethyl)-1,3-dihydro-2*H*-benzimidazol-2-one
13. 6-Bromo-1-{1-[*trans*-4-(propyloxy)cyclohexyl]-4-piperidinyl}-5-(trifluoromethyl)-1,3-dihydro-2*H*-benzimidazol-2-one
14. *cis* 1-{1-[4-(Ethyloxy)cyclohexyl]-4-piperidinyl}-5,6-dimethyl-1,3-dihydro-2*H-*benzimidazol-2-one
15. *trans* 1-{1-[4-(Ethyloxy)cyclohexyl]-4-piperidinyl}-5,6-dimethyl-1,3-dihydro-2*H-*benzimidazol-2-one
16. 5-Bromo-6-methyl-1-[1-(*trans*-4-ethoxycyclohexyl)-4-piperidinyl]-1,3-dihydro-2*H-*benzimidazol-2-one
17.5-Chloro-1-{1-[*trans*-4-(ethyloxy)cyclohexyl]-4-piperidinyl}-6-methyl-1,3-dihydro-2*H-*benzimidazol-2-one
18.6-Chloro-5-methyl-1-[1-(*trans*-4-ethoxycyclohexyl)-4-piperidinyl]-1,3-dihydro-2*H-*benzimidazol-2-one
19. 6-Chloro-1-{1-[4-(ethyloxy)cyclohexyl]-4-piperidinyl}-5-methyl-1,3-dihydro-2*H-*benzimidazol-2-one
20. 5-Bromo-6-methyl-1-[1-(*trans*-4-propoxycyclohexyl)-4-piperidinyl]-1,3-dihydro-2*H-*benzimidazol-2-one
21.6-Chloro-1-{1-[*trans*-4-(propyloxy)cyclohexyl]-4-piperidinyl}-5-(trifluoromethyl)-1,3-dihydro-2*H*-benzimidazol-2-one
22. 5-Fluoro-6-methyl-1-(1-{*trans*-4-[(1-methylethyl)oxy]cyclohexyl}-4-piperidinyl)-1,3-dihydro-2*H*-benzimidazol-2-one
23. 5-Fluoro-6-methyl-1-{1-[*trans*-1-methyl-4-(methyloxy)cyclohexyl]-4-piperidinyl}-1,3-dihydro-2*H*-benzimidazol-2-one
24. 1-{1-[*trans*-4-(Ethyloxy)-1-methylcyclohexyl]-r4-piperidinyl}-5-fluoro-6-methyl-1,3-dihydro-2*H*-benzimidazol-2-one
25.5-Chloro-1-{1-[*trans*-4-(ethyloxy)-1-methylcyclohexyl]-4-piperidinyl}-6-methyl-1,3-dihydro-2*H*-benzimidazol-2-one
26. 5-Fluoro-6-methyl-1-{1-[*cis*-1-methyl-4-(propyloxy)cyclohexyl]-4-piperidinyl}-1,3-dihydro-2*H-*benzimidazol-2-one
27. 5-Fluoro-6-methyl-1-{1-[*trans*-1-methyl-4-(propyloxy)cyclohexyl]-4-piperidinyl}-1,3-dihydro-2*H*-benzimidazol-2-one hydrochloride
28. 5-Chloro-6-methyl-1-{1-[*cis*-1-methyl-4-(propyloxy)cyclohexyl]-4-piperidinyl}-1,3-dihydro-2*H*-benzlmidazol-2-ons
29. 5-Chloro-6-methyl-1-{1-[*trans*-1-methyl-4-(propyloxy)cyclohexyl]-4-piperidinyl}-1,3-dihydro-2*H*-benzimidazol-2-one
30. 6-Bromo-1-{1-[*trans*-1-methyl-4-(propyloxy)cyclohexyl]-4-piperidinyl}-5-(trifiuoromethyl)-1,3-dihydro-2*H*-benzimidazol-2-one
31. 1-{1-[*trans*-4-(Ethyloxy)-1-methylcyclohexyl]-4-piperidinyl}-5,6-dimethyl-1,3-dihydro-2*H-*benzimidazol-2-one
32. 1-{1-[*trans*-4-(Ethyloxy)-1-methylcyclohexyl]-4-piperidinyl}-5,6-difluoro-1,3-dihydro-2*H-*benzimidazol-2-one
33. 5-Chloro-1-{1-[*trans*-1-methyl-4-(methyloxy)cyclohexyl]-4-piperidinyl}-1,3-dihydro-2*H-*benzimidazol-2-one
34. 5-Chloro-1-{1-[*trans*-4-(ethyloxy)-1-methylcyclohexyl]-4-piperidinyl}-1,3-dihydro-2*H-*benzimidazol-2-one
35. 6-Chloro-3-{1-[*trans*-4-(ethyloxy)cyclohexyl]-4-piperidinyl}-2-oxo-2,3-dihydro-1H-benzimidazole-5-carbonitrile
36. 5,6-Dimethyl-1-{1-[*trans*-1-methyl-4-(methyloxy)cyclohexyl]-4-piperidinyl}-1,3-dihydro-2*H-*benzimidazol-2-one
37. 5,6-Difluoro-1-{1-[*trans*-1-methyl-4-(methyloxy)cyclohexyl]-4-piperidinyl}-1,3-dihydro-2*H-*benzimidazol-2-one
38. 6-chloro-3-{1-[*trans*-1-methyl-4-(methyloxy)cyclohexyl]-4-piperidinyl}-2-oxo-2,3-dihydro-1*H*-benzimidazole-5-carbonitrile
39. 6-Chloro-3-{1-[*trans*-4-(ethyloxy)-1-methylcyclohexyl]-4-piperidinyl}-2-oxo-2,3-dihydro-1*H*-benzimidazole-5-carbonitrile
and salts and solvates thereof.

7. A pharmaceutical composition comprising a compound claimed in any one of claims 1 to 6 and a pharmaceutically acceptable carrier.

8. A compound as claimed in any one of claim 1 to 6 for use in therapy.

9. A compound as claimed in any one of claims 1 to 6 for use in the treatment of a condition which requires agonism of a muscarinic M₁ receptor.

10. A compound as claimed in any one of claims 1 to 6 for use in the treatment of a psychotic disorder or cognitive impairment.

11. Use of a compound as claimed in any one of claims 1 to 6 in the manufacture of a medicament for the treatment of a condition which requires agonism of a muscarinic M₁ receptor.

12. Use of a compound as claimed in any one of claims 1 to 6 in the manufacture of a medicament for the treatment of a psychotic disorder or cognitive impairment.

13. A process for preparing a compound as claimed in claim 1, which process is selected from:
- process (A1) which comprises coupling a compound of formula (II) 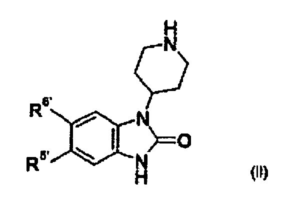 with a compound of formula (III) 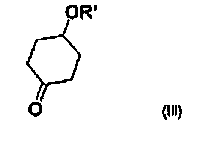 wherein R^{5'} is a group R⁵ defined in claim 1, or a group convertible to R⁵, R^{8'} is a group R⁶ as defined in claim 1, or a group convertible to R⁸, R' is a group R as defined in claim 1, or a group convertible to R, under conditions suitable for reductive alkylation;
- process (A2) which comprises reacting a compound of formula (II) with a compound of formula (III) in the presence of a source of cyanide to form the cyano intermediate (XXXX) which can be reacted with an alkyl Grlgnard reagent QMgX to form compounds of formula (I): 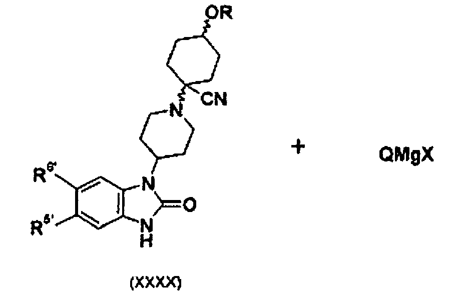 wherein R^{5'} is a group R⁵ defined in claim 1, or a group convertible to R⁵, R^{6'} is a group R⁶ as defined in claim 1, or a group convertible to R⁶, R' is a group R as defined in claim 1, or a
group convertible to R; Q is C₁₋₆alkyl, and X is bromo, iodo or chloro; under conditions suitable for Grignard reactions;
- process (B) which comprises coupling a compound of formula (IV): 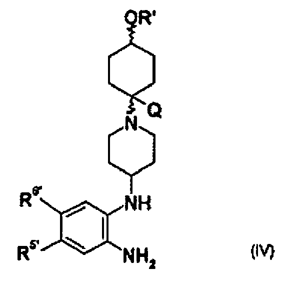 with a compound of formula (V): 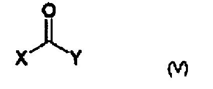 wherein R^{5'} is a group R⁵ defined in claim 1, or a group convertible to R⁵, R^{6'} is a group R⁶ as defined in claim 1, or a group convertible to R⁶, R' is a group R as defined in claim 1, or a group convertible to R, Q is as defined in claim 1, and X and Y both represent leaving groups, optionally in an inert solvent, optionally in the presence of a base, and optionally with heating;
- process (C) which comprises treatment of a compound of formula (VI): 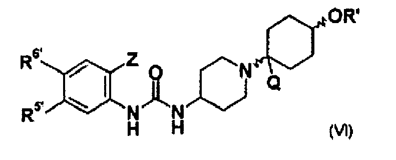 with a palladium or copper catalyst (VII) to effect an intramolecular cyclisation, wherein R^{5'} is a group R⁵ defined in claim 1, or a group convertible to R⁵, R^{6'} is a group R⁶ as defined in claim 1, or a group convertible to R⁶, R' is a group R as defined in claim 1, or a group convertible to R, Q is as defined in claim 1, and Z is a leaving group such as bromo, iodo, chloro or triflate;
- process (D) which comprises coupling a compound of formula (VIII): 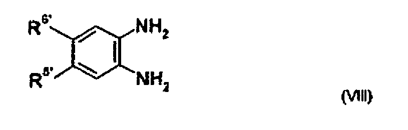 with a compound of formula (IX): 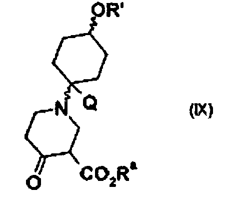 wherein R^{5'} is a group R⁵ defined in claim 1, or a group convertible to R⁵, R^{6'} is a group R⁶ as defined in claim 1, or a group convertible to R⁶, R' is a group R as defined in claim 1, or a group convertible to R, Q is as defined in claim 1, and R^{a} is a C₁₋₅ alkyl group, by heating in an inert solvent, for example xylene, followed by reduction of the piperidine double bond;
- process (E) which comprises reaction of a compound of formula (X): 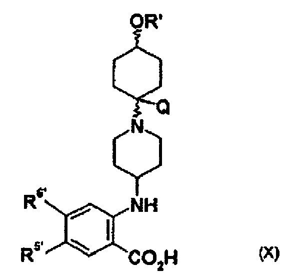 with a reagent/combination of reagents to effect the Curtius rearrangement of compound (X), followed by intramolecular cyclisation; wherein R^{5'} is a group R⁵ defined in claim 1, or a group convertible to R⁵, R^{6'} is a group R⁶ as defined in claim 1, or a group convertible to R⁶, R' is a group R as defined in claim 1, or a group convertible to R, Q is as defined in claim 1; and
- process (F) which comprises coupling a compound of formula (XI): 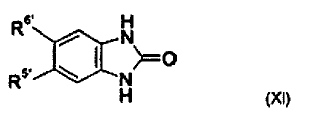 with a compound of formula (XII): 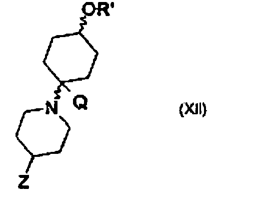 wherein R^{5'} is a group R⁵ defined in claim 1, or a group convertible to R⁶, R^{6'} is a group R⁶ as defined in claim 1, or a group convertible to R⁶, R' is a group R as defined in claim 1, or a group convertible to R, Q is as defined in claim 1, and Z is hydroxy or a leaving group under alkylation or Mitsunobu reaction conditions.

14. A compound of formula (IV) 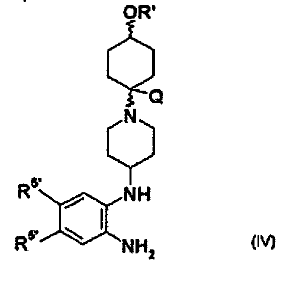 a compound of formula (VI) 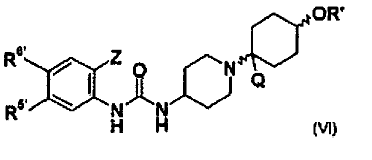 or a compound of formula (X) 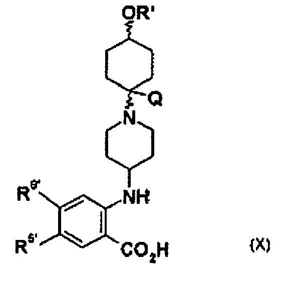 wherein R^{5'} is a group R⁵ defined in claim 1. □ R^{6'} is a group R⁶ as defined in claim 1 , R' is a group R as defined in claim 1, □ Z is bromo, iodo chloro or triflate and Q is as defined in claim 1.

## Patentansprüche

1. Eine Verbindung der Formel (I) oder ein Salz oder Solvat davon: 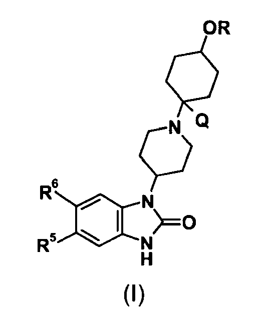 wobei:
- R⁵ aus Halogen, Cyano, C₁₋₆-Alkyl, C₁₋₆-Alkyl, substituiert mit einem oder mehreren Fluoratomen, C₁₋₆-Alkoxy und C₁₋₆-Alkoxy, substituiert mit einem oder mehreren Fluoratomen, ausgewählt ist;
- R⁶ aus Wasserstoff, Halogen, Cyano, C₁₋₆-Alkyl, C₁₋₆-Alkyl, substituiert mit einem oder mehreren Fluoratomen, C₁₋₆-Alkylsulfonyl, C₃₋₆-Cycloalkyl, C₃₋₆-Cycloalkyl, substituiert mit einem oder mehreren Fluoratomen, C₁₋₆-Alkoxy und C₁₋₆-Alkoxy, substituiert mit einem oder mehreren Fluoratomen, ausgewählt ist;
- R aus C₁₋₆-Alkyl, C₃₋₆-Cycloalkyl, C₃₋₆-Cycloalkyl-C₁₋₆-alkyl und C₂₋₆-Alkinyl ausgewählt ist, wobei jeder Alkyl- oder Cycloalkylrest gegebenenfalls mit einem oder mehreren Fluoratomen substituiert ist; und
- Q Wasserstoff oder C₁₋₆-Alkyl ist.

2. Eine Verbindung wie in Anspruch 1 beansprucht, wobei R⁵ aus Halogen, Cyano, C₁₋₄-Alkyl, C₁₋₄-Alkyl, substituiert mit einem, zwei oder drei Fluoratomen, C₁₋₄-Alkoxy und C₁₋₄-Alkoxy, substituiert mit einem, zwei oder drei Fluoratomen, ausgewählt ist.

3. Eine Verbindung wie in Anspruch 1 oder Anspruch 2 beansprucht, wobei R⁶ aus Wasserstoff, Halogen, Cyano, C₁₋₄-Alkyl, C₁₋₄-Alkyl, substituiert mit einem, zwei oder drei Fluoratomen, C₁₋₄-Alkylsulfonyl, C₃₋₆-Cycloalkyl, C₃₋₆-Cycloalkyl, substituiert mit einem oder mehreren Fluoratomen, C₁₋₄-Alkoxy und C₁₋₄-Alkoxy, substituiert mit einem, zwei oder drei Fluoratomen, ausgewählt ist.

4. Eine Verbindung wie in Anspruch 1, 2 oder 3 beansprucht, wobei R aus C₁₋₄-Alkyl, C₃₋₆-Cycloalkyl, C₃₋₆-Cycloalkyl-C₁₋₄-alkyl und C₂₋₄-Alkinyl ausgewählt ist, wobei jeder Alkyl- oder Cycloalkylrest gegebenenfalls mit einem, zwei oder drei Fluoratomen substituiert ist.

5. Eine Verbindung wie in einem der Ansprüche 1-4 beansprucht, wobei Q aus Wasserstoff, Methyl, Ethyl und Propyl ausgewählt ist.

6. Eine Verbindung wie in Anspruch 1 beansprucht, die aus
1. 5-Fluor-6-methyl-1-{1-[*cis*-4-(methyloxy)cyclohexyl]-4-piperidinyl}-1,3-dihydro-2*H*-benzimidazol-2-on
2. 5-Fluor-6-methyl-1-{1-[*trans*-4-(methyloxy)cyclohexyl]-4-piperidinyl}-1,3-dihydro-2*H*-benzimidazol-2-on
3. 5-Chlor-6-methyl-1-{1-[*cis*-4-(methyloxy)cyclohexyl]-4-piperidinyl}-1,3-dihydro-2*H*-benzimidazol-2-on
4. 5-Chlor-6-methyl-1-{1-[*trans*-4-(methyloxy)cyclohexyl]-4-piperidinyl}-1,3-dihydro-2*H*-benzimidazol-2-on
5. 6-Brom-1-{1-[*cis*-4-(methyloxy)cyclohexyl]-4-piperidinyl}-5-(trifluormethyl)-1,3-dihydro-2*H*-benzimidazol-2-on
6. 6-Brom-1-{1-[*trans*-4-(methyloxy)cyclohexyl]-4-piperidinyl}-5-(trifluormethyl)-1,3-dihydro-2*H-*benzimidazol-2-on
7. 1-{1-[*trans*-4-(Ethyloxy)cyclohexyl]-4-piperidinyl}-5-fluor-6-methyl-1,3-dihydro-2*H*-benzimidazol-2-on
8. 5-Chlor-6-methyl-1-{1-[*cis*-4-(propyloxy)cyclohexyl]-4-piperidinyl}-1,3-dihydro-2*H*-benzimidazol-2-on
9. 5-Chlor-6-methyl-1-{1-[*trans*-4-(propyloxy)cyclohexyl]-4-piperidinyl}-1,3-dihydro-2*H*-benzimidazol-2-on
10. 5-Fluor-6-methyl-1-{1-[*cis*-4-(propyloxy)cyclohexyl]-4-piperidinyl}-1,3-dihydro-2*H*-benzimidazol-2-on
11. 5-Fluor-6-methyl-1-(1-[*trans*-4-(propyloxy)cyclohexyl]-4-piperidinyl)-1,3-dihydro-2*H*-benzimidazol-2-on
12. 6-Brom-1-{1-[*cis*-4-(propyloxy)cyclohexyl]-4-piperidinyl}-5-(trifluormethyl)-1,3-dihydro-2*H*-benzimidazol-2-on
13. 6-Brom-1-{1-[*trans*-4-(propyloxy)cyclohexyl]-4-piperidinyl}-5-(trifluormethyl)-1,3-dihydro-2*H-*benzimidazol-2-on
14. *cis*-1-{1-[4-(Ethyloxy)cyclohexyl]-4-piperidinyl}-5,6-dimethyl-1,3-dihydro-2*H-*benzimidazol-2-on
15. *trans*-1-{1-[4-(Ethyloxy)cyclohexyl]-4-piperidinyl}-5,6-dimethyl-1,3-dihydro-2*H-*benzimidazol-2-on
16. 5-Brom-6-methyl-1-[1-(*trans*-4-ethoxycyclohexyl)-4-piperidinyl]-1,3-dihydro-2*H-*benzim idazol-2-on
17. 5-Chlor-1-{1-[*trans*-4-(ethyloxy)cyclohexyl]-4-piperidinyl}-6-methyl-1,3-dihydro-2*H*-benzimidazol-2-on
18. 6-Chlor-5-methyl-1-[1-(*trans*-4-ethoxycyclohexyl)-4-piperidinyl]-1,3-dihydro-2*H-*benzimidazol-2-on
19. 6-Chlor-1-{1-[4-(ethyloxy)cyclohexyl]-4-piperidinyl}-5-methyl-1,3-dihydro-2*H-*benzimidazol-2-on
20. 5-Brom-6-methyl-1-[1-(*trans*-4-propoxycyclohexyl)-4-piperidinyl]-1,3-dihydro-2*H*-benzimidazol-2-on
21. 6-Chlor-1-{1-[*trans*-4-(propyloxy)cyclohexyl]-4-piperidinyl}-5-(trifluormethyl)-1,3-dihydro-2*H-*benzimidazol-2-on
22. 5-Fluor-6-methyl-1-(1-(*trans*-4-[(1-methylethyl)oxy]cyclohexyl)-4-piperidinyl)-1,3-dihydro-2*H-*benzimidazol-2-on
23. 5-Fluor-6-methyl-1-{1-[*trans*-1-methyl-4-(methyloxy)cyclohexyl]-4-piperidinyl }-1,3-dihydro-2*H-*benzimidazol-2-on
24. 1-{(1-[*trans*-4-(Ethyloxy)-1-methylcyclohexyl]-4-piperidinyl)-5-fluor-6-methyl-1,3-dihydro-2*H-*benzimidazol-2-on
25. 5-Chlor-1-{1-[*trans*-4-(ethyloxy)-1-methylcyclohexyl]-4-piperidinyl}-6-methyl-1,3-dihydro-2*H-*benzimidazol-2-on
26. 5-Fluor-6-methyl-1-{1-[*cis*-1-methyl-4-(propyloxy)cyclohexyl]-4-piperidinyl}-1,3-dihydro-2*H*-benzimidazol-2-on
27. 5-Fluor-6-methyl-1-{1-[*trans*-1-methyl-4-(propyloxy)cyclohexyl]-4-piperidinyl}-1,3-dihydro-2*H*-benzimidazol-2-on hydrochlorid
28. 5-Chlor-6-methyl-1-{1-[*cis*-1-methyl-4-(propyloxy)cyclohexyl]-4-piperidinyl }-1,3-dihydro-2*H*-benzimidazol-2-on
29. 5-Chlor-6-methyl-1-{ 1-[*trans*-1-methyl-4-(propyloxy)cyclohexyl]-4-piperidinyl}-1,3-dihydro-2*H-*benzimidazol-2-on
30. 6-Brom-1-{1-[*trans*-1-methyl-4-(propyloxy)cyclohexyl]-4-piperidinyl}-5-(trifluormethyl)-1,3-dihydro-2*H*-benzimidazol-2-on
31. 1-{1-[*trans*-4-(Ethyloxy)-1-methylcyclohexyl]-4-piperidinyl}-5,6-dimethyl-1,3-dihydro-2*H*-benzimidazol-2-on
32. 1-{1-[*trans*-4-(Ethyloxy)-1-methylcyclohexyl]-4-piperidinyl}-5,6-difluor-1,3-dihydro-2*H*-benzimidazol-2-on
33. 5-Chlor-1-{1-[*trans*-1-methyl-4-(methyloxy)cyclohexyl]-4-piperidinyl}-1,3-dihydro-2*H*-benzimidazol-2-on
34. 5-Chlor-1-{ 1-[*trans*-4-(ethyloxy)-1-methylcyclohexyl]-4-piperidinyl}-1,3-dihydro-2*H*-benzimidazol-2-on
35. 6-Chlor-3-{1-[*trans*-4-(ethyloxy)cyclohexyl]-4-piperidinyl}-2-oxo-2,3-dihydro-1*H*-benzimidazol-5-carbonitril
36. 5,6-Dimethyl-1-{1-[*trans*-1-methyl-4-(methyloxy)cyclohexyl]-4-piperidinyl}-1,3-dihydro-2*H*-benzimidazol-2-on
37. 5,6-Difluor-1-{1-[*trans*-1-methyl-4-(methyloxy)cyclohexyl]-4-piperidinyl}-1,3-dihydro-2*H*-benzimidazol-2-on
38. 6-Chlor-3-{1-[*trans*-1-methyl-4-(methyloxy)cyclohexyl]-4-piperidinyl}-2-oxo-2,3-dihydro-1*H*-benzimidazol-5-carbonitril
39. 6-Chlor-3-{1-[*trans*-4-(ethyloxy)-1-methylcyclohexyl]-4-piperidinyl}-2-oxo-2,3-dihydro-1*H*-benzimidazol-5-carbonitril
und Salzen und Solvaten davon ausgewählt ist.

7. Ein Arzneimittel, umfassend eine Verbindung wie in einem der Ansprüche 1 bis 6 beansprucht und einen pharmazeutisch verträglichen Träger.

8. Eine Verbindung wie einem der Ansprüche 1 bis 6 beansprucht zur Verwendung bei der Therapie.

9. Eine Verbindung wie in einem der Ansprüche 1 bis 6 beansprucht zur Verwendung bei der Behandlung eines Zustandes, der einen Agonismus eines Muskarin-M₁-Rezeptors erfordert.

10. Eine Verbindung wie in einem der Ansprüche 1 bis 6 beansprucht zur Verwendung bei der Behandlung einer psychotischen Störung oder kognitiven Beeinträchtigung.

11. Verwendung einer Verbindung wie in einem der Ansprüche 1 bis 6 beansprucht bei der Herstellung eines Medikaments zur Behandlung eines Zustandes, der einen Agonismus eines Muskarin-M₁-Rezeptors erfordert.

12. Verwendung einer Verbindung wie in einem der Ansprüche 1 bis 6 beansprucht bei der Herstellung eines Medikaments zur Behandlung einer psychotischen Störung oder kognitiven Beeinträchtigung.

13. Ein Verfahren zur Herstellung einer Verbindung wie in Anspruch 1 beansprucht, wobei das Verfahren aus:
- Verfahren (A1), welches das Kuppeln einer Verbindung der Formel (II) 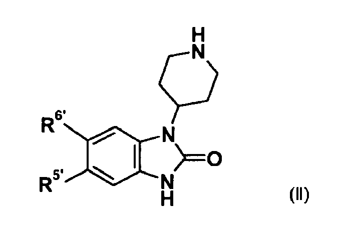 mit einer Verbindung der Formel (III) 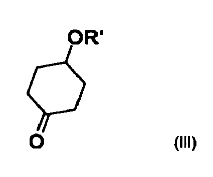 wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, bei Bedingungen, die zu reduktiver Alkylierung geeignet sind, umfasst;
- Verfahren (A2), welches das Umsetzen einer Verbindung der Formel (II) mit einer Verbindung der Formel (III)in Gegenwart einer Cyanidquelle zur Bildung des Cyano-Zwischenproduktes (XXXX) umfasst, das zur Bildung von Verbindungen der Formel (I) mit einem Alkyl-Grignardreagens QMgX umgesetzt werden kann: 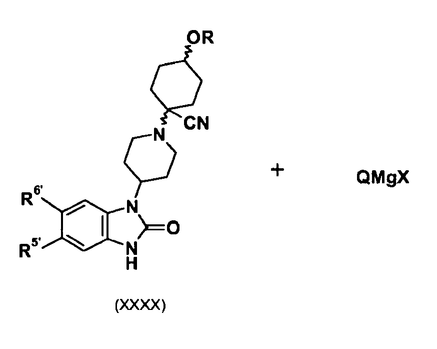 wobei R^{5'} ein wie in Anspruch 1 definierter Rest R⁵ ist oder ein Rest ist, der in R⁵. umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann; Q C₁₋₆-Alkyl ist und X Brom, Iod oder Chlor ist; bei Bedingungen, die für Grignard-Reaktionen geeignet sind;
- Verfahren (B), welches das Kuppeln einer Verbindung der Formel (IV): 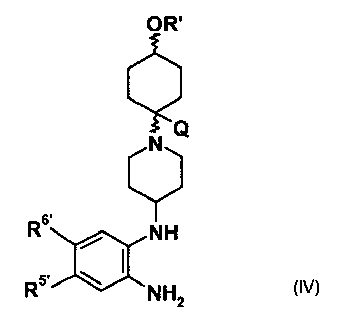 mit einer Verbindung der Formel (V): 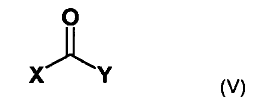 wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist,
und X und Y beide für Abgangsgruppen stehen, gegebenenfalls in einem inerten Lösungsmittel, gegebenenfalls in Gegenwart einer Base und gegebenenfalls unter Erwärmen, umfasst;
- Verfahren (C), welches das Behandeln einer Verbindung der Formel (VI): 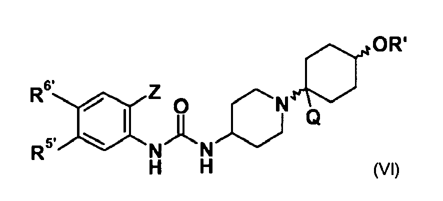 mit einem Palladium- oder Kupferkatalysator (VII) zum Herbeiführen einer intramolekularen Cyclisierung, wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist und Z eine Abgangsgruppe wie etwa Brom, Iod, Chlor oder Triflat ist, umfasst;
- Verfahren (D), welches das Kuppeln einer Verbindung der Formel (VIII): 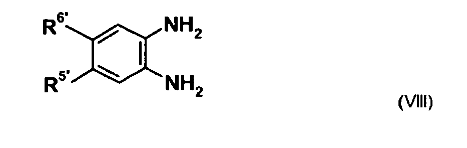 mit einer Verbindung der Formel (IX): 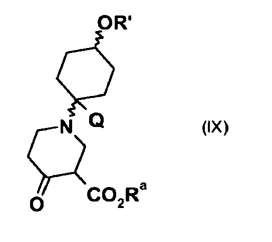 wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist und R^{a} ein C₁₋₅-Alkylrest ist, durch Erwärmen in einem inerten Lösungsmittel, beispielsweise Xylol, gefolgt von Reduzieren der Piperidin-Doppelbindung, umfasst;
- Verfahren (E), welches die Umsetzung einer Verbindung der Formel (X): 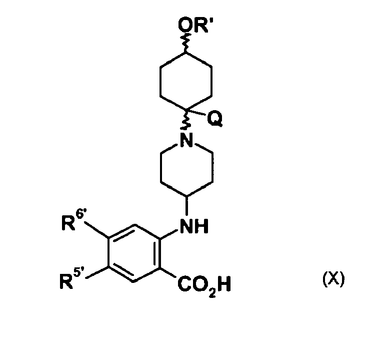 mit einem/-r Reagens/Kombination von Reagenzien zum Herbeiführen einer Curtius-Umlagerung der Verbindung (X), gefolgt von intramolekularer Cyclisierung; wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist, umfasst; und
- Verfahren (F), welches das Kuppeln einer Verbindung der Formel (XI): 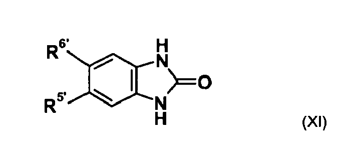 mit einer Verbindung der Formel (XII): 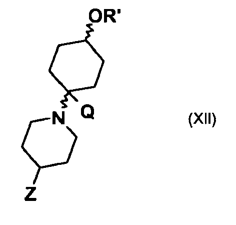 wobei R^{5'} ein wie in Anspruch 1 definierter Rest R⁵ ist oder ein Rest ist, der in R⁵ umgewandelt werden kann, R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist und Z Hydroxy oder eine Abgangsgruppe ist, unter Alkylierungs- oder Mitsunobu-Reaktionsbedingungen, umfasst;
ausgewählt ist.

14. Eine Verbindung der Formel (IV) 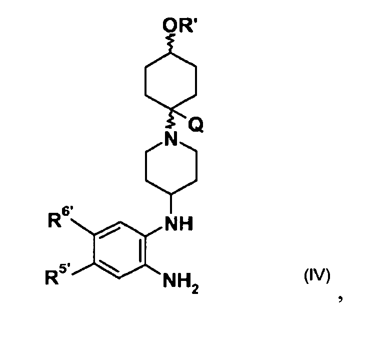 eine Verbindung der Formel (VI) 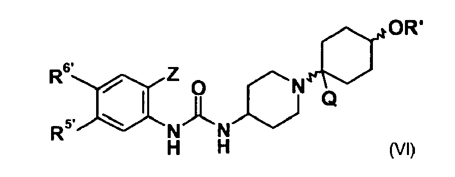 oder eine Verbindung der Formel (X) 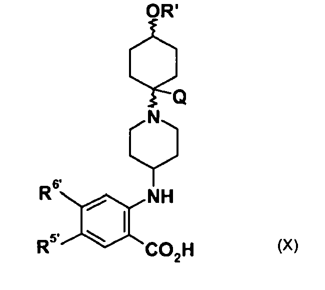 wobei R^{5'} ein wie in Anspruch 1 definierter Rest R⁵ ist, R^{6'} ein wie in Anspruch 1 definierter Rest R⁶ ist, R' ein wie in Anspruch 1 definierter Rest R ist, Z Brom, Iod, Chlor oder Triflat ist und Q wie in Anspruch 1 definiert ist.

## Revendications

1. Composé de formule (I) ou un de ses sels ou produits de solvatation : 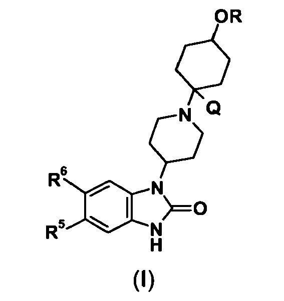 formule dans laquelle :
- R⁵ est choisi entre un atome d'halogène, des groupes cyano, alkyle en C₁ à C₆, alkyle en C₁ à C₆ substitué avec un ou plusieurs atomes de fluor, alkoxy en C₁ à C₆, et alkoxy en C₁ à C₆ substitué avec un ou plusieurs atomes de fluor ;
- R⁶ est choisi entre un atome d'hydrogène et un atome d'halogène, des groupes cyano, alkyle en C₁ à C₆, alkyle en C₁ à C₆ substitué avec un ou plusieurs atomes de fluor, alkylsulfonyle en C₁ à C₆, cycloalkyle en C₃ à C₆, cycloalkyle en C₃ à C₆ substitué avec un ou plusieurs atomes de fluor, alkoxy en C₁ à C₆ et alkoxy en C₁ à C₆ substitué avec un ou plusieurs atomes de fluor ;
- R est choisi entre des groupes alkyle en C₁ à C₆, cycloalkyle en C₃ à C₆, (cycloalkyle en C₃ à C₆) (alkyle en C₁ à C₆) et alcynyle en C₂ à C₆, n' importe quel groupe alkyle ou cycloalkyle étant facultativement substitué avec un ou plusieurs atomes de fluor ; et
Q représente un atome d'hydrogène ou un groupe alkyle en C₁ à C₆.

2. Composé suivant la revendication 1, dans lequel R⁵ est choisi entre un atome d'halogène, des groupes cyano, alkyle en C₁ à C₄, alkyle en C₁ à C₄ substitué avec 1, 2 ou 3 atomes de fluor, alkoxy en C₁ à C₄, et alkoxy en C₁ à C₄ substitué avec 1, 2 ou 3 atomes de fluor.

3. Composé suivant la revendication 1 ou la revendication 2, dans lequel R⁶ est choisi entre un atome d'hydrogène, un atome d'halogène, des groupes cyano, alkyle en C₁ à C₄, alkyle en C₁ à C₄ substitué avec 1, 2 ou 3 atomes de fluor, alkylsulfonyle en C₁ à C₄, cycloalkyle en C₃ à C₆, cycloalkyle en C₃ à C₆ substitué avec un ou plusieurs atomes de fluor, alkoxy en C₁ à C₄, et alkoxy en C₁ à C₄ substitué avec 1, 2 ou 3 atomes de fluor.

4. Composé suivant la revendication 1, 2 ou 3, dans lequel R est choisi entre des groupes alkyle en C₁ à C₄, cycloalkyle en C₃ à C₆, (cycloalkyle en C₃ à C₆) (alkyle en C₁ à C₄) et alcynyle en C₁ à C₄, n'importe quel groupe alkyle ou cycloalkyle étant facultativement substitué avec 1, 2 ou 3 atomes de fluor.

5. Composé suivant l'une quelconque des revendications 1 à 4, dans lequel Q est choisi entre un atome d'hydrogène, des groupes méthyle, éthyle et propyle.

6. Composé suivant la revendication 1, qui est choisi entre
1. la 5-fluoro-6-méthyl-1-{1-[*cis*-4-(méthyloxy)cyclohexyl]-4-pipéridinyl}-1,3-2*H*-benzimidazole-2-one,
2. la 5-fluoro-6-méthyl-1-{1-[*trans*-4-(méthyloxy)-cyclohexyl]-4-pipéridinyl}-1,3-2*H*-benzimidazole-2-one,
3. la 5-chloro-6-méthyl-1-{1-[*cis*-4-(méthyloxy)cyclohexyl]-4-pipéridinyl}-1,3-dihydro-2*H*-benzimidazole-2-one,
4. la 5-chloro-6-méthyl-1-{1-[*trans*-4-(méthyloxy)-cyclohexyl]-4-pipéridinyl}-1,3-dihydro-2*H*-benzimidazole-2-one,
5. la 6-bromo-1-{1-[*cis*-4-(méthyloxy)cyclohexyl]-4-pipéridinyl}-5-(trifluorométhyl)-1,3-dihydro-2*H*-benzimidazole-2-one,
6. la 6-bromo-1-{1-[*trans*-4-(méthyloxy)cyclohexyl]-4-pipéridinyl}-5-(trifluorométhyl)-1,3-dihydro-2*H*-benzimidazole-2-one,
7. la 1-{1-[*trans*-4-(éthyloxy)cyclohexyl]-4-pipéridinyl}-5-fluoro-6-méthyl-1,3-dihydro-2*H*-benzimidazole-2-one,
8. la 5-chloro-6-méthyl-1-{1-[*cis*-4-(propyloxy)cyclohexyl]-4-pipéridinyl}-1,3-dihydro-2*H*-benzimidazole-2-one,
9. la 5-chloro-6-méthyl-1-{1-[*trans*-4-(propyloxy)-cyclohexyl]-4-pipéridinyl}-1,3-dihydro-2*H*-benzimidazole-2-one,
10. la 5-fluoro-6-méthyl-1-{1-[*cis*-4-(propyloxy)-cyclohexyl]-4-pipéridinyl}-1,3-dihydro-2*H*-benzimidazole-2-one,
11. la 5-fluoro-6-méthyl-1-{1-[*trans*-4-(propyloxy)-cyclohexyl]-4-pipéridinyl}-1,3-dihydro-2*H*-benzimidazole-2-one,
12. la 6-bromo-1-{1-[*cis*-4-(propyloxy)cyclohexyl]-4-pipéridinyl}-5-(trifluorométhyl)-1,3-dihydro-2*H*-benzimidazole-2-one,
13. la 6-bromo-1-{1-[*trans*-4-(propyloxy)cyclohexyl]-4-pipéridinyl}-5-(trifluorométhyl)-1,3-dihydro-2*H*-benzimidazole-2-one,
14. la *cis*-1-{1-[4-(éthyloxy) cyclohexyl]-4-pipéridinyl}-5,6-diméthyl-1,3-dihydro-2*H*-benzimidazole-2-one,
15. la *trans*-1-{1-[4-(éthyloxy)cyclohexyl]-4-pipéridinyl}-5,6-diméthyl-1,3-dihydro-2*H*-benzimidazole-2-one,
16. la 5-bromo-6-méthyl-1-{1-[*trans*-4-(éthoxycyclohexyl]-4-pipéridinyl}-1,3-dihydro-2*H*-benzimidazole-2-one,
17. la 5-chloro-1-{1-[*trans*-4-(éthyloxy)-cyclohexyl}-4-pipéridinyl}-6-méthyl-1,3-dihydro-2*H*-benzimidazole-2-one,
18. la 6-chloro-5-méthyl-1-[1-(*trans*-4-éthoxycyclohexyl)-4-pipéridinyl]-1,3-dihydro-2*H*-benzimidazole-2-one,
19. la 6-chloro-1-{1-[4-(éthyloxy)cyclohexyl]-4-pipéridinyl]-5-méthyl-1,3-dihydro-2*H*-benzimidazole-2-one,
20. la 5-bromo-6-méthyl-1-[1-(*trans*-4-propoxycyclohexyl)-4-pipéridinyl]-1,3-dihydro-2*H*-benzimidazole-2-one,
21. la 6-chloro-1-{1-[*trans*-4-(propyloxy)cyclohexyl]-4-pipéridinyl}-5-(trifluorométhyl)-1,3-dihydro-2*H*-benzimidazole-2-one,
22. la 5-fluoro-6-méthyl-1-(1-{*trans*-4-[(1-méthyl-éthyl)oxy]cyclohexyl}-4-pipéridinyl)-1,3-dihydro-2*H-*benzimidazole-2-one,
23. la 5-fluoro-6-méthyl-1-{1-[*trans*-1-méthyl-4-(méthyloxy)cyclohexyl]-4-pipéridinyl}-1,3-dihydro-2*H-*benzimidazole-2-one,
24. la 1-{1-[*trans*-4-(éthyloxy)-1-méthylcyclohexyl]-4-pipéridinyl}-5-fluoro-6-méthyl-1,3-dihydro-2*H*-benzimidazole-2-one,
25. la 5-chloro-1-{1-[*trans*-4-(éthyloxy)-1-méthyl-cyclohexyl]-4-pipéridinyl}-6-méthyl-1,3-dihydro-2*H-*benzimidazole-2-one,
26. la 5-fluoro-6-méthyl-1-{1-[*cis*-1-méthyl-4-(propyl-oxy) cyclohexyl]-4-pipéridinyl}-1,3-dihydro-2*H*-benzimidazole-2-one,
27. le chlorhydrate de 5-fluoro-6-méthyl-1-{1-[*trans-*1-méthyl-4-(propyloxy)cyclohexyl]-4-pipéridinyl}-1,3-dihydro-2*H*-benzimidazole-2-one,
28. la 5-chloro-6-méthyl-1-{1-[*cis*-1-méthyl-4-(propyloxy)cyclohexyl]-4-(propyloxy)cyclohexyl]-4-pipéridinyl}-1,3-dihydro-2*H*-benzimidazole-2-one,
29. la 5-chloro-6-méthyl-1-{1-[*trans*-1-méthyl-4-(propyloxy)cyclohexyl]-4-pipéridinyl}-1,3-dihydro-2*H-*benzmidazole-2-one,
30. la 6-bromo-1-{1-[*trans*-1-méthyl-4-(propyloxy)-cyclohexyl]-4-pipéridinyl}-5-(trifluorométhyl)-1,3-dihydro-2*H*-benzimidazole-2-one
31. la 1-{1-[*trans*-4-(éthyloxy)-1-méthylcyclohexyl]-4-pipéridinyl}-5,6-diméthyl-1,3-dihydro-2*H*-benzimidazole-2-one
32. la 1-{1-[*trans*-4-(éthyloxy)-1-méthylcyclohexyl]-4-pipéridinyl}-5,6-difluoro-1,3-dihydro-2*H*-benzimidazole-2-one,
33. la 5-chloro-1-{1-[*trans*-1-méthyl-4-(méthyloxy)-cyclohexyl-4-pipéridinyl]-1,3-dihydro-2*H*-benzimidazole-2-one
34. la 5-chloro-1-{1-[*trans*-4-(éthyloxy)-1-méthyl-cyclohexyl]-4-pipéridinyl]-1,3-dihydro-2*H*-benzimidazole-2-one
35. la 6-chloro-3-{1-[*trans*-4-(éthyloxy)cyclohexyl]-4-pipéridinyl]-2-oxo-2,3-dihydro-1*H*-benzimidazole-5-carbonitrile
36. la 5,6-diméthyl-1-{1-[*trans*-1-méthyl-4-(méthyl-oxy)cyclohexyl]-4-pipéridinyl]-1,3-dihydro-2*H*-benzimidazole-2-one
37. la 5,6-difluoro-1-{1-[*trans*-1-méthyl-4-(méthyloxy)-cyclohexyl]-4-pipéridinyl}-1,3-dihydro-2*H*-benzimidazole-2-one
38. le 6-chloro-3-{1-[*trans*-1-méthyl-4-(méthyloxy)-cyclohexyl]-4-pipéridinyl}-2-oxo-2,3-dihydro-1*H*-benzimidazole-5-carbonitrile
39. le 6-chloro-3-{1-[*trans*-4-(éthyloxy)-1-méthyl-cyclohexyl]-4-pipéridinyl]-2-oxo-2,3-dihydro-1*H*-benzimidazole-5-carbonitrile
et ses sels et produits de solvatation.

7. Composition pharmaceutique comprenant un composé suivant l'une quelconque des revendications 1 à 6 et un support pharmaceutiquement acceptable.

8. Composé suivant l'une quelconque des revendications 1 à 6, pour une utilisation en thérapie.

9. Composé suivant l'une quelconque des revendications 1 à 6, pour une utilisation dans le traitement d'une affection qui nécessite l'agonisme d'un récepteur muscarinique M₁.

10. Composé suivant l'une quelconque des revendications 1 à 6, pour une utilisation dans le traitement d'un trouble psychotique ou d'une altération cognitive.

11. Utilisation d'un composé suivant l'une quelconque des revendications 1 à 6 dans la production d'un médicament pour le traitement d'une affection qui nécessite l'agonisme d'un récepteur muscarinique M₁.

12. Utilisation d'un composé suivant l'une quelconque des revendications 1 à 6 dans la production d'un médicament pour le traitement d'un trouble psychotique ou d'une altération cognitive.

13. Procédé pour la préparation d'un composé suivant la revendication 1, procédé qui est choisi entre :
- le procédé (A1), qui comprend le couplage d'un composé de formule (II) 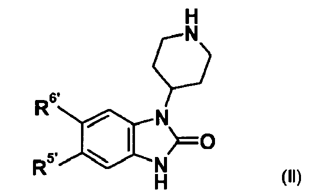 avec un composé de formule (III) 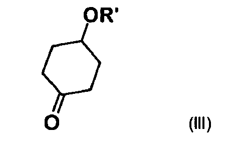 formules dans lesquelles R⁵' représente un groupe R⁵ répondant à la définition figurant dans la revendication 1, ou un groupe pouvant être converti en un groupe R⁵, R⁶' représente un groupe R⁶ répondant à la définition figurant dans la revendication 1, ou un groupe pouvant être converti en un groupe R⁶, R' représente un groupe R répondant à la définition figurant dans la revendication 1, ou un groupe pouvant être converti en un groupe R, dans des conditions convenables pour une alkylation réductrice ;
- le procédé (A2), qui comprend la réaction d'un composé de formule (II) avec un composé de formule (III) en présence d'une source de cyanure pour former l'intermédiaire à fonction cyano (XXXX) qui peut être amené à réagir avec un réactif alkylique de Grignard QMgX pour former des composés de formule (I) : 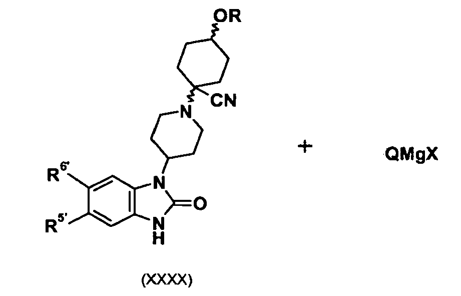 dans laquelle R⁵' représente un groupe R⁵ défini dans la revendication 1, ou un groupe pouvant être converti en un groupe R⁵, R⁶' représente un groupe R⁶ tel que défini dans la revendication 1 ou un groupe pouvant être converti en un groupe R ; Q représente un groupe alkyle en C₁ à C₆, et X représente un groupe bromo, iodo ou chloro ; dans des conditions convenables pour des réactions de Grignard ;
- le procédé (B), qui comprend le couplage d'un composé de formule (IV) : 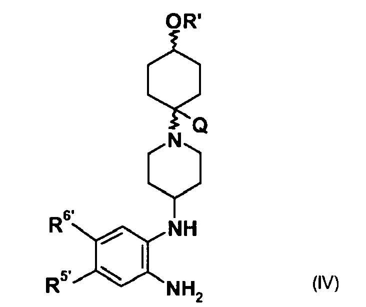 avec un composé de formule (V) : 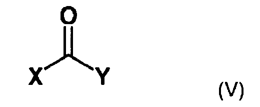 formules dans lesquelles R⁵' représente un groupe R⁵ défini dans la revendication 1, ou un groupe pouvant être converti en un groupe R⁵, R⁶' représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, et X et Y représentent l'un et l'autre des groupes partant, éventuellement dans un solvant inerte, éventuellement en présence d'une base et éventuellement avec un chauffage ;
- le procédé (C), qui comprend le traitement d'un composé de formule (VI) : 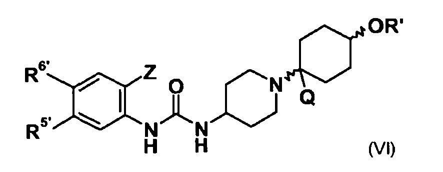 avec un catalyseur au palladium ou au cuivre (VII) pour effectuer une cyclisation intramoléculaire, formule dans laquelle R⁵' représente un groupe R⁵ défini dans la revendication 1, ou un groupe pouvant être converti en un groupe R⁵, R⁶' représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, et Z représente un groupe partant tel qu'un groupe bromo, iodo ou triflate ;
- le procédé (D), qui comprend le couplage d'un composé de formule (8) : 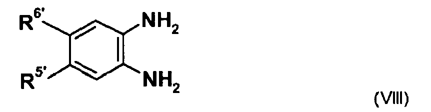 avec un composé de formule (IX) : 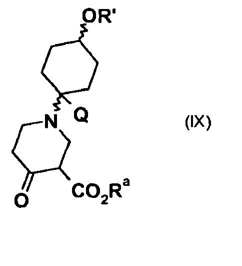 formules dans lesquelles R⁵' représente un groupe R⁵ défini dans la revendication 1, ou un groupe pouvant être converti en un groupe R⁵, R⁶' représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, et R^{a} représente un groupe alkyle en C₁ à C₅, par chauffage dans un solvant inerte, par exemple le xylène, avec ensuite une réduction de la double liaison de la pipéridine ;
- le procédé (E), qui comprend la réaction d'un composé de formule (X) : 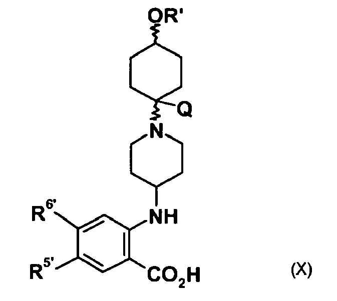 avec un réactif/une association de réactifs pour effectuer le réarrangement de Curtius du composé (X), avec ensuite une cyclisation intramoléculaire ; formule dans laquelle R⁵' représente un groupe R⁵ défini dans la revendication 1, ou un groupe pouvant être converti en un groupe R⁵, R⁶' représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1 ; et
- le procédé (F), qui comprend le couplage d'un composé de formule (XI) : 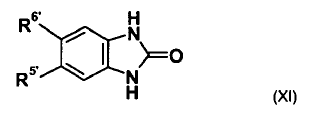 avec un composé de formule (XII) : 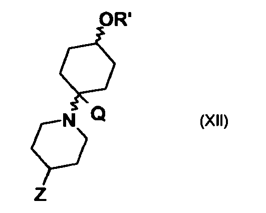 formules dans lesquelles R⁵' représente un groupe R⁵ défini dans la revendication 1, ou un groupe pouvant être converti en un groupe R⁵, R⁶' représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, et Z représente un groupe hydroxy ou un groupe partant dans des conditions de réaction d'alkylation ou de Mitsunobu.

14. Composé de formule (IV) 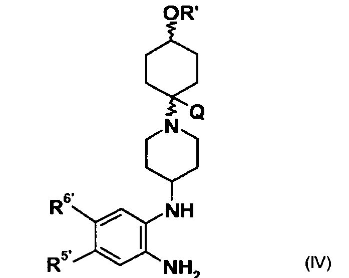 composé de formule (VI) 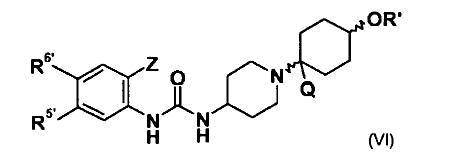 ou composé de formule (X) 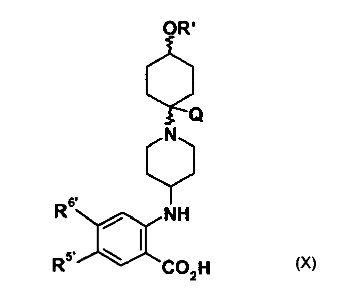 formules dans lesquels R⁵' représente un groupe R⁵ défini dans la revendication 1, R⁶' représente un groupe R⁶ tel que défini dans la revendication 1, R' représente un groupe R tel que défini dans la revendication 1, Z représente un groupe bromo, iodo, chloro ou triflate et Q est tel que défini dans la revendication 1.
